(19) Europäisches Patentamt
European Patent Office
Office européen des brevets

(11) Veröffentlichungsnummer : **0 054 513**
**B1**

(12) # EUROPÄISCHE PATENTSCHRIFT

(45) Veröffentlichungstag der Patentschrift : **30.10.85**

(51) Int. Cl.⁴ : **G 03 C 5/52**, G 03 C 7/00, G 03 C 7/30

(21) Anmeldenummer : **81810487.9**

(22) Anmeldetag : **09.12.81**

(54) **Verfahren zur Herstellung photographischer Farbbilder nach dem Silberfarbbleichverfahren und das in diesem Verfahren verwendete photographische Material.**

(30) Priorität : **15.12.80 CH 9248/80**

(43) Veröffentlichungstag der Anmeldung : **23.06.82 Patentblatt 82/25**

(45) Bekanntmachung des Hinweises auf die Patenterteilung : **30.10.85 Patentblatt 85/44**

(84) Benannte Vertragsstaaten : **BE CH DE FR GB IT LI NL**

(56) Entgegenhaltungen :
**GB-A- 1 020 047**
**KEINE**
**ULLMANNS ENCYKLOPÄDIE DER TECHNISCHEN CHEMIE, 4. Auflage, Band 9, 1975, VERLAG CHEMIE, Weinheim, Bergstrasse, BRD, Seiten 393-402, 565, 570**

(73) Patentinhaber : **CIBA-GEIGY AG**
**Postfach**
**CH-4002 Basel (CH)**

(72) Erfinder : **Forte, Eddy, Dr.**
**Avenue Secrétan 1**
**CH-1005 Lausanne (CH)**
Erfinder : **Fryberg, Mario, Dr.**
**Dery-le-Mont**
**CH-1724 Praroman-le-Mouret (CH)**
Erfinder : **Jan, Gérald, Dr.**
**Rte de la Poudrière 33**
**CH-1700 Fribourg (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 054 513 B1

Jouve, 18, rue St-Denis, 75001 Paris, France

**Beschreibung**

Die vorliegende Erfindung betrifft ein Verfahren zur Herstellung photographischer Farbbilder nach dem Silberfarbbleichverfahren durch Belichtung, Silberentwicklung, Farbbildung, Farbbleichung, Silberbleichung und Fixierung eines photographischen Materials, welches auf einem transparenten oder opaken Träger mindestens eine Schicht mit einer lichtempfindlichen Silberhalogenidemulsion enthält, wobei die Farbbildung gegebenenfalls mit der Farbbleichung, Silberbleichung und Fixierung, und die Silberbleichung gegebenenfalls mit der Farbbleichung und/oder Fixierung in einem einzigen Verarbeitungsbad gleichzeitig durchgeführt werden kann.

Photographische Materialien zur Herstellung von Bildern nach dem Silberfarbbleichverfahren enthalten normalerweise mindestens drei Schichten mit je einem bleichbaren Azofarbstoff, der mit je einer lichtempfindlichen Silberhalogenidemulsion kombiniert ist. Damit die ganze Skala der natürlich vorkommenden Farben wiedergegeben werden kann, hat es sich als vorteilhaft erwiesen, je einen blaugrünen, purpurfarbenen und gelben Bildfarbstoff zu verwenden. Als Farbstoffe kommen sowohl wasserlösliche Azofarbstoffe in Frage, deren Molekülgrösse so gewählt wird, dass sie in den Schichten diffusionsfest eingelagert werden können, als auch öllösliche Farbstoffe, die nach üblichen Methoden fein verteilt als Dispersion in die Schichten eingelagert werden.

Ein inhärentes Merkmal aller Silberfarbbleichmaterialien ist ihre Eigenschaft, wegen der eingelagerten Farbstoffe einen wesentlichen Anteil der bei der Belichtung aufgewendeten Strahlung zu absorbieren. Darunter leidet selbstverständlich die photographische Empfindlichkeit. Silberfarbbleichmaterialien eignen sich daher in erster Linie nur für Kopiermaterialien und sind als Kamerafilme im allgemeinen nicht geeignet.

Eine Methode, mit welcher die Nachteile von Silberfarbbleichmaterialien mit eingelagerten Azofarbstoffen vermieden werden können, besteht z. B. darin, anstelle der Azofarbstoffe deren farblose Ausgangsverbindungen, nämlich diazotierbare Amine und Kupplungskomponenten zu verwenden. Diese Komponenten werden dann frühestens nach erfolgter Belichtung durch Diazotierung der Amine und Kupplung in entsprechende Azofarbstoffe umgewandelt und schliesslich unter Mitwirkung des durch Entwicklung entstandenen Bildsilbers dem Silberfarbbleichprozess unterworfen. Dabei können wahlweise die diazotierbaren Amine oder die Kupplungskomponente in den Schichten eingelagert werden. Die Farbstoffbildung erfolgt dann durch Nachbehandlung in einem Bad, welches die zweite Komponente enthält. Es können aber auch von Anfang an beide Komponenten in der Schicht vorhanden sein, wobei die Nachbehandlung in einer Folge von Bädern erfolgt, welche die Diazotierung und Kupplung unter geeigneten pH-Bedingungen bewirkt und schliesslich mit der eigentlichen Silberfarbbleichung ihren Abschluss findet. Voraussetzung ist in jedem Fall, dass die in den Schichten eingelagerten Komponenten diffusionsfest sind, und dass der photographische Prozess weder durch die eingelagerten Komponenten noch durch die farbstofferzeugenden Bäder gestört wird.

Silberfarbbleichverfahren, bei denen die Azobildfarbstoffe durch Diazotierung und Kupplung in der Schicht nach erfolgter Belichtung und gegebenenfalls Entwicklung des Bildsilbers gebildet werden, sind in einer ganzen Reihe von Patentschriften schon früher vorgeschlagen worden, z. B. in der GB-Patentschrift 488 853, oder in den US-Patentschriften 2 071 688, 2 166 049, 2 333 126, 2 361 541, 2 368 463, 2 514 233 und 2 514 234.

Die direkte Verwendung kupplungsfähiger Diazoverbindungen in photographischen Schichten ist wegen deren geringer Stabilität im allgemeinen nicht möglich. Daher wird das Diazoniumsalz immer erst während der Verarbeitung des photographischen Materials durch Diazotierung des entsprechenden Amins gebildet, sei dies in einer photographischen Schicht oder in einer vorher zubereiteten Verarbeitungslösung. Es ist jedoch bekannt, dass gewisse Diazoniumverbindungen wie etwa die Diazosulfonate oder die Diazoamino- und -iminoverbindungen (Triazene) im Gegensatz zu den bei der Umsetzung mit salpetriger Säure in mineralsaurer Lösung entstehenden Diazoniumsalzen sehr stabil sind und sich gegebenenfalls auch für die Einlagerung in photographische Schichten eignen.

Die Verwendung solcher stabiler Diazoverbindungen ist z. B. in den US-Patentschriften 2 368 463, 2 340 051, 3 338 711 erwähnt. Insbesondere ist die Verwendung von Triazenen in den US-Patentschriften 2 071 688, 2 616 806, 2 653 874 und 2 681 856 beschrieben. Solche stabilen Diazoniumverbindungen besitzen zwar die erforderliche Haltbarkeit in den photographischen Schichten, da sie jedoch als solche nicht kupplungsfähig sind, müssen sie im Laufe der Verarbeitung zuerst durch Einwirkung einer starken Säure in die kupplungsfähigen Diazoniumsalze gespalten werden. Dabei ist für die Kupplung im allgemeinen eine neuerliche Erhöhung des pH-Wertes erforderlich. Die weitere Verarbeitung erfolgt dann entweder durch den Silberfarbbleichprozess oder durch Zersetzung der kupplungsfähigen Diazoniumverbindung am vorhandenen Bildsilber.

Die Verwendung von Triazenen und anderen stabilen Diazoniumverbindungen bietet zwar den Vorteil, dass bei der Verarbeitung die eigentliche Diazotierungsreaktion entfällt. Da jedoch die stabilisierte Diazoniumverbindung zuerst in das kupplungsfähige Diazoniumsalz umgewandelt werden muss, ist trotzdem eine zusätzliche Verarbeitungsstufe notwendig.

Im weiteren müssen alle in die Schichten einzulagernden Verbindungen diffusionsbeständig sein. Diese Forderung ist bei den Triazenen nicht leicht zu erfüllen. Sie erfordert meist die Verwendung von

speziellen Fällungs- und/oder Beizmitteln, welche die Verbindung in unlöslicher Form in der Schicht fixieren. Im allgemeinen wird man es vorziehen, solche leicht löslichen Verbindungen nicht in der Schicht einzulagern, sondern sie im Lauf der Verarbeitung aus einem Bad in die Schicht eindiffundieren zu lassen. Dabei ergibt sich jedoch eine neue Schwierigkeit, indem die aus dem Triazen entstehenden Diazoniumsalze durch vorhandenes Bildsilber leicht zersetzt werden. Beim Eindiffundieren besteht deshalb die Gefahr, dass die Diazoniumverbindungen durch Silberbilder in nicht zugeordneten Schichten ebenfalls angegriffen werden, womit nicht beabsichtigte und stark störende Zwischenschichteffekte entstehen können.

In der US-Patentschrift 2 653 874 ist ein Verfahren beschrieben worden, das diese Schwierigkeit dadurch umgeht, dass vor der Spaltung des Triazens das vorhandene Bildsilber durch Oxydation zu inaktivem Silberhalogenid umgewandelt und erst nach beendeter Farbstoffkupplung wiederum zu metallischem Silber entwickelt wird, damit am Schluss die bildmässige Farbbleichung stattfinden kann. Dieses Verfahren führt zu einem sehr komplizierten 9-stufigen Verarbeitungsprozess.

Aufgabe der vorliegenden Erfindung ist somit die Bereitstellung eines neuen Verfahrens zur Herstellung photographischer Farbbilder nach dem Silberfarbbleichverfahren unter Verwendung eines Materials erhöhter Empfindlichkeit, welches nach Belichtung und Entwicklung eine vereinfachte Verarbeitung zum Farbbild erlaubt.

Es wurde nun ein Verfahren gefunden, in welchem ein photographisches Silberfarbbleichmaterial verwendet wird, das mindestens eine Schicht mit fein verteilten, eine Triazen- und eine Kupplungskomponente enthaltenden Oeltröpfchen enthält und nach Belichtung und Entwicklung mit einer sauren, einen Phasentransferkatalysator enthaltenden Verarbeitungslösung photographische Farbbilder hoher Qualität liefert.

Wenn man ein solches Material nach Belichtung und Entwicklung des Silberbilds unter Zufügen eines Phasentransferkatalysators mit einem üblichen Silberfarbbleichbad behandelt, so gelingt es, die drei Stufen

1. Spaltung des Triazens zum Diazoniumsalz
2. Kupplung zum Farbstoff und
3. Silberfarbbleichung

in einem Arbeitsgang zu vollziehen, und damit ein fertiges Farbbild zu erhalten, das nur noch der üblichen Fixierung zur Entfernung des verbleibenden Silberhalogenids bedarf.

Gegenstand der vorliegenden Erfindung ist daher ein Verfahren zur Herstellung photographischer Farbbilder nach dem Silberfarbbleichverfahren durch Belichtung, Silberentwicklung, Farbbildung, Farbbleichung, Silberbleichung und Fixierung eines photographischen Materials, welches auf einem transparenten oder opaken Träger mindestens eine Schicht mit einer lichtempfindlichen Silberhalogenidemulsion enthält, wobei die Farbbildung gegebenenfalls mit der Farbbleichung, Silberbleichung und Fixierung, und die Silberbleichung gegebenenfalls mit der Farbbleichung und/oder der Fixierung in einem einzigen Verarbeitungsbad gleichzeitig durchgeführt werden kann, dadurch gekennzeichnet, dass die lichtempfindliche(n) Silberhalogenidemulsionsschicht(en) oder (je) eine dieser Silberhalogenidemulsionsschicht(en) benachbarte Schicht ein öllösliches Triazen der Formel

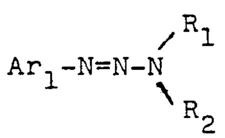 (1) oder 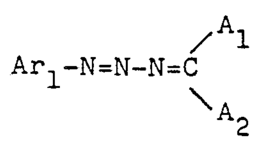 (2)

und eine öllösliche Kupplungskomponente der Formel

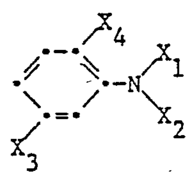 (3)

in Oel dispergiert enthält (enthalten), worin

$Ar_1$ gegebenenfalls substituiertes Aryl oder ein gegebenenfalls substituierter, aromatischer, heterocyclischer Rest,

$R_1$ Wasserstoff, gegebenenfalls substituiertes Alkyl mit 1 bis 6 Kohlenstoffatomen, $—(CH_2CH_2O)_r—L_1$ oder $—OL_1$, worin $L_1$ Alkyl mit 1 bis 12 Kohlenstoffatomen und r 1, 2 oder 3 ist, gegebenenfalls substituiertes Aryl, Hydroxyl oder ein Rest der Formel

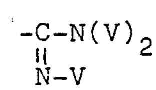

ist, worin V Wasserstoff oder Alkyl mit 1 bis 12 Kohlenstoffatomen ist,

$R_2$ gegebenenfalls substituiertes Alkyl mit 1 bis 6 Kohlenstoffatomen, $-(CH_2CH_2O)_r-L_1$, worin $L_1$ und r die oben angegebenen Bedeutungen haben, oder gegebenenfalls substituiertes Aryl ist, oder

$R_1$ und $R_2$ zusammen mit dem Stickstoffatom, an das sie gebunden sind, einen gegebenenfalls substituierten, gesättigten oder ungesättigten, gegebenenfalls ein weiteres Heteroatom enthaltenden 5-, 6- oder 7-gliedrigen Ring bilden,

$A_1$ und $A_2$ unabhängig voneinander ein Rest

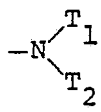

sind,

worin $T_1$ und $T_2$ unabhängig voneinander Wasserstoff, gegebenenfalls substituiertes Alkyl mit 1 bis 12 Kohlenstoffatomen oder gegebenenfalls substituiertes Aryl sind,

$X_1$ und $X_2$ unabhängig voneinander Wasserstoff oder gegebenenfalls substituiertes Alkyl mit 1 bis 20 Kohlenstoffatomen sind,

$X_3$ Wasserstoff, gegebenenfalls substituiertes Alkyl mit 1 bis 6 Kohlenstoffatomen, gegebenenfalls substituiertes Alkoxy mit 1 bis 20 Kohlenstoffatomen, $-O(C_2H_4O)_n-H$, $-O(CH_2)_m-OH$, $-O(CH_2)_m-OZ_1$ oder $-O-(C_2H_4O)_n-Z_1$, worin $Z_1$ Alkyl mit 1 bis 8 Kohlenstoffatomen und n eine ganze Zahl von 1 bis 5 und m 2, 3 oder 4 ist, gegebenenfalls substituiertes Aryloxy, Hydroxyl, Halogen, $-NHCO-Y_1$, NHCOH, $-NHCO-O-Y_1$, $-NHP(O)(OY_1)_2$ oder $-NHSO_2-Y_1$, worin $Y_1$ gegebenenfalls substituiertes Alkyl mit 1 bis 16 Kohlenstoffatomen, $-O(C_2H_4O)_n-H$, $-O(CH_2)_m-OH$, $-O(CH_2)_m-OZ_1$ oder $-O(C_2H_4O)_n-Z_1$ worin $Z_1$, m und n die oben angegebenen Bedeutungen haben, oder $Y_1$ gegebenenfalls substituiertes Aryl ist, und $X_4$ Wasserstoff, gegebenenfalls substituiertes Alkyl mit 1 bis 12 Kohlenstoffatomen, $-O(C_2H_4O)_n-H$, $-O(CH_2)_m-OH$, $-O(CH_2)_m-OZ_1$ oder $-O-(C_2H_4O)_n-Z_1$, worin $Z_1$, m und n die oben angegebenen Bedeutungen haben, gegebenenfalls substituiertes Alkoxy mit 1 bis 16 Kohlenstoffatomen, gegebenenfalls substituiertes Aryloxy oder Halogen ist, und die Summe der Kohlenstoffatome in den Substituenten $X_1$, $X_2$, $X_3$ und $X_4$ mindestens 10 beträgt;

und das Material nach Entwickeln des Bildsilbers mit einer sauren Verarbeitungslösung behandelt wird, die einen zur Uebertragung von Kationen befähigten Phasentransferkatalysator enthält.

Ein weiterer Gegenstand der Erfindung betrifft ein für das erfindungsgemässe Verfahren geeignetes photographisches Material.

Gegenstand der Erfindung sind ferner Zubereitungen zur Verarbeitung des im erfindungsgemässen Verfahren verwendeten Materials.

Unter benachbarten Schichten sind solche Schichten zu verstehen, die durch ihre gegenseitige Lage den Austausch chemischer Spezies — Moleküle oder Ionen — begünstigen. Der Begriff umfasst deshalb auch solche Schichten, die nicht unmittelbar benachbart sind, sondern gegebenenfalls durch eine oder mehrere dünne, die Diffusion nicht behindernde Schichten voneinander getrennt sind.

Der Substituent $Ar_1$ ist gegebenenfalls substituiertes Aryl. Als Arylreste können Phenyl und Naphthyl in Frage kommen, wobei Phenyl bevorzugt ist. Diese Reste können mit Alkyl oder Alkoxy mit je 1 bis 4 Kohlenstoffatomen, z. B., n-Butyl, i-Butyl, t-Butyl, n-Propyl, i-Propyl oder vorzugsweise Aethyl oder Methyl, oder n-Butoxy, i-Butoxy, t-Butoxy, n-Propoxy, i-Propoxy oder vorzugsweise Aethoxy oder Methoxy, substituiert sein. Die Alkylreste können mit Halogen wie z. B. Fluor, Chlor oder Brom weiter substituiert sein. Bevorzugte Reste sind z. B., $-CF_3$, $-C_2F_5$, $-CCl_3$ oder $-CBr_3$. Als Substituenten können ferner in Frage kommen; Carboxyl (—COOH) oder Alkoxycarbonyl, wobei der Alkoxyteil 2 bis 12, vorzugsweise 2 bis 7 Kohlenstoffatome enthält. Ein besonders geeigneter Alkoxycarbonylrest enthält 2 bis 4 Kohlenstoffatome im Alkoxyteil.

Diese Alkoxyteilgruppierungen können sowohl geradkettige als auch verzweigte Kohlenstoffreste enthalten. Die Reste $-SO_2T_1$, $-SO_2N(T_1)_2$ und $-SO_2NT_1T_2$ können weitere Substituenten für die Arylreste sein. Darin bedeuten $T_1$ und $T_2$ unabhängig voneinander Wasserstoff, gegebenenfalls substituiertes Alkyl mit 1 bis 12 Kohlenstoffatomen oder gegebenenfalls substituiertes Aryl. Substituenten der Alkylreste können Methoxy, Hydroxy oder Cyano sein. Alkylreste mit 1 bis 6 Kohlenstoffatomen, insbesondere Methyl und Aethyl sind bevorzugt. In der Bedeutung von Aryl sind $T_1$ und $T_2$ vorzugsweise Phenyl, welches gegebenenfalls mit Methylgruppen weitersubstituiert sein kann. Weitere Substituenten an $Ar_1$ sind Halogen wie z. B. Fluor, Chlor oder Brom, insbesondere Chlor oder Brom, Cyano oder Nitro.

Alle bisher aufgezählten Substituenten von $Ar_1$ können die ortho-Stellung an einem Naphthylrest oder vorzugsweise die ortho-Stellungen an einem Phenylrest besetzen.

Als weitere Substituenten von $Ar_1$, welche die para-Stellung eines Naphthyl- oder, vorzugsweise, eines Phenylrestes einnehmen können, kommen in Frage: Alkyl mit 1 bis 20 Kohlenstoffatomen, wie z. B. Methyl, Aethyl, n-Propyl, i-Propyl, n-Butyl, i-Butyl, t-Butyl, Amyl, tert-Amyl (1,1-Dimethylpropyl), 1,1,3,3-Tetramethylbutyl, 1-Methyläthylpentyl, Hexyl, 1-Methylpentyl, Neopentyl, 1-, 2- oder 3-Methylhexyl, Heptyl, n-Octyl, tert-Octyl, 2-Aethylhexyl, n-Nonyl, i-Nonyl, tert-Nonyl, Decyl, tert-Decyl, Undecyl, Dodecyl Tetradecyl, Hexadecyl, Octadecyl oder Eicosyl, wobei Alkylketten mit 1 bis 16 Kohlenstoffatomen bevorzugt werden, ferner Alkoxy mit 1 bis 20, insbesondere 1 bis 16 Kohlenstoffatomen, wobei die den

Alkylresten analogen Reste in Frage kommen können, Alkoxycarbonyl mit 2 bis 21, insbesondere 2 bis 16 Kohlenstoffatomen, wobei der Alkoxyteil mit Alkoxy mit 1 bis 6 Kohlenstoffatomen substituiert sein kann, $—SO_2T_3$, $—SO_2N(T_3)_2$ oder $—SO_2NT_1T_3$, worin $T_3$ Alkyl mit 1 bis 20, vorzugsweise 1 bis 16 Kohlenstoffatomen, oder ein Rest der Formel $—(CH_2)_p—OT_1$ ist, worin $T_1$ die oben angegebenen Bedeutungen hat und p 2, 3 oder 4 ist, ferner Halogen, wie z. B. Fluor, Chlor oder Brom, wobei Chlor und Brom bevorzugt sind, Cyano, Nitro, $—CF_3$, $—C_2F_5$, $—CCl_3$ oder $—CBr_3$ oder ein Rest der Formel

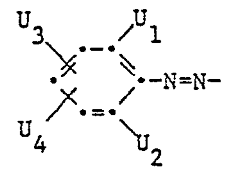

worin $U_1$ und $U_2$ unabhängig voneinander Wasserstoff, Halogen, wie z. B. Fluor, Chlor oder Brom, vorzugsweise Chlor oder Brom, Cyano, Nitro oder $—SO_2U_5$ sind, worin $U_5$ Alkyl mit 1 bis 4 Kohlenstoffatomen, insbesondere Methyl oder Aethyl ist, und $U_3$ Wasserstoff, Halogen, wovon Chlor und Brom bevorzugt sind, oder Nitro ist. $U_4$ bedeutet Alkyl oder Alkoxy mit je 1 bis 20, insbesondere 1 bis 16 Kohlenstoffatomen, wobei diese Reste den oben genannten Alkyl- und Alkoxygruppen entsprechen. Als Substituenten an den Alkyl- und Alkoxygruppen können Halogen, z. B. Fluor, oder Alkoxy mit 1 bis 6 Kohlenstoffatomen in Frage kommen. Trifluormethyl ist ein sehr geeigneter Substituent. $U_4$ bedeutet ferner $—SO_2T_3$ und $—SO_2NT_1T_3$ · $T_1$ und $T_3$ haben die oben angegebenen Bedeutungen. Ist $U_4$ Alkoxycarbonyl, so enthält der Alkoxyteil 20, insbesondere 16 Kohlenstoffatome. Der Alkoxyteil kann mit Alkoxy mit 1 bis 6 Kohlenstoffatomen weitersubstituiert sein.

Die folgenden Substituenten von $Ar_1$ können die meta-Position eines Naphthyl- oder, vorzugsweise, die meta-Positionen eines Phenylringes besetzen.

Alkyl oder Alkoxy mit je 1 bis 20 Kohlenstoffatomen, wobei Beispiele dieser Reste oben aufgezählt sind, und Alkyl- und Alkoxygruppen mit je 1 bis 12 Kohlenstoffatomen bevorzugt sind. Substituenten dieser Gruppen können Alkoxy mit 1 bis 12 Kohlenstoffatomen, Phenoxy oder Hydroxyl sein. Ferner kommt als Substituent für $Ar_1$ Alkoxycarbonyl mit 2 bis 21 Kohlenstoffatomen in Frage, wobei der Alkoxyteil gegebenenfalls mit Alkoxy mit 1 bis 12 Kohlenstoffatomen, Phenoxy oder Hydroxyl substituiert ist, $—SO_2T_3$, $—SO_2N(T_3)_2$ und $—SO_2NT_1T_3$, wobei $T_1$ und $T_3$ die oben angegebenen Bedeutungen haben.

Ferner kann ein Substituent, der die meta-Position eines Naphthyl- oder, vorzugsweise eines Phenylringes besetzt, mit dem Substituenten in der entsprechenden para-Stellung einen Rest der Formel $—CH=CT_4—CH=CH—$ oder mit dem Substituenten in der entsprechenden ortho-Stellung einen Rest der Formel $—CT_4=CH—CT_5=CH—$ bilden, worin $T_4$ Wasserstoff, Nitro, $—SO_2T_3$, $—SO_2N(T_3)_2$ oder $—SO_2NT_1T_3$ und $T_5$ Wasserstoff, $—OT_3$, $—SO_2T_3$ oder $—SO_2NT_1T_3$ bedeutet. $T_1$ und $T_3$ haben die oben angegebenen Bedeutungen.

Die Summe der Kohlenstoffatome in den ortho-, meta- und para-ständigen Substituenten beträgt mindestens 8, vorzugsweise 10.

$Ar_1$ bedeutet ferner einen gegebenenfalls substituierten, aromatischen heterocyclischen Rest. Dieser kann 1 bis 3 Heteroatome enthalten, wie z. B. Sauerstoff, Schwefel und/oder Stickstoff. 5- oder 6-gliedrige heterocyclische Ringe sind bevorzugt. Die Bindung an die Azogruppierung erfolgt vorzugsweise über ein Kohlenstoffatom der Ringe. Als Substituenten dieser Ringe kommen Cyano, Nitro, gegebenenfalls substituiertes Alkyl mit 6 bis 18 Kohlenstoffatomen, gegebenenfalls substituiertes Phenyl oder Alkoxycarbonyl mit 2 bis 25 Kohlenstoffatomen in Frage. Als Beispiele lassen sich aufzählen :

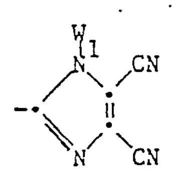 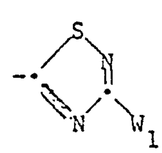 und 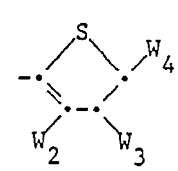

worin $W_1$ gegebenenfalls substituiertes Alkyl mit 6 bis 18, insbesondere 6 bis 12 Kohlenstoffatomen, wobei als Substituenten Alkoxy mit 1 bis 4 Kohlenstoffatomen in Frage kommen, und ferner gegebenenfalls mit Alkyl oder Alkoxy mit je 6 bis 18, insbesondere 6 bis 12 Kohlenstoffatomen substituiertes Phenyl ist. $W_2$ bedeutet Alkoxycarbonyl mit 2 bis 25, insbesondere 2 bis 19 Kohlenstoffatomen, Nitro oder Cyano. $W_3$ und $W_4$ sind unabhängig voneinander Wasserstoff oder Alkyl mit 1 bis 6, insbesondere 1 bis 4 Kohlenstoffatomen. $W_3$ und $W_4$ können auch zusammen einen Rest der Formeln

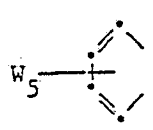 , 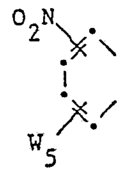 oder 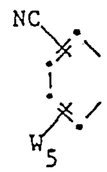

bilden, worin $W_5$ Alkyl mit 6 bis 18 insbesondere 6 bis 12 Kohlenstoffatomen bedeutet. Ferner beträgt die Summe der Kohlenstoffatome in den Substituenten $W_1$ bis $W_5$ mindestens 8. Die für $W_1$ bis $W_5$ genannten Alkyl- und Alkoxyreste entsprechen bezüglich ihrer Struktur den oben genannten Beispielen.

$R_1$ bedeutet Wasserstoff oder gegebenenfalls substituiertes Alkyl mit 1 bis 6, insbesondere 1 bis 4 Kohlenstoffatomen. Besonders geeignete Alkylreste sind Methyl und Aethyl. Als Substituenten der genannten Alkylreste können Hydroxyl, Methoxy, Carboxyl ($-COOH$), Alkoxycarbonyl mit 2 bis 7, insbesondere 2 Kohlenstoffatomen, wobei der Alkoxyteil mit Methoxy oder Cyano substituiert sein kann, und $-SO_3M$, worin M Wasserstoff, Ammonium oder ein Alkalimetall, vorzugsweise Natrium oder Kalium ist, in Frage kommen. Ferner bedeutet $R_1$ einen Rest der Formel $-(CH_2CH_2O)_r-L_1$ oder $-OL_1$, worin $L_1$ Alkyl mit 1 bis 12, insbesondere 1 bis 8 Kohlenstoffatomen bedeutet. Alkyl mit 1 bis 6 Kohlenstoffatomen ist besonders geeignet. Der Index r bedeutet 1, 2 oder 3. Ist $R_1$ Aryl, so ist Phenyl bevorzugt. Die Arylreste können mit Alkyl oder Alkoxy mit je 1 bis 6 Kohlenstoffatomen wie z. B. Methyl, Aethyl, Butyl, Hexyl, Methoxy, Aethoxy, Butoxy oder Hexoxy oder Halogen, wie z. B. Fluor, Chlor oder Brom, substituiert sein. $R_1$ ist auch Hydroxyl oder ein Rest der Formel

$$-\underset{\underset{N-V}{\|}}{C}-N(V)_2$$

worin V Wasserstoff oder Alkyl mit 1 bis 12, insbesondere 1 bis 6 Kohlenstoffatomen ist.

$R_2$ bedeutet gegebenenfalls substituiertes Alkyl mit 1 bis 6 Kohlenstoffatomen. Methyl und Aethyl sind bevorzugt. Substituenten dieser Alkylreste können Hydroxyl, Cyano, Methoxy, Carboxyl ($-COOH$), Alkoxycarbonyl mit 2 bis 7, insbesondere 2 Kohlenstoffatomen, wobei der Alkoxyteil gegebenenfalls mit Methoxy oder Carboxyl ($-COOH$) substituiert ist, oder $-SO_3M$ sein, worin M die oben genannten Bedeutungen hat. $R_2$ ist desweiteren $-(CH_2CH_2O)_r-L_1$, worin $L_1$ und r die oben angegebenen Bedeutungen haben. Ist $R_2$ gegebenenfalls substituiertes Aryl, so ist Phenyl bevorzugt. Diese Reste können mit Alkyl mit 1 bis 6 Kohlenstoffatomen, insbesondere Methyl, oder Halogen, wie z. B. Chlor oder Brom, substituiert sein. Bevorzugte Phenylreste sind Tolyl, Chlorphenyl oder Bromphenyl.

$R_2$ und $R_1$ können zusammen mit dem Stickstoffatom, an das sie gebunden sind, einen 5-, 6- oder 7-gliedrigen heterocyclischen Ring bilden. Als weitere Heteroatome kommen wiederum Stickstoff und Sauerstoff in Frage. Die Heterocyclen können mit Alkyl mit 1 bis 6 Kohlenstoffatomen, insbesondere Methyl, substituiert sein. Beispiele für aus $R_2$ und $R_1$ gebildete, gesättigte und ungesättigte, zweiwertige Reste sind : $-(CH_2)_4-$, $-(CH_2)_5-$, $-CH_2CH_2-O-CH_2CH_2-$, $-CH_2CH_2-NH-CH_2CH_2-$, $-CH_2CH_2-N(CH_3)-CH_2CH_2-$, $-CH=N-CH=CH-$ und $-CH=CH-CH=CH-$.

Die Reste $A_1$ und $A_2$ sind unabhängig voneinander ein Amin der Formel $-NT_1T_2 \cdot T_1$ und $T_2$ haben die oben angegebenen Bedeutungen.

$X_1$ und $X_2$ sind unabhängig voneinander Wasserstoff oder gegebenenfalls substituiertes Alkyl mit 1 bis 20, insbesondere 1 bis 16 Kohlenstoffatomen. Beispiele für in Frage kommende Alkylreste sind oben aufgezählt. Als Substituenten eignen sich Alkoxy mit 1 bis 5 Kohlenstoffatomen oder Carboxyl ($-COOH$). Besonders bevorzugte Alkylreste sind solche der Formel $-CHM_1-CH_2M_2$. Darin ist $M_1$ Wasserstoff oder Alkyl mit 1 bis 4 Kohlenstoffatomen, insbesondere Methyl oder Aethyl. $M_2$ ist Cyano oder ein Rest der Formel $-OM_3$ oder $-CO_2M_3$, worin $M_3$ Wasserstoff, Alkyl mit 1 bis 16, insbesondere 1 bis 6 Kohlenstoffatomen, gegebenenfalls mit Alkyl mit 1 bis 4 Kohlenstoffatomen, insbesondere Methyl oder Butyl, substituiertes Phenyl, oder ein Rest der Formeln $-(C_2H_4O)_n-M_4$ oder $-(CH_2)_m-OM_4$ ist. $M_4$ bedeutet darin Wasserstoff oder Alkyl mit 1 bis 4, insbesondere 1 oder 2 Kohlenstoffatomen. Die Alkylreste können gegebenenfalls mit Methoxy, Cyano oder Carboxyl ($-COOH$) substituiert sein. Der Index m bedeutet 2, 3 oder 4, un n 1, 2, 3, 4 oder 5. $X_1$ ist vorzugsweise Alkyl mit 1 bis 6 Kohlenstoffatomen oder ein Rest der Formel $-CHM_1-CH_2M_2$, worin $M_1$ und $M_2$ die oben angegebenen Bedeutungen haben, wenn $X_2$ Wasserstoff, Alkyl mit 1 bis 16 Kohlenstoffatomen, ein Rest der Formel $-CHM_1-CH_2M_2$, Benzyl oder Phenyläthyl ist.

$X_3$ ist Wasserstoff oder gegebenenfalls substituiertes Alkyl mit 1 bis 6, insbesondere 1 bis 4 Kohlenstoffatomen. Geeignete Substituenten für die Alkylreste sind Methoxy, Carboxyl ($-COOH$) und Cyano. $X_3$ ist desweiteren Alkoxy mit 1 bis 20, insbesondere 1 bis 16 Kohlenstoffatomen. Alkoxyreste mit 1 bis 6 Kohlenstoffatomen sind ganz besonders geeignet. Beispiele solcher Alkoxyreste sind oben aufgeführt. Ist $X_3$ Aryloxy, so ist Phenoxy bevorzugt, welches gegebenenfalls 1 oder 2 Alkylsubstituenten mit 1 bis 4 Kohlenstoffatomen enthält. Ferner ist $X_3$ ein Rest der Formeln $-O-(C_2H_4O)_n-H$, $-O-(CH_2)_m-OH$, $-O-(CH_2)_m-OZ_1$ oder $-O-(C_2H_4O)_n-Z_1$, worin $Z_1$ Alkyl mit 1 bis 8, vorzugsweise 1 bis 4 Kohlenstoffatomen bedeutet, m 2, 3 oder 4 und n 1, 2, 3, 4 oder 5 ist. Desweiteren ist $X_3$ Hydroxyl, Halogen, wie z. B. Fluor, Chlor oder Brom, wobei Chlor und Brom bevorzugt sind, oder ein Rest der Formeln $-NHCOY_1$, $-NHCOOY_1$, $-NHCOH$, $-NHP(O)(OY_1)_2$ oder $-NHSO_2Y_1$ · $Y_1$ ist gegebenenfalls substituiertes Alkyl mit 1 bis 16 Kohlenstoffatomen. Beispiele solcher Alkylreste sind oben aufgeführt. Als Substituenten für diese Alkylreste können Alkoxy mit 1 bis 4 Kohlenstoffatomen, Carboxyl ($-COOH$) oder Cyano in Frage kommen. Ferner bedeutet $Y_1$, in Rest der Formel $-(C_2H_4O)_n-H$, $-(CH_2)_m-OH$, $-(C_2H_4O)_n-Z_1$ oder $-(CH_2)_m-OZ_1$, worin $Z_1$, n und m die oben angegebenen Bedeutungen haben. Ferner ist $Y_1$ gegebenenfalls substituiertes Aryl, insbesondere Phenyl. Geeignete Substituenten für diese Reste sind 1 oder 2 Alkylgruppen mit 1 bis 4 Kohlenstoffatomen. $X_4$ ist Wasserstoff oder gegebenenfalls substituiertes Alkyl mit 1 bis 12 Kohlenstoffatomen. Beispiele solcher Alkylreste sind oben aufgezählt. Sie können Carboxyl ($-COOH$), Hydroxyl-, Alkoxy- und Cyanogruppen als Substituenten enthalten, wobei die Alkoxygruppen 1 bis 6 Kohlenstoffatome enthalten und vorzugsweise Methoxy, Aethoxy oder Butoxy sind. $X_4$ ist vorzugsweise Alkyl mit 1 bis 8 Kohlenstoffatomen. Diese Alkylreste können mit den genannten Substituenten substituiert sein. Besonders geeignete Alkylreste $X_4$ enthalten 1 bis 4 Kohlenstoffatome. $X_4$ ist ferner ein Rest der Formeln $-O(CH_2CH_2O)_n-H$, $-O(CH_2)_m-OH$, $-C-(CH_2CH_2O)_nZ_1$ oder $-O(CH_2)_m-OZ_1$, worin $Z_1$, m und n die oben angegebenen Bedeutungen haben. Desweiteren ist $X_4$ gegebenenfalls substituiertes Alkoxy mit 1 bis 16, insbesondere 1 bis 8 Kohlenstoffatomen. Beispiele solcher Reste sind oben aufgezählt. Als Substituenten kommen dafür Methoxy, Aethoxy oder Carboxyl ($-COOH$) in Frage. Alkoxyreste mit 1 bis 4 Kohlenstoffatomen sind besonders geeignete Substituenten $X_4$. Ist $X_4$ Aryloxy, wie z. B. Naphthoxy oder Phenoxy, so ist Phenoxy bevorzugt. Als Substituenten für die Arylreste können 1 oder 2 Alkylreste mit 1 bis 4 Kohlenstoffatomen in Frage kommen. Ferner ist $X_4$ Hydroxyl oder Halogen, wie z. B. Fluor, Chlor oder Brom, wobei Chlor und Brom bevorzugt sind.

Die Summe der Kohlenstoffatome in den Substituenten $X_1$, $X_2$, $X_3$ und $X_4$ beträgt mindestens 10.

Bevorzugt ist ein Verfahren, in welchem ein Triazen der Formel

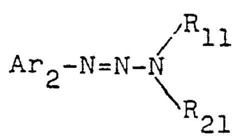 (4) oder 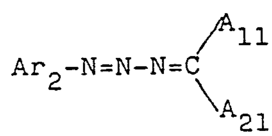 (5)

und eine öllösliche Kupplungskomponente der Formel

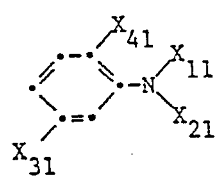 (6)

verwendet werden, worin

$Ar_2$ gegebenenfalls substituiertes Phenyl, gegebenenfalls substituiertes Naphthyl oder ein gegebenenfalls substituierter, aromatischer, 1 bis 3 Heteroatome enthaltender Rest,

$R_{11}$ gegebenenfalls substituiertes Alkyl mit 1 bis 6 Kohlenstoffatomen, $-(CH_2CH_2O)_r-L_{11}$ oder $-OL_{11}$, worin $L_{11}$ Alkyl mit 1 bis 8 Kohlenstoffatomen und r 1, 2 oder 3 ist, gegebenenfalls substituiertes Phenyl oder Hydroxyl ist,

$R_{21}$ gegebenenfalls substituiertes Alkyl mit 1 bis 6 Kohlenstoffatomen, $-(CH_2CH_2O)_r-L_{11}$, worin $L_{11}$ und r die oben angegebenen Bedeutungen haben, oder gegebenenfalls substituiertes Phenyl ist, oder $R_{21}$ und $R_{11}$ zusammen mit dem Stickstoffatom, an das sie gebunden sind, einen gegebenenfalls substituierten, gesättigten oder ungesättigten, gegebenenfalls ein weiteres Heteroatom enthaltenden 5-, 6- oder 7-gliedrigen Ring bilden, und

$A_{11}$ und $A_{21}$ unabhängig voneinander ein Rest der Formel

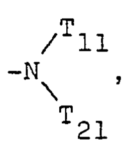 ,

sind,

worin $T_{11}$ und $T_{21}$ unabhängig voneinander Wasserstoff, Alkyl mit 1 bis 12 Kohlenstoffatomen oder Phenyl sind,

$X_{11}$ und $X_{21}$ unabhängig voneinander Wasserstoff oder gegebenenfalls substituiertes Alkyl mit 1 bis 16 Kohlenstoffatomen sind,

$X_{31}$ Wasserstoff, gegebenenfalls substituiertes Alkyl mit 1 bis 4 Kohlenstoffatomen, gegebenenfalls substituiertes Alkoxy mit 1 bis 16 Kohlenstoffatomen, $-O(C_2H_4O)_n-H$, $-O(CH_2)_m-OH$,

$-O(CH_2)_m-OZ_{11}$ oder $-O-(C_2H_4O)_n-Z_{11}$, worin $Z_{11}$ Alkyl mit 1 bis 4 Kohlenstoffatomen ist und m und n die oben angegebenen Bedeutungen haben, gegebenenfalls substituiertes Phenoxy, Hydroxyl, Halogen, $-NHCO-Y_2$, $-NHCO-OY_2$, $-NHCOH$, $-NHP(O)(OY_2)_2$ oder $-NHSO_2-Y_2$, worin $Y_2$ gegebenenfalls substituiertes Alkyl mit 1 bis 16 Kohlenstoffatomen, $-(C_2H_4O)_n-H$, $-(CH_2)_m-OH$, $-(CH_2)_m-OZ_{11}$ oder $-(C_2H_4O)_n-Z_{11}$, worin $Z_{11}$, n und m die oben angegebenen Bedeutungen haben, oder $Y_2$ gegebenenfalls substituiertes Phenyl ist, und

$X_{41}$ Wasserstoff, gegebenenfalls substituiertes Alkyl mit 1 bis 8 Kohlenstoffatomen, $-O(C_2H_4O)_n-H$, $-O(CH_2)_m-OH$, $-O(CH_2)_m-OZ_{11}$ oder $-O-(C_2H_4O)_n-Z_{11}$, worin $Z_{11}$ und n die oben angegebenen Bedeutungen haben, gegebenenfalls substituiertes Alkoxy mit 1 bis 16 Kohlenstoffatomen, gegebenenfalls substituiertes Phenoxy oder Halogen ist, und die Summe der Kohlenstoffatome in den Substituenten $X_{11}$, $X_{21}$, $X_{31}$ und $X_{41}$ mindestens 10 beträgt.

Bevorzugte Triazene der Formel (4) und Kupplungskomponenten der Formel (6) sind solche der Formel

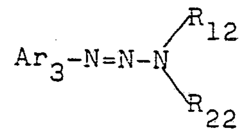

(7)

und

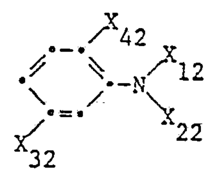

(8)

worin

$Ar_3$ gegebenenfalls substituiertes Phenyl oder gegebenenfalls substituiertes Naphthyl,

$R_{12}$ gegebenenfalls mit Hydroxyl, Cyano, Methoxy, Carboxyl, Alkoxycarbonyl mit 2 bis 7 Kohlenstoffatomen, wobei der Alkoxyteil gegebenenfalls weitersubstituiert ist, oder $-SO_3M$, worin M Wasserstoff, Ammonium oder ein Alkalimetall ist, substituiertes Alkyl mit 1 bis 6 Kohlenstoffatomen, $-(CH_2CH_2O)_r-L_{11}$ oder $-OL_{11}$, worin $L_{11}$ und r die oben angegebenen Bedeutungen haben, gegebenenfalls mit Alkyl oder Alkoxy mit je 1 bis 6 Kohlenstoffatomen oder Halogen substituiertes Phenyl oder Hydroxyl ist,

$R_{22}$ gegebenenfalls mit Hydroxyl, Cyano, Methoxy, Carboxyl, Alkoxycarbonyl mit 2 bis 7 Kohlenstoffatomen, wobei der Alkoxyteil gegebenenfalls weitersubstituiert ist, oder $-SO_3M$, worin M Wasserstoff, Ammonium oder ein Alkalimetall ist, substituiertes Alkyl mit 1 bis 6 Kohlenstoffatomen, $-(CH_2CH_2O)_r-L_{11}$, worin $L_{11}$ und r die oben angegebenen Bedeutungen haben, oder gegebenenfalls mit Alkyl mit 1 bis 6 Kohlenstoffatomen oder Halogen substituiertes Phenyl ist, oder $R_{22}$ und $R_{12}$ zusammen mit dem Stickstoffatom, an das sie gebunden sind, einen gegebenenfalls mit Alkyl mit 1 bis 6 Kohlenstoffatomen substituierten, gesättigten oder ungesättigten, gegebenenfalls ein Stickstoff- oder Sauerstoffatom als weiteres Heteroatom enthaltenden 5-, 6- oder 7-gliedrigen Ring bilden,

$X_{12}$ und $X_{22}$ unabhängig voneinander Wasserstoff, Alkyl mit 1 bis 16 Kohlenstoffatomen, Benzyl, Phenyläthyl oder ein Rest der Formel $-CHM_1-CH_2M_2$ sind, worin $M_1$ Wasserstoff oder Alkyl mit 1 bis 4 Kohlenstoffatomen und $M_2$ Cyano oder ein Rest der Formel $-OM_3$ oder $-CO_2M_3$, worin $M_3$ Wasserstoff, Alkyl mit 1 bis 16 Kohlenstoffatomen, gegebenenfalls mit Alkyl mit 1 bis 4 Kohlenstoffatomen substituiertes Phenyl oder ein Rest der Formel $-(C_2H_4O_n-M_4$ oder $-(CH_2)_m-OM_4$ ist, worin $M_4$ Wasserstoff oder gegebenenfalls substituiertes Alkyl mit 1 bis 4 Kohlenstoffatomen ist, und n und m die oben angegebenen Bedeutungen haben,

$X_{32}$ Wasserstoff, Alkyl mit 1 bis 4 Kohlenstoffatomen, Alkoxy mit 1 bis 16 Kohlenstoffatomen, $-O(C_2H_4O)_n-H$, $-O(CH_2)_m-OH$, $-O(C_2H_4O)m-Z_{11}$ oder $-O(CH_2)_m-OZ_{11}$, worin $Z_{11}$ die oben angegebenen Bedeutung hat und n und m die oben angegebenen Bedeutungen haben, Phenoxy, Hydroxyl, Halogen, $-NHCO-Y_3$, $-NHCOH$, $-NHCO-OY_3$, $-NHP(O)(OY_3)_2$ oder $-NHSO_2-Y_3$, worin $Y_3$ Alkyl mit 1 bis 16 Kohlenstoffatomen, gegebenenfalls mit Alkyl mit 1 bis 4 Kohlenstoffatomen substituiertes Phenyl oder ein Rest der Formel $-(C_2H_4O)_n-H$, $-(CH_2)_m-OH$, $-(C_2H_4O)_n-Z_{11}$ oder $-(CH_2)_m-OZ_{11}$ ist, worin $Z_{11}$, n und m die oben angegebenen Bedeutung haben, und

$X_{42}$ Wasserstoff, Alkyl mit 1 bis 6 Kohlenstoffatomen, Halogen, Alkoxy mit 1 bis 8 Kohlenstoffatomen, oder $-O(C_2H_4O)_n-H$, $-O(CH_2)_m-OH$, $-O(CH_2)_m-OZ_{11}$ oder $-O(C_2H_4O)_n-Z_{11}$ ist, worin $Z_{11}$, n und m die oben angegebenen Bedeutungen haben, oder gegebenenfalls substituiertes Phenoxy ist, und wobei die Summe der Kohlenstoffatome in den Substituenten $X_{12}$, $X_{22}$, $X_{32}$ und $X_{42}$ mindestens 10 beträgt.

Bevorzugte Triazene der Formel (7) sind solche der Formel

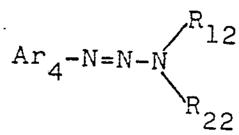

(9)

worin

$Ar_4$ der Formel

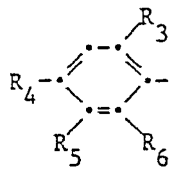

entspricht, worin $R_3$ und $R_6$ unabhängig voneinander Wasserstoff, gegebenenfalls substituiertes Alkyl oder Alkoxy mit je 1 bis 4 Kohlenstoffatomen, Carboxyl, Alkoxycarbonyl 2 bis 12 Kohlenstoffatomen, $—SO_2T_1$, $—SO_2N(T_1)_2$ oder $—SO_2NT_1T_2$, worin $T_1$ und $T_2$ die oben angegebene Bedeutung haben, Halogen, Cyano oder Nitro sind, $R_4$ Wasserstoff, gegebenenfalls substituiertes Alkyl oder Alkoxy mit je 1 bis 20 Kohlenstoffatomen, Alkoxycarbonyl mit 2 bis 21 Kohlenstoffatomen, wobei der Alkoxyteil gegebenenfalls weiter substituiert ist, $—SO_2T_3$, $—SO_2N(T_3)_2$ oder $—SO_2NT_1T_3$, worin $T_3$ Alkyl mit 1 bis 20 Kohlenstoffatomen oder $—(CH_2)_p—OT_1$ ist, $T_1$ die oben angegebenen Bedeutungen hat und p 2, 3 oder 4 ist, oder $R_4$ Halogen, Trifluormethyl, Cyano, Nitro, oder ein Rest der Formel

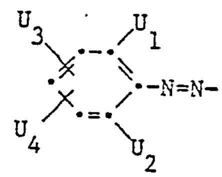

ist, worin $U_1$ und $U_2$ unabhängig voneinander Wasserstoff, $—SO_2U_5$, worin $U_5$ Alkyl mit 1 bis 4 Kohlenstoffatomen ist, Halogen, Cyano oder Nitro sind, $U_3$ Wasserstoff, Halogen oder Nitro und $U_4$ gegebenenfalls substituiertes Alkyl oder Alkoxy mit je 1 bis 20 Kohlenstoffatomen, $—SO_2T_3$, $—SO_2NT_1T_3$, worin $T_1$ und $T_3$ die oben angegebenen Bedeutungen haben, oder Alkoxycarbonyl mit 2 bis 21 Kohlenstoffatomen ist, wobei der Alkoxyteil gegebenenfalls weitersubstituiert ist, $R_5$ Wasserstoff, gegebenenfalls substituiertes Alkyl oder Alkoxy mit je 1 bis 20 Kohlenstoffatomen, Alkoxycarbonyl mit 2 bis 21 Kohlenstoffatomen, wobei der Alkoxyteil gegebenenfalls weitersubstituiert ist, $—SO_2T_3$, $—SO_2N(T_3)_2$ oder $—SO_2NT_1T_3$ ist, worin $T_1$ und $T_3$ die oben angegebenen Bedeutungen haben, oder $R_5$ zusammen mit $R_4$ einen Rest der Formel

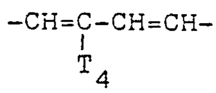

oder $R_5$ zusammen mit $R_6$ einen Rest der Formel

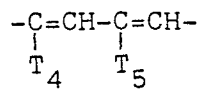

bildet, worin $T_4$ Wasserstoff, Nitro, $—SO_2T_3$, $—SO_2N(T_3)_2$ oder $—SO_2NT_1T_3$ und $T_5$ Wasserstoff, $—OT_3$, $—SO_2T_3$ oder $—SO_2NT_1T_3$ ist, worin $T_1$ und $T_3$ die oben angegebenen Bedeutungen haben, und worin die Summe der Kohlenstoffatome in den Substituenten $R_3$, $R_4$, $R_5$ und $R_6$ mindestens 8 beträgt, und $R_{12}$, $R_{22}$ sowie $X_{12}$, $X_{22}$, $X_{32}$ und $X_{42}$ die oben angegebenen Bedeutungen haben.

Die Triazene der Formel (9) werden vorzugsweise in Kombination mit der Kupplungskomponente der Formel (8) verwendet.

Bevorzugte Triazene der Formel (9) sind solche der Formel

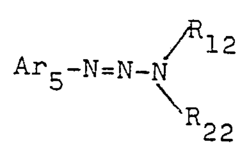

(10)

worin

$Ar_5$ der Formel 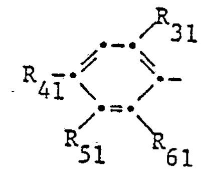

entspricht, worin $R_{31}$ und $R_{61}$ unabhängig voneinander Wasserstoff, Alkyl oder Alkoxy mit je 1 oder 2 Kohlenstoffatomen, Trifluormethyl, Carboxyl, Alkoxycarbonyl mit 2 bis 7 Kohlenstoffatomen, $—SO_2T_{11}$, $—SO_2N(T_{11})_2$ oder $—SO_2NT_{11}T_{21}$, worin $T_{11}$ und $T_{21}$ die oben angegebenen Bedeutungen haben, Fluor, Chlor, Brom, Cyano oder Nitro sind, $R_{41}$ Wasserstoff, Alkyl mit 1 bis 20 Kohlenstoffatomen, Trifluormethyl, gegebenenfalls mit Alkoxy mit 1 bis 6 Kohlenstoffatomen substituiertes Alkoxy mit 1 bis 20 Kohlenstoffatomen, Alkoxycarbonyl mit 2 bis 21 Kohlenstoffatomen, wobei der Alkoxyteil gegebenenfalls mit Alkoxy mit 1 bis 6 Kohlenstoffatomen weitersubstituiert ist, $—SO_2T_{31}$, $—SO_2N(T_{31})_2$ oder $—SONT_{11}T_{31}$, worin $T_{31}$ Alkyl mit 1 bis 20 Kohlenstoffatomen oder $—(CH_2)_p—OT_{11}$ ist, worin $T_{11}$ die oben angegebenen Bedeutungen hat und p 2, 3 oder 4 ist, oder $R_4$ Chlor, Brom, Cyano, Nitro oder ein Rest der Formel

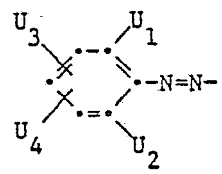

ist, worin $U_{41}$ Wasserstoff, Alkyl mit 1 bis 20 Kohlenstoffatomen, Trifluormethyl, gegebenenfalls mit Alkoxy mit 1 bis 6 Kohlenstoffatomen substituiertes Alkoxy mit 1 bis 20 Kohlenstoffatomen, $—SO_2T_{31}$, $—SO_2NT_{11}T_{31}$, worin $T_{11}$ und $T_{31}$ die oben angegebenen Bedeutungen haben, oder Alkoxycarbonyl mit 2 bis 21 Kohlenstoffatomen ist, wobei der Alkoxyteil gegebenenfalls mit Alkoxy mit 1 bis 6 Kohlenstoffatomen weitersubstituiert ist, $R_{51}$ Wasserstoff, Alkyl mit 1 bis 20 Kohlenstoffatomen, gegebenenfalls mit Alkoxy mit 1 bis 12 Kohlenstoffatomen oder Hydroxyl substituiertes Alkoxy mit 1 bis 20 Kohlenstoffatomen, Alkoxycarbonyl mit 2 bis 21 Kohlenstoffatomen, wobei der Alkoxyteil gegebenenfalls mit Alkoxy mit 1 bis 12 Kohlenstoffatomen weitersubstituiert ist, $—SO_2T_{31}$, $—SO_2N(T_{31})_2$ oder $—SO_2NT_{11}T_{31}$ ist, worin $T_{11}$ und $T_{31}$ die oben angegebenen Bedeutungen haben, oder $R_{51}$ zusammen mit $R_{41}$ einen Rest der Formel

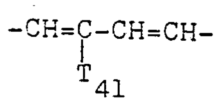

oder $R_{51}$ zusammen mit $R_{61}$ einen Rest der Formel

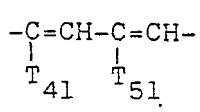

bildet, worin $T_{41}$ Wasserstoff, Nitro, $—SO_2T_{31}$, $—SO_2N(T_{31})_2$ oder $—SO_2NT_{11}T_{31}$ und $T_{51}$ Wasserstoff, $—OT_{31}$, $—SO_2T_{31}$ oder $—SO_2NT_{11}T_{31}$ ist, worin $T_{11}$ und $T_{31}$ die oben angegebenen Bedeutungen haben, und wobei die Summe der Kohlenstoffatome in den Substituenten $R_{31}$, $R_{41}$, $R_{51}$ und $R_{61}$ mindestens 10 beträgt, und $R_{12}$, $R_{22}$ sowie $U_1$, $U_2$ und $U_3$ die oben angegebenen Bedeutungen haben.

Vorzugsweise werden die Triazene der Formel (10) in Kombination mit den Kupplungskomponenten der Formel (8) verwendet. Bevorzugte Kupplungskomponenten der Formel (8) sind solche der Formel

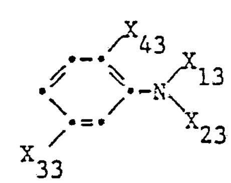 (11)

worin

$X_{13}$ Alkyl mit 1 bis 16 Kohlenstoffatomen oder ein Rest der Formel $—CHM_1—CH_2M_2$, worin $M_1$ und $M_2$ die oben angegebenen Bedeutungen haben,

$X_{23}$ Wasserstoff, Alkyl mit 1 bis 16 Kohlenstoffatomen, ein Rest der Formel $—CHM_1—CH_2M_2$, worin $M_1$ und $M_2$ die oben angegebenen Bedeutungen haben, Benzyl oder Phenyläthyl,

$X_{33}$ Wasserstoff, Alkyl mit 1 bis 4 Kohlenstoffatomen, Alkoxy mit 1 bis 12 Kohlenstoffatomen, $—O(C_2H_4O)_n—Z_{11}$ oder $—O(CH_2)_m—OZ_{11}$, worin $Z_{11}$, n und m die obenangegebenen Bedeutungen haben, Phenoxy, Hydroxyl, Chlor, Brom, $—NHCO—Y_3$, —NHCOH, $—NHCO—OY_3$, $—NHP(O)(OY_3)_2$ oder $—NHSO_2—Y_3$, worin $Y_3$ die oben genannten Bedeutungen hat,

$X_{43}$ Wasserstoff, Alkyl mit 1 bis 4 Kohlenstoffatomen, Chlor, Brom, Alkoxy mit 1 bis 8 Kohlenstoffatomen oder $—O—(CH_2CH_2)O_n—Z_{11}$ ist, worin $Z_{11}$ und n die oben angegebene Bedeutung haben, und wobei die Summe der Kohlenstoffatome in den Substituenten $X_{13}$, $X_{23}$, $X_{33}$ und $X_{43}$ mindestens 10 beträgt.

Vorzugsweise werden die Kupplungskomponenten der Formel (11) in Kombination mit den Triazenen der Formel (10) verwendet.

Bevorzugte Triazene der Formel (10) und Kupplungskomponenten (11) sind solche der Formel

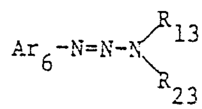

(12)

und

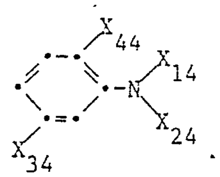

(13)

wobei

$Ar_6$ der Formel

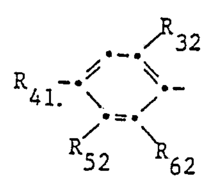

entspricht, worin $R_{32}$ und $R_{62}$ unabhängig voneinander Wasserstoff, Methyl, Methoxy, Trifluormethyl, Alkoxycarbonyl mit 2 bis 4 Kohlenstoffatomen, $—SO_2T_{13}$, $—SO_2NT_{13}$, $—SO_2N(T_{13})_2$ oder $—SO_2NT_{13}T_{23}$, worin $T_{13}$ und $T_{23}$ unabhängig voneinander Wasserstoff oder Alkyl mit 1 oder 2 Kohlenstoffatomen sind, Fluor, Chlor, Brom, Cyano oder Nitro sind, $R_{52}$ Wasserstof, Alkyl mit 1 bis 20 Kohlenstoffatomen, $—OT_{31}$, $—CO—OT_{31}$ oder $—SO_2T_{31}$, worin $T_{31}$ die oben angegebenen Bedeutungen hat, $R_{52}$ zusammen mit $R_{41}$ einen Rest der Formel

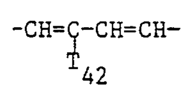

oder $R_{52}$ zusammen mit $R_{62}$ einen Rest der Formel

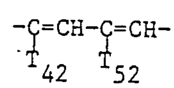

bildet, worin $T_{42}$ Wasserstoff, Nitro, $—SO_2T_{32}$ oder $—SO_2N(T_{32})_2$ und $T_{52}$ Wasserstoff, $—OT_{32}$, $—SO_2T_{32}$ oder $—SO_2NT_{12}T_{32}$ ist, worin $T_{32}$ Alkyl mit 1 bis 10 Kohlenstoffatomen oder $—(CH_2)_p—OT_{12}$, und p und $T_{12}$ sowie $R_{41}$ die oben angegebenen Bedeutungen haben, und wobei die Summe der Kohlenstoffatome in den Substituenten $R_{32}$, $R_{41}$, $R_{52}$ und $R_{62}$ mindestens 10 beträgt,

$R_{13}$ Alkyl mit 1 bis 4 Kohlenstoffatomen, gegebenenfalls mit Hydroxyl, Cyano, Carboxyl, $—COOCH_3$ oder Methoxy substituiertes Alkyl mit 1 oder 2 Kohlenstoffatomen, $—(CH_2CH_2O)_r—L_{12}$ oder $—OL_{12}$,

worin $L_{12}$ Alkyl mit bis 6 Kohlenstoffatomen und r die oben angegebenen Bedeutungen hat, Phenyl, Tolyl, Chlorphenyl, Bromphenyl oder Methoxyphenyl,

$R_{23}$ Alkyl mit 1 bis 4 Kohlenstoffatomen, gegebenenfalls mit Hydroxyl, Cyano, Carboxyl, $—COOCH_3$ oder Methoxy substituiertes Alkyl mit 1 oder 2 Kohlenstoffatomen, $—(CH_2CH_2O)_r—L_{12}$, worin $L_{12}$ und r die oben angegebenen Bedeutung haben, Phenyl, Tolyl, Chlorphenyl oder Bromphenyl ist, oder $R_{23}$ und $R_{13}$ einen Rest der Formel $—(CH_2)_4—$, $—(CH_2)_5—$, $—C_2H_4—O—C_2H_4—$, $—C_2H_4—NH—C_2H_4—$, $—C_2H_4—N(CH_3)—C_2H_4—$, $—CH = N—CH = CH—$ oder $—CH = CH—CH = CH—$ bilden, und

$X_{14}$ Alkyl mit 1 bis 16 Kohlenstoffatomen oder ein Rest der Formel $—CHM_{11}—CH_2M_{21}$, worin $M_{11}$ Wasserstoff, Methyl oder Aethyl und $M_{21}$ Cyano oder $—OM_{31}$ ist, worin $M_{31}$ Wasserstoff, Alkyl mit 1 bis 6 Kohlenstoffatomen oder gegebenenfalls mit Alkyl mit 1 bis 4 Kohlenstoffatomen substituiertes Phenyl ist,

$X_{24}$ Wasserstoff, Alkyl mit 1 bis 16 Kohlenstoffatomen oder Benzyl, $X_{34}$ Wasserstoff, Alkyl mit 1 bis 4 Kohlenstoffatomen, Phenoxy, Chlor, Hydroxyl, Alkoxy mit 1 bis 6 Kohlenstoffatomen, $—O—CH_2CH_2—OH$, $—O—CH_2CH_2—OZ_{11}$, $—NHCO—Y_4$, $—NHCOH$, $—NHCO—CH_2CH_2—OH$, $—NHCO—CH_2CH_2—OZ_{11}$, $—NHP(O)(OY_4)_2$, $—NHP(O)(OC_6H_4Y_5)_2$ oder $—NHSO_2Y_4$, worin $Y_4$ Alkyl mit 1 bis 16 Kohlenstoffatomen, $Y_5$ Wasserstoff oder Alkyl mit 1 bis 6 Kohlenstoffatomen und $Z_{11}$ die oben angegebenen Bedeutungen hat, und

$X_{44}$ Wasserstoff, Alkyl oder Alkoxy mit je 1 bis 4 Kohlenstoffatomen oder $—O—(CH_2CH_2O)_n—Z_{11}$ ist, worin $Z_{11}$ und n die oben angegebenen Bedeutungen haben, und wobei die Summe der Kohlenstoffatome in den Substituenten $X_{14}$, $X_{24}$, $X_{34}$ und $X_{44}$ mindestens 10 beträgt.

Bevorzugte Triazene der Formel (12) sind solche der Formel

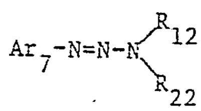

(14)

worin $Ar_7$ ein gegebenenfalls substituierter, aromatischer, 1 bis 3 Sauerstoff-, Schwefel- und/oder Stickstoffatome enthaltender, 5- oder 6-gliedriger Rest ist, und $R_{12}$ und $R_{22}$ die oben angegebenen Bedeutungen haben.

Vorzugsweise werden die Triazene der Formel (14) in Kombination mit den Kupplungskomponenten der Formel (8) verwendet,

Bevorzugte Triazene der Formel (14) sind solche der Formel

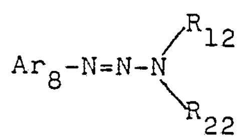

(15)

worin $Ar_8$ ein Rest der Formel

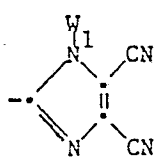

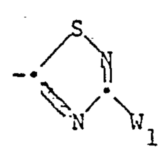

oder

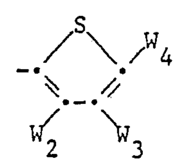

ist, worin

$W_1$ gegebenenfalls substituiertes Alkyl mit 6 bis 18 Kohlenstoffatomen oder gegebenenfalls substituiertes Phenyl, $W_2$ Alkoxycarbonyl mit 2 bis 25 Kohlenstoffatomen, Nitro oder Cyano ist, und $W_3$ und $W_4$ unabhängig voneinander Wasserstoff oder Alkyl mit 1 bis 6 Kohlenstoffatomen sind, oder $W_3$ zusammen mit $W_4$ einen Rest der Formel

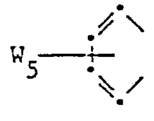

,

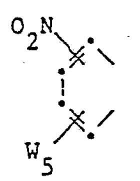

oder

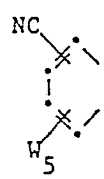

bildet, worin

$W_5$ Alkyl mit 6 bis 18 Kohlenstoffatomen bedeutet, und wobei die Summe der Kohlenstoffatome in den Substituenten $W_1$, $W_2$, $W_3$ und $W_4$ mindestens 8 beträgt und

$R_{12}$ und $R_{22}$ die oben angegebenen Bedeutungen haben.

Vorzugsweise werden die Triazene der Formel (14) in Kombination mit den Kupplungskomponenten der Formel (8) verwendet.

Eine bevorzugte Kombination bilden die Triazene der Formel (15) und die Kupplungskomponenten der Formel (11).

Bevorzugte Triazene der Formel (15) sind solche der Formel

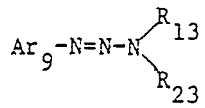

(16)

wobei $Ar_9$ ein Rest der Formel

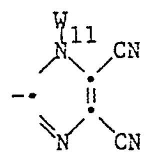 , 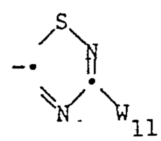 oder 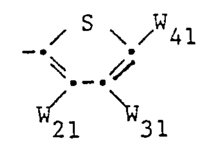

ist, worin

$W_{11}$ Alkyl mit 6 bis 18 Kohlenstoffatomen oder gegebenenfalls mit Alkyl oder Alkoxy mit je 6 bis 18 Kohlenstoffatomen substituiertes Phenyl, $W_{21}$ Alkoxycarbonyl mit 2 bis 19 Kohlenstoffatomen, Nitro oder Cyano ist, und $W_{31}$ und $W_{41}$ unabhängig voneinander Wasserstoff oder Alkyl mit 1 bis 4 Kohlenstoffatomen sind, oder $W_{31}$ und $W_{41}$ zusammen einen Rest der Formel

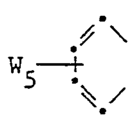 , 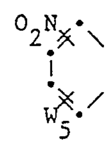 oder 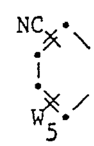

bilden, worin

$W_5$ die oben angegebene Bedeutung hat, und wobei die Summe der Kohlenstoffatome in den Substituenten $W_{11}$, $W_{21}$, $W_{31}$, $W_{41}$ und $W_5$ mindestens 10 beträgt und

$R_{13}$ und $R_{23}$ die in oben angegebenen Bedeutungen haben.

Vorzugsweise werden die Triazene der Formel (16) in Kombination mit den Kupplungskomponenten der Formel (13) verwendet.

Von grosser Bedeutung ist ein Verfahren, worin ein Material verwendet wird, welches in mindestens einer Silberhalogenidemulsionsschicht oder in je einer benachbarten Schicht ein öllösliches Triazen der Formel (1) oder (2) und eine öllösliche Kupplungskomponente der Formel (3) enthält, wobei Triazen und Kupplungskomponente in einem mit Wasser nicht mischbaren Lösungsmittel oder Lösungsmittelgemisch gelöst in feinverteilter Form der (den) lichtempfindlichen Silberhalogenidemulsionsschicht(en) oder in einer dieser (diesen) benachbarten Schicht eingearbeitet vorliegen, und das Material nach Belichtung und Entwicklung des Bildsilbers mit einem wässrigen Verarbeitungsbad behandelt wird, das zur Farbbildung

a) eine starke Säure und

b) einen Kationenphasentransferkatalysator,

gegebenenfalls zur gleichzeitigen Farbbleichung

c) einen Liganden, der Silberkomplexe bildet,

d) einen Farbbleichkatalysator und

e) ein Oxidationsschutzmittel,

gegebenenfalls zur gleichzeitigen Silberbleichung

f) ein Oxidationsmittel und

gegebenenfalls zur gleichzeitigen Fixierung

g) ein Silberhalogenidlösungsmittel

enthält.

In einer Variante des Verfahrens enthält das wässrige Verarbeitungsbad die 7 Komponenten a) bis g).

In einer weiteren Variante enthält das wässrige Verarbeitungsbad die 6 Komponenten a) bis f), und die Fixierung wird in einem separaten Verarbeitungsbad durchgeführt.

Es ist ferner möglich, dass das wässrige Verarbeitungsbad nur die 5 Komponenten a) bis e) enthält. Silberbleichung und Fixierung werden getrennt in einem oder zwei separaten Verarbeitungsbädern durchgeführt.

Enthält das wässrige Verarbeitungsbad die beiden Komponenten a) und b), so werden Farbbleichung, Silberbleichung und Fixierung getrennt in einem, zwei oder drei separaten Verarbeitungsbädern durchgeführt.

Als starke Säuren (Komponente a)) können Alkyl- oder Arylsulfonsäuren und insbesondere p-Toluolsulfonsäure, Schwefelsäure, Sulfaminsäure, oder gegebenenfalls auch Gemische dieser Säuren eingesetzt werden.

Der zur Uebertragung von Kationen befähigte Phasentransferkatalysator (Komponente b) kann beispielsweise eine starke anorganische Säure, eine perhalogenierte aliphatische Säure, eine mit 1 oder 2 Alkyl- oder Alkoxygruppen mit je 1 bis 12 Kohlenstoffatomen substituierte Benzolsulfonsäure, mit 1 bis 3 Halogenatomen substituierte Benzolsulfonsäure, gegebenenfalls halogenierte Alkylsulfonsäure mit 1 bis 12 Kohlenstoffatomen, Monoalkylschwefelsäure mit 1 bis 12 Kohlenstoffatomen oder ein Alkali- oder Ammoniumsalz dieser Säuren sein.

Ein besonders geeigneter Kationentransferkatalysator ist eine Halogenwasserstoffsäure, eine perhalogenierte Alkansäure mit 1 bis 6 Kohlenstoffatomen, eine mit 1 oder 2 Alkyl- oder Alkoxygruppen mit je 1 bis 8 Kohlenstoffatomen substituierte Benzolsulfonsäure, mit 1 bis 3 Halogenatomen substituierte Benzolsulfonsäure, gegebenenfalls halogenierte Alkylsulfonsäure mit 1 bis 8 Kohlenstoffatomen, Monoalkylschwefelsäure mit 1 bis 8 Kohlenstoffatomen oder ein Alkali- oder Ammoniumsalz dieser Säuren.

Ein wertvoller Kationentransferkatalysator ist Perchlor- oder Perjodsäure, Bromwasserstoff- oder Jodwasserstoffsäure, Trifluor-, Trichlor- oder Tribromessigsäure, Pentafluor- oder Pentachlorpropionsäure, p-Toluolsulfonsäure, p-Isopropylbenzolsulfonsäure, 2,4-Dimethylbenzolsulfonsäure, p-Chlorbenzolsulfonsäure, 2,4-Dichlorbenzolsulfonsäure, 3,4-Dichlorbenzolsulfonsäure, 3,6-Dichlorbenzolsulfonsäure, p-Methoxybenzolsulfonsäure, p-Butoxybenzolsulfonsäure, Methansulfonsäure, Aethansulfonsäure, Hexansulfonsäure, Trifluormethan- oder Trichlormethansulfonsäure oder ein Natrium-, Kalium- der Ammoniumsalz dieser Säuren.

Ganz besonders geeignet sind Trifluor- oder Trichloressigsäure oder Perchlorsäure.

Der Kationentransferkatalysator wird in Mengen von 10 bis 200 g, insbesondere 10 bis 100 g pro Liter Verarbeitungslösung eingesetzt.

Als Liganden, die Silberkomplexe bilden (Komponente c) können z. B. wasserlösliche Jodide wie z. B. Alkalimetalliodide, ferner Thioharnstoff oder wasserlösliche, substituierte Alkyl- und Arylphosphine in Frage kommen. Die Komponenten c) und g) können gegebenenfalls identisch sein.

Komponente d) ist ein Diazinderivat. Vorzugsweise kommen Chinoxaline, Pyrazine, Phenazine oder Cinnoline in Frage.

Das Oxidationsschutzmittel, Komponente e), ist vorzugsweise ein Redukton oder eine wasserlösliche Mercaptoverbindung.

Als Oxidationsmittel, Komponente f), verwendet man zweckmässigerweise wasserlösliche, aromatische Mono- oder Dinitroverbindungen sowie Anthrachinonsulfonsäurederivate.

Das erfindungsgemäss verwendete photographische Material enthält auf einem opaken oder transparenten Träger mindestens eine Silberhalogenidemulsionsschicht und in der gleichen oder (in) benachbarten Schicht(en) eine Dispersion des öllöslichen Triazens der Formel (1) oder (2) und der öllöslichen Kupplungskomponente der Formel (3), bevorzugt in stöchiometrischem Verhältnis, in einem mit Wasser nicht mischbaren Lösungsmittel oder Lösungsmittelgemisch.

In einer bevorzugten Ausführungsform enthält das photographische Material in je einer Schicht, je eine rot-, grün- und blauempfindliche Silberhalogenidgelatineemulsion und je in der gleichen oder einer benachbarten Schicht eine zugeordnete, die jeweilige Komplementärfarbe Cyan, Magenta oder Gelb bildende Dispersion einer Lösung aus einem öllöslichen Triazen der Formel (1) oder (2) und einer Kupplungskomponente der Formel (3) in einer mit Wasser nicht mischbaren Lösungsmittel oder Lösungsmittelgemisch.

Für die Verarbeitung des erfindungsgemäss verwendeten photographischen Materials kommt eine Zubereitung in Frage, die

a) eine starke Säure,
b) einen Kationenphasentransferkatalysator, gegebenenfalls
c) einen Liganden, der Silberkomplexe bildet,
d) einen Farbbleichkatalysator und
e) ein Oxidationsschutzmittel, gegebenenfalls
f) ein Oxidationsmittel und gegebenenfalls
g) ein Silberhalogenidlösungsmittel

enthält.

Die erfindungsgemäss verwendenten Triazen- und Kupplungskomponenten sind in Oelen gut, in Wasser dagegen nicht löslich. Beide Komponenten werden in Oel gelöst und in feindisperser Form in eine Silberhalogenidemulsionsschicht oder in eine benachbarte Schicht eingearbeitet. Im allgemeinen

werden diese Zubereitungen so hergestellt, dass sich das Triazen und die Kupplungskomponenten zusammen in der gleichen Oelphase befinden, indem man die beiden Komponenten zusammen im Oel löst und nachher dispergiert, oder auch indem man zwei getrennte Oellösungen herstellt, diese in geeignetem Verhältnis mischt und nachher dispergiert. Es ist aber auch möglich, zwei getrennte Oelphasen mit je einer Komponente zu verwenden, die jede für sich in einer gemeinsamen wässerigen Phase dispergiert werden. Unter dem Einfluss des Phasentransferkatalysators findet auch in diesem Fall ein genügender Stoffaustausch zwischen den Komponenten statt, der bei der Verarbeitung die Farbstoffbildung ermöglicht. Unter einer benachbarten Schicht ist eine beliebige photographische Schicht zu verstehen, wie z. B. eine weitere Silberhalogenidemulsionsschicht oder eine Zwischenschicht. Das Molverhältnis, in dem Triazen- und Kupplungskomponente in Oel gelöst werden, beträgt 4 : 1 bis 1 : 4, vorzugsweise 1 : 1. Oel bedeutet ein Lösungsmittel oder Lösungsmittelgemisch, welches mit Wasser nicht mischbar ist.

Als Oele für die Zubereitung der Lösungen von Triazenen und Kupplungskomponenten eignen sich die üblichen schwerflüchtigen Lösungsmittel, vorzugsweise Ester wie Triphenylphosphat, Trikresylphosphat, Phosphate von Isopropyl- substituierten Phenolen, Dibutylphthalat oder Dioctylphthalat, denen zur Unterstützung des Lösungsvorgangs auch flüchtige Lösungsmittel wie Kohlenwasserstoffe, halogenierte Kohlenwasserstoffe, Aether, Ester, Ketone, höhere Alkohole, wie z. B. Hexan, Methylenchlorid, Brombenzol, Dichlorbenzol, Chloroform, Diisopropyläther, Aethylacetat, Butylacetat, Aethylpropionat und ähnliche gebräuchliche Lösungsmittel zugesetzt werden können, die gegebenenfalls vor oder nach der Emulgierung durch Verdampfen wieder entfernt werden.

Geeignete lichtempfindliche Silberhalogenidemulsionen für das erfindungsgemässe Verfahren enthalten Silberchlorid, Silberbromid oder -jodid oder Gemische dieser Halogenide, wobei der Gehalt an Silberjodid in der Regel 10 Mol-% nicht übersteigt. Zur Herstellung der Emulsionen wird gewöhnlich Gelatine als Schutzkolloid verwendet ; es können aber auch andere wasserlösliche Schutzkolloide wie Polyvinylalkohol oder Polyvinylpyrrolidon verwendet werden. Ferner kann ein Teil der Gelatine durch Dispersionen nicht-wasserlöslicher hochmolekularer Stoffe ersetzt werden. Gebraüchlich ist z. B. die Verwendung von Dispersionspolymerisaten aus α, β-ungesättigten Verbindungen wie Acryl- oder Methacrylsäureestern, Vinylestern und -äthern, Vinylchlorid, Vinylidenchlorid sowie aus anderen Gemischen und Copolymerisaten.

Die lichtempfindlichen Emulsionen können sich in der gleichen Schicht befinden wie die zugeordneten Oelemulsionen, welche die Triazene und Kupplungskomponenten enthalten. Sie können aber auch in einer zu der Schicht mit den Oelemulsionen benachbarten Schicht angeordnet werden, wobei die bildmässige Bleichung der Farbstoffe durch einen Nachbareffekt erfolgt, wie dies aus den deutschen Offenlegungsschriften 2 036 918, 2 132 835 und 2 132 836 bekannt ist.

Als Zubereitungen für die Durchführung der oben beschriebenen Reaktionsfolge eignen sich z. B. solche, wie sie in den deutschen Offenlegungsschriften 1 924 723, 2 036 918, 2 258 076 oder 2 423 814 beschrieben sind. Diese Farbbleichzubereitungen enthalten neben einer starken Säure, einem wasserlöslichen Jodid und mindestens einem Farbbleichkatalysator (meistens einer Diazinverbindung) ein Oxydationsschutzmittel für das Jodid. Solche Zubereitungen bleichen Azofarbstoffe in Gegenwart von metallischem Silber rasch und zuverlässig und sind gut haltbar. Für die nachfolgende Rehalogenierung von überschüssigem Bildsilber muss jedoch ein separates Bad mit einem Oxydationsmittel, wie etwa einem $Fe^{+++}$ oder $Cu^{++}$-Salz verwendet werden. Es kann für die Silberbleichung auch eine Schwermetalfreie Zubereitung, wie sie in der deutschen Offenlegungsschrift 2 530 469 beschrieben wurde, verwendet werden.

Ein Silberfarbbleichverfahren, bei welchem diese Stufe der Silberbleichung mit der nachfolgenden Fixierstufe zu einem einzigen Behandlungsbad zusammengefasst ist, wurde z. B. in der deutschen Offenlegungsschrift 2 309 526 beschrieben. Danach wird als weitere Komponente ein Silberhalogenidlösungsmittel der Zubereitung beigegeben.

Es sind auch Verfahren beschrieben worden, bei denen die Stufen der Farbbleichung und der Silberbleichung in einem einzigen Bad zusammengefasst sind, so z. B. in den deutschen Offenlegungsschriften 2 448 433, 2 547 720 oder 2 831 814. Im weiteren ist ein Verfahren bekannt geworden, bei welchem die drei Schritte (2) Farbbleichung, (3) Silberbleichung und (4) Fixierung in einem einzigen Bad durchgeführt werden, wie dies z. B. in der deutschen Patentschrift 735 672 vorgeschlagen wurde.

Alle diese Varianten des Silberfarbbleichverfahrens lassen sich für die Durchführung der vorliegenden Erfindung verwenden, unter der Voraussetzung, dass zu den üblichen Badbestandteilen noch ein Phasentransferktalysator zugefügt wird, welcher die Fähigkeit besitzt, Kationen, insbesondere Protonen, durch die Phasengrenzen Oel-Wasser zu transportieren.

Die Spaltung des Triazens und seine Kupplung zum Azofarbstoff kann von der nachfolgenden Farbbleichung getrennt werden, indem man das Material mit einem stark sauren, im wesentlichen nur den Phasentransferkatalysator enthaltenden Bad behandelt. Die Farb- und Silberbleichung erfolgen dann in üblicher Weise in ein oder zwei nachfolgenden Behandlungsstufen. Eine solche Reaktionsfolge wird man z. B. dan wählen, wenn der Phasenstransferktalysator mit einem Bestandteil des Farbbleichbades in unerwünschter Weise reagieren sollte.

Unter der Wirkung eines sauren, den Phasentransferkatalysator enthaltenden Verarbeitungsbades können sich die folgenden Vorgänge abspielen :

1. Bildung freien Protons, Diffusion in die Oeltröpfchen unter Einfluss des Phasentransferkatalysators (PTC):

$$nH_3O^{\oplus} \xrightarrow{PTC^{-}} nH_2O + \{nH^{\oplus}\}_{Oel} \qquad (101)$$

Die nachfolgenden Reaktionen verlaufen vollständig innerhalb der Oeltrömpfchen:
2. Spaltung des Triazens in Amin und freies Diazoniumion:

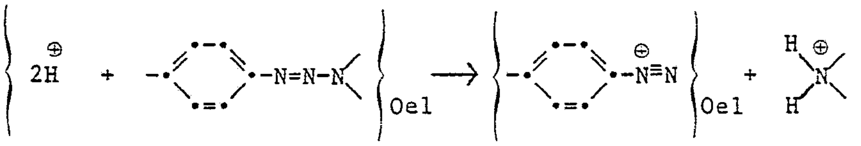

(102)

3. Kupplung zu einem öllöslichen Farbstoff:

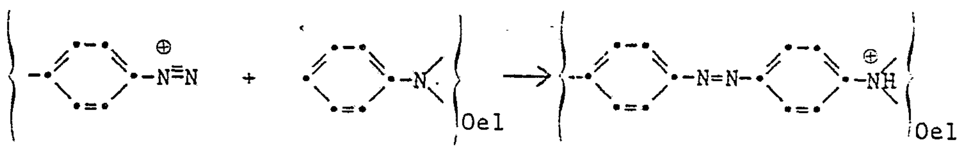

(103)

4. Farbbleichung in Gegenwart von metallischem Silber und eines Bleichkatalysators (K):

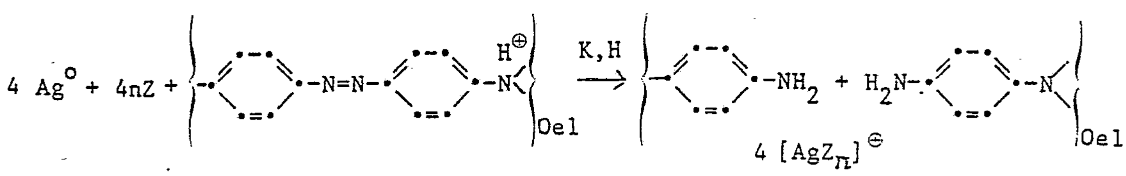


(104)

Die vorliegende Erfindung ist jedoch nicht an diese Reaktionsfolge gebunden.

In Gleichung (104) ist Z ein Ligand, der lösliche Silberkomplexe bilden kann, wie z. B. Jodid, Thioharnstoff, Thiosulfat oder ein substituiertes wasserlösliches Alkyl- oder Arylphosphin. n ist 1, 2, 3 und 4.

Für das erfindungsgemässe Verfahren besonders geeignete Triazene sind in der folgenden Tabelle 1 zusammengestellt.

Tabelle 1
Triazene der Formel

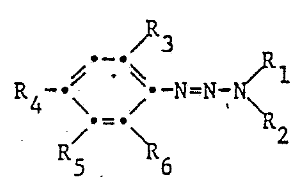

(101)

| $R_1$ | $R_2$ | $R_3$ | $R_4$ | $R_5$ | $R_6$ |
|---|---|---|---|---|---|
| $C_2H_5$ | $C_2H_5$ | H | $n-C_{12}H_{25}$ | H | H |
| $CH_3$ | $CH_3$ | H | $n-C_{12}H_{25}$ | H | H |
| $C_2H_4OH$ | $-C_2H_4OH$ | H | $n-C_{16}H_{33}$ | H | H |
| $C_2H_5$ | $-C_2H_5$ | Br | $n-C_{12}H_{25}$ | H | Br |
| $-CH_2CH_2O-CH_2CH_2-$ | | Br | $n-C_{12}H_{25}$ | H | Br |

Tabelle 1 (Fortsetzung)

| $R_1$ | $R_2$ | $R_3$ | $R_4$ | $R_5$ | $R_6$ |
|---|---|---|---|---|---|
| $C_2H_5$ | $C_2H_5$ | Br | n-$C_{12}H_{25}$ | H | H |
| $C_2H_5$ | $C_2H_5$ | H | $CO_2C_8H_{17}$ | H | H |
| $C_2H_5$ | $C_2H_5$ | H | $CO_2C_{10}H_{21}$ | H | H |
| $C_2H_5$ | $C_2H_5$ | H | $CO_2C_{12}H_{23}$ | H | H |
| $C_2H_5$ | $C_2H_5$ | H | $CO_2C_{16}H_{33}$ | H | H |
| $C_2H_5$ | $C_2H_5$ | H | $CO_2C_{18}H_{37}$ | H | H |
| $C_2H_5$ | $C_2H_5$ | $CO_2C_{16}H_{33}$ | H | H | H |
| $CH_3$ | $CH_3$ | H | H | $CO_2C_{16}H_{33}$ | H |
| $CH_2CH_2OH$ | $CH_2CH_2OH$ | H | H | $CO_2C_{16}H_{33}$ | H |
| $-(CH_2)_4-$ | | H | H | $CO_2C_{16}H_{33}$ | H |
| $-CH_2CH_2OCH_2CH_2-$ | | H | H | $CO_2C_{16}H_{33}$ | H |
| $NCCH_2CH_2-$ | $NCCH_2CH_2-$ | H | H | $CO_2C_{16}H_{33}$ | H |
| $C_6H_5$ | $CH_3$ | H | H | $CO_2C_{16}H_{33}$ | H |
| $C_6H_5$ | $CH_2CH_2OH$ | H | H | $CO_2C_{16}H_{33}$ | H |
| $=C[N(CH_3)_2]_2$ | | H | H | $CO_2C_{16}H_{33}$ | H |
| $C_2H_5$ | $C_2H_5$ | H | H | $OC_{16}H_{33}$ | H |
| $C_2H_5$ | $C_2H_5$ | H | $SO_2C_{16}H_{33}$ | H | H |
| $CH_2CH_2OH$ | $C_2H_4OH$ | Br | $SO_2C_{16}H_{33}$ | H | Br |
| $C_2H_5$ | $C_2H_5$ | H | H | $CO_2C_{16}H_{33}$ | H |
| $C_2H_4OH$ | $C_2H_4OH$ | H | $SO_2N(C_8H_{17})_2$ | H | H |
| $C_2H_4OH$ | $C_2H_4OH$ | CN | $C_5H_{11}$ | $C_6H_{13}$ | CN |
| p-$CH_3$-$C_6H_4$ | $C_2H_4OH$ | $NO_2$ | $CO_2C_{16}H_{33}$ | H | H |
| $C_2H_4CN$ | $C_2H_4CN$ | H | $NO_2$ | $CO_2C_{12}H_{25}$ | H |
| $C_2H_4OH$ | $C_2H_4OH$ | H | $NO_2$ | $CO_2C_{16}H_{33}$ | H |
| $C_2H_4OH$ | $C_2H_4OH$ | CN | $SO_2N(C_8H_{17})_2$ | H | $NO_2$ |
| $C_2H_4OH$ | $C_2H_4OH$ | CN | $C_{12}H_{25}$ | H | CN |
| $C_2H_4CN$ | $C_2H_4CN$ | $NO_2$ | $NO_2$ | $CO_2C_{12}H_{25}$ | H |
| $C_2H_4OH$ | $C_2H_4OH$ | $NO_2$ | $NO_2$ | $CO_2C_{12}H_{25}$ | CN |

Tabelle 1 (Fortsetzung)

| $R_1$ | $R_2$ | $R_3$ | $R_4$ | $R_5$ | $R_6$ |
|---|---|---|---|---|---|
| $C_2H_4OH$ | $C_2H_4OH$ | CN | $SO_2C_{16}H_{33}$ | H | CN |
| $C_2H_4OH$ | $C_2H_4OH$ | $NO_2$ | $NO_2$ | $OC_{16}H_{33}$ | CN |
| $C_2H_5$ | $C_2H_5$ | H | $SO_2N(C_4H_9)_2$ | -CH=CH-CH=CH- | |
| $-CH_2CH_2OCH_2CH_2-$ | | H | $SO_2N(C_4H_9)_2$ | -CH=CH-CH=CH- | |
| $-CH_2CH_2OCH_2CH_2-$ | | H | $NO_2$ | $-CH=CH-C(OCH_2CH_2CH(CH_3)_2)=CH-$ | |
| $-CH_2CH_2OCH_2CH_2-$ | | H | $NO_2$ | $-CH=CH-C(SO_2N(C_4H_9)_2)=CH-$ | |
| $-CH_2CH_2OCH_2CH_2-$ | | CN | $NO_2$ | $-CH=CH-C(SO_2N(C_4H_9)_2)=CH-$ | |
| $C_2H_4OH$ | $C_2H_4OH$ | $NO_2$ | 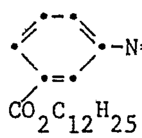 -N=N- | H | H |
| $C_2H_4OH$ | $C_2H_4OH$ | $NO_2$ | $H_{25}C_{12}O_2C-$ 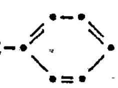 -N=N- | H | H |
| $C_2H_4OH$ | $C_2H_4OH$ | $NO_2$ | $H_{25}C_{12}O_2C-$ 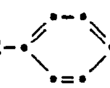 -N=N- | H | CN |
| $C_2H_4OH$ | $C_2H_4OH$ | $NO_2$ | $(C_4H_9)_2NSO_2-$ 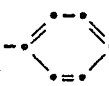 -N=N- | H | H |
| $C_2H_4OH$ | $C_2H_4OH$ | H | $(C_4H_9)_2NSO_2-$ 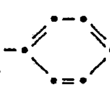 -N=N- | H | H |
| $C_2H_4OH$ | $C_2H_4OH$ | $NO_2$ | $(C_4H_9)_2NSO_2-$ 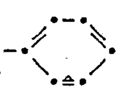 -N=N- | H | CN |

Für das erfindungsgemässe Verfahren besonders geeignete Kupplungskomponenten sind in der folgenden Tabelle 2 angegeben.

(Siehe Tabelle 2 Seite 19 ff.)

Tabelle 2

Kupplungskomponenten der Formel

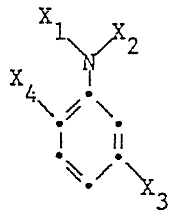

(102)

| $X_1$ | $X_2$ | $X_3$ | $X_4$ |
|---|---|---|---|
| $C_8H_{17}$ | $C_8H_{17}$ | H | H |
| $C_2H_5$ | $C_2H_5$ | $OC_{16}H_{33}$ | H |
| $C_8H_{17}$ | $C_8H_{17}$ | OH | H |
| $C_6H_{13}$ | $C_6H_{13}$ | $OC_4H_9$ | H |
| $C_6H_{13}$ | $C_6H_{13}$ | $OC_2H_4OCH_3$ | H |
| $C_6H_{13}$ | $C_6H_{13}$ | $OCH_3$ | $OCH_3$ |
| $C_8H_{17}$ | $C_8H_{17}$ | $OCH_3$ | $OCH_3$ |
| $C_6H_{13}$ | $C_6H_{13}$ | $OC_2H_4OCH_3$ | $OC_2H_4OCH_3$ |
| $C_{12}H_{23}$ | H | $OCH_3$ | H |
| $C_{12}H_{23}$ | H | $OCH_3$ | $OCH_3$ |
| $C_6H_{13}$ | $C_6H_{13}$ | OH | $OCH_3$ |
| $C_4H_9$ | $C_4H_9$ | $NHCOCH_2CH(CH_3)_2$ | $OCH_3$ |
| $C_6H_{13}$ | $C_6H_{13}$ | $NHCOCH_2CH(CH_3)_2$ | $OCH_3$ |
| $C_6H_{13}$ | $C_6H_{13}$ | $NHCOCH_2CH(CH_3)_2$ | H |
| $C_8H_{17}$ | $C_8H_{17}$ | $NHCOCH_3$ | H |
| $CH_3$ | $CH_3$ | $NHCOC_{11}H_{23}$ | H |
| $C_2H_5$ | $C_2H_5$ | $NHCOC_{11}H_{23}$ | H |
| $C_{12}H_{25}$ | H | $NHCOCH_2CH(CH_3)_2$ | H |
| $C_{10}H_{21}$ | H | $NHCOCH_2CH(CH_3)_2$ | $OCH_3$ |
| $C_2H_4CN$ | H | $NHCOC_{11}H_{23}$ | H |
| $CH(CH_3)CH_2OCH_3$ | H | $NHCOC_{11}H_{23}$ | $OCH_3$ |

Tabelle 2 (Fortsetzung)

| $X_1$ | $X_2$ | $X_3$ | $X_4$ |
|---|---|---|---|
| $CH-CH_2CH(CH_3)_2$<br>$CH_3$ | H | $NHCOC_{11}H_{23}$ | $OCH_3$ |
| $C_6H_{13}$ | $C_6H_{13}$ | $NHCOCH_2CH(CH_3)_2$ | $OC_2H_4OCH_3$ |
| $C_4H_9$ | $C_4H_9$ | $NHCOCH_2CH(CH_3)_2$ | $OCH_2CH_2OCH_3$ |
| $C_2H_4CN$ | H | $NH\overset{O}{\overset{\|}{P}}(OC_8H_{17})_2$ | H |
| $C_2H_4CN$ | H | $NH\overset{O}{\overset{\|}{P}}(OC_6H_{13})_2$ | H |
| $C_2H_4CN$ | H | $NH\overset{O}{\overset{\|}{P}}(OC_6H_5)_2$ | H |
| $C_4H_9$ | $C_4H_9$ | $NH\overset{O}{\overset{\|}{P}}(OC_2H_5)_2$ | $OCH_3$ |
| $C_6H_{13}$ | $C_6H_{13}$ | $NH\overset{O}{\overset{\|}{P}}(OC_2H_5)_2$ | H |
| $C_2H_5$ | $C_2H_5$ | $NH\overset{O}{\overset{\|}{P}}(OC_4H_9)_2$ | $OCH_3$ |
| $C_{12}H_{25}$ | H | $NHCOCH_3$ | H |
| $C_{12}H_{25}$ | H | $NHSO_2CH_3$ | H |
| $C_6H_{13}$ | $C_6H_{13}$ | $NHSO_2CH_3$ | H |
| $C_6H_{13}$ | $C_6H_{13}$ | $NHSO_2CH_3$ | $OCH_3$ |
| $C_2H_4CN$ | H | $NHSO_2C_{12}H_{25}$ | H |
| $CH_2CH_2OH$ | H | $NHCOC_{11}H_{23}$ | H |
| $C_{12}H_{25}$ | H | $NH\overset{O}{\overset{\|}{P}}(OC_2H_5)_2$ | H |

Die erfindungsgemäss verwendeten Triazene können nach gebräuchlichen Methoden aus den entsprechenden Aminen durch Diazotierung, und nachfolgender Kondensation des Diazoniumsalzes mit einem Amin dargestellt werden.

Eine weitere Methode führt von Nitrosoverbindungen durch Kondensation mit einem asymmetrischen Hydrazin ebenfalls zu Triazenen. Die Methode eignet sich vor allem für heterocyclische Triazene. Eine eingehende Beschreibung der gangbaren Synthesemethoden findet sich z. B. in I.G. Laing, in Rodd's Chemistry of Carbon Compounds, 2nd ed. by S. Coffey, Vol. IIIc p. 89-102, 1973 Amsterdam, ferner in C. Süling, « Methoden zur Herstellung und Umwandlung von aromatischen Triazenen und höheren Azahomologen, in Houben-Weyl, Methoden der organischen Chemie, Bd. 10/3, p. 699-743 (1965, G. Thieme, Stuttgart), ausserdem in K.H. Saunders, The Aromatic Diazo Compounds and their Technical Applications, 2nd. ed. London 1949.

Die erfindungsgemäss verwendeten Kupplungskomponenten werden nach üblichen Methoden hergestellt, also z. B. durch Alkylierung, Acylierung, Nitrierung, Reduktion oder Cyanoäthylierung. Kupplungskomponenten mit der Gruppierung

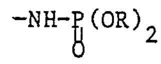

können nach der in der schweizerischen Patentanmeldung 3.340/80-0 angegebenen Methode hergestellt werden.

Beispiel 1 : Herstellung des Triazens der Formel

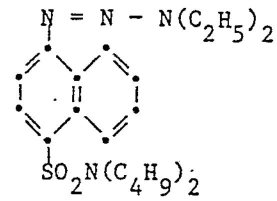

a) 4-Acetamido-naphthalin-1-sulfochlorid wird gemäss deutscher Patentschrift 532 399 hergestellt.

b) 8,5 g (0,03 m) 4-Acetamido-naphthalin-1-sulfochlorid werden portionsweise einer Lösung von 8,1 g (0,063 m) Dibutylamin in 50 ml 1,2-Dichloräthan zugefügt. Das Gemisch wird während 15 Minuten gerührt und schliesslich während 30 Minuten unter Rückfluss gekocht. 50 ml Chloroform werden zugefügt. Dann wird das Gemisch mit 2 %-iger Chlorwasserstoffsäure und mit Wasser gewaschen. Die organische Lösung wird auf Magnesiumsulfat getrocknet, das Lösungsmittel abgedampft und der Rückstand unter Hochvakuum getrocknet.

Man erhält 9,5 g (84 %) 4-Acetamido-naphthalin-1-(N,N-dibutyl-sulfonamid).

c) Ein Gemisch von 9,5 g (0,025 m) 4-Acetamido-naphthalin-1-(N,N-dibutyl-sulfonamid), 55 ml Schwefelsäure 25 % und 80 ml Aethanol wird bei 60 °C 4 Stunden gerührt. Das Gemisch wird in 200 ml Wasser gegossen und mit konzentrierter Ammoniaklösung neutralisiert. Die ölige Schicht wird mit Chloroform extrahiert. Die organische Lösung wird auf Magnesiumsulfat getrocknet und das Lösungsmittel abgedampft. Der ölige Rückstand wird unter Hochvakuum getrocknet.

Man erhält 8,1 g (96 %) öliges 4-Amino-naphthalin-1-(N,N-dibutyl-sulfonamid), welches beim Lagern kristallisiert.

d) 6,7 g (0,02 m) 4-Amino-naphthalin-1-(N,N-dibutyl-sulfonamid) werden in 30 ml Propanol, 12 ml Wasser und 8 g konzentrierter Chlorwasserstoffsäure gelöst. Bei 5 °C werden 1,45 g (0,021 m) Natriumnitrit in 10 ml Wasser zugetropft. Das Gemisch wird 30 Minuten bei 5 °C gerührt. Der Ueberschuss von Nitrit wird mit Sulfaminsäure zerstört. Die Diazoniumsuspension wird portionsweise einer Lösung von 8,8 g (0,12 m) Diäthylamin in 50 ml Propanol und 50 ml Wasser zugefügt. Das Gemisch wird eine Stunde gerührt. Dann werden 100 ml Wasser zugefügt. Die ölige Schicht wird mit Chloroform extrahiert, der Extrakt mit Wasser gewaschen und zum Schluss über Magnesiumsulfat getrocknet. Das Lösungsmittel wird abgedampft und der ölige Rückstand auf Kieselgel mit Methylenchlorid eluiert.

Man erhält 2,1 g (25 %) 1-[4-(N,N-dibutyl-sulfonamido)-1-naphthyl]-3,3-diäthyl-triazen.

Das Kernresonanzspektrum (in $CDCl_3$) und die Elementaranalyse bestätigen die chemische Struktur des Triazens.

Beispiel 2 : Herstellung des Triazens der Formel

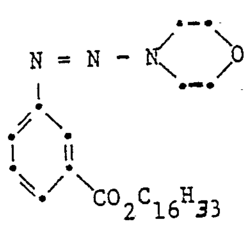

a) Ein Gemisch von 25 g (0,15 m) 3-Nitro-benzoesäure, 40 g (0,165 m) n-Hexadecanol und 3 g-Toluolsulfonsäure in 300 ml Toluol wird unter Rückfluss gekocht. Das gebildete Wasser wird mittels eines Wasserabscheiders entfernt. Die Lösung wird abgekühlt, mit Natriumbicarbonatlösung gewaschen und schliesslich über Magnesiumsulfat getrocknet. Das Lösungsmittel wird zur Trockne abgedampft und der Rückstand in 150 ml Propanol in Gegenwart von Tierkohle umkristallisiert. Man erhält 40 g (68 %) 3-Nitro-benzoesäure-hexadecyl-ester (Smp. 51-53 °C).

b) 39 g (0,099 m) 3-Nitro-benzoesäure-hexadecyl-ester werden in 250 ml 2-Methoxy-äthanol suspendiert, mit 2 g 10 %-igem Palladium-Kohlenstoff-Katalysator versetzt und unter Normaldruck hydriert. Nach beendeter Reaktion wird der Katalysator unter Stickstoff abfiltriert. Das Lösungsmittel wird abgedampft, und das Rohprodukt wird in 150 ml Propanol umkristallisiert.

Man erhält 31 g (87 %) 3-Amino-benzoesäure-hexadecyl-ester (Smp. 52-53 °C).

c) 1,08 g (0,003 m) 3-Amino-benzoesäure-hexadecyl-ester werden in 15 ml Chloroform in Gegenwart von 0,6 g (0,006 m) Methansulfonsäure mit 0,43 g (0,003 m) 90 %-iger Nitrosylschwefelsäure bei 5 °C 60 Minuten diazotiert. Die erhaltene Diazolösung wird zu einer Lösung von 1,75 g (0,020 m) Morpholin in 20 ml Chloroform zugetropft. Das Gemisch wird eine Stunde gerührt. Die organische Lösung wird dreimal mit Wasser gewaschen und über Magnesiumsulfat getrocknet. Das Lösungsmittel wird abgedampft und der Rückstand wird in 25 ml Acetonitril umkristallisiert. Man erhält 1,1 g (80 %) N-(3-hexadecyloxycarbonyl-phenyl-azo)-morpholin (Smp. 40-42 °C). Das Kernresonanzspektrum (in $CDCl_3$) und die Elementaranalyse bestätigen die chemische Struktur des Triazens.

In analoger Weise können ähnliche Verbindungen hergestellt werden, z. B.

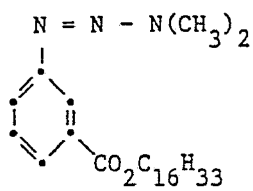
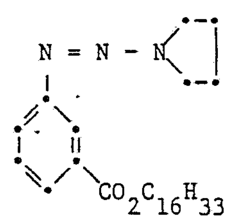
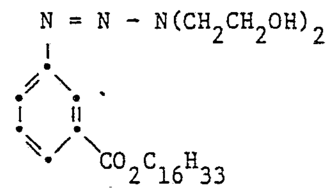

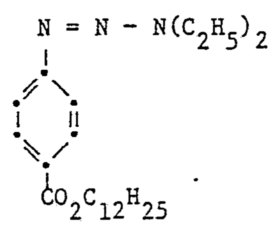
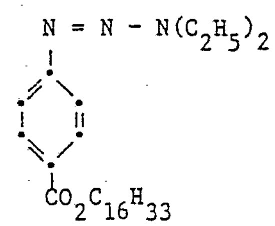
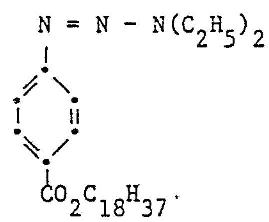

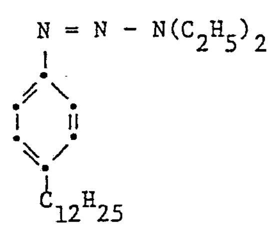
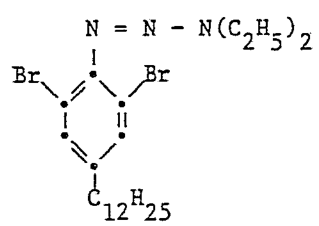
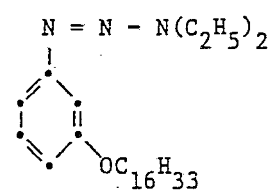

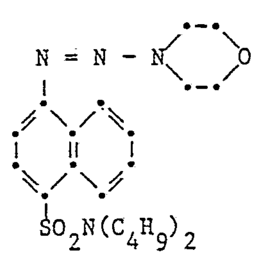

Beispiel 3 : Herstellung des Triazens der Formel

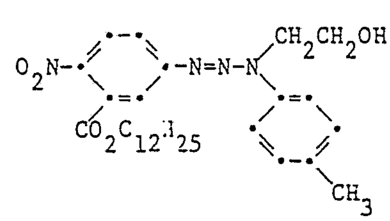

a) 27,3 g (0,15 m) 5-Amino-2-nitro-benzoesäure werden in 100 ml N,N-Dimethylformamid unter Stickstoff gelöst. 28,4 g (0,158 m) 30 %-iger Natriummethylat-Lösung werden zugefügt. Das Gemisch wird während 15 Minuten gerührt. 100 mg Natrium-Jodid werden zugefügt, und schliesslich werden 39,2 g (0,157 m) Bromdodecan zugetropft. Das Gemisch wird vier Stunden bei 150 °C erhitzt (ein wenig Methanol abdestilliert). Das Reaktionsgemisch wird in Wasser eingerührt. Der Niederschlag wird abgesaugt, mit Wasser, dann mit Methanol gewaschen und unter Vakuum getrocknet. Das Rohprodukt wird in Gegenwart von Tierkohle in 100 ml Hexan und dann in 60 ml Methanol umkristallisiert. Man erhält 26,8 g (51 %) 5-Amino-2-nitro-benzoesäure-dodecyl-ester (Smp. 58-59 °C).

b) 3,5 g (0,01 m) 5-Amino-2-nitro-benzoesäure-dodecyl-ester werden in einem Gemisch von 20 ml Essigsäure und 7,0 g (0,04 m) 50 %-iger Borfluorwasserstoffsäure-Lösung suspendiert. Bei Raumtemperatur werden 1,3 g (0,011 m) Isopentylnitrit zugetropft. Das Gemisch wird 30 Minuten gerührt. Der weisse Niederschlag wird abgesaugt, mit Wasser gewaschen und gut abgepresst. Man erhält 6 g feuchtes 3-(Dodecyloxycarbonyl)-4-nitro-benzol-diazonium-tetrafluoroborat.

c) 3,0 g (ca. 0,005 m) feuchtes 3-(Dodecyloxycarbonyl)-4-nitro-benzoldiazonium-tetrafluoroborat werden portionsweise einem Gemisch von 0,76 g (0,005 m) N-(2-Hydroxyäthyl)-toluidin, 0,6 g (0,005 5 m) Natrium-carbonat und 30 ml Methanol zugefügt. Das Reaktionsgemisch wird in Wasser gegossen, der sich bildende Niederschlag abgesaugt, mit Wasser gewaschen und unter Vakuum bei 50 °C getrocknet. Das Rohprodukt wird in 20 ml Hexan in Gegenwart von Tierkohle umkristallisiert. Man erhält 1,25 g (49 %) 1-(3-Dodecyloxycarbonyl-4-nitro-phenyl)-3-(2-hydroxyäthyl)-3-(p-methyl-phenyl)-triazen (Smp. 64-66 °C).

Das Kernresonanzspektrum (in $CDCl_3$) und die Elementaranalyse bestätigen die chemische Struktur des Triazens.

Beispiel 4 : Herstellung des Triazens der Formel

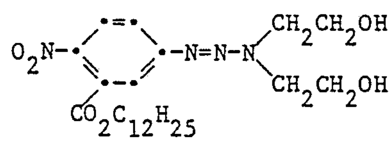

3,0 g (ca. 0,005 m) feuchtes 3-(Dodecyloxycarbonyl)-4-nitro-benzol-diazonium-tetrafluoroborat werden portionsweise einer Lösung von 2,63 g (0,025 m) Diäthanolamin in 30 ml Methanol hinzugefügt. Die Lösung wird 30 Minuten gerührt und schliesslich in Wasser eingerührt. Die ölige Schicht wird mit Chloroform extrahiert, der Extrakt mit Wasser gewaschen und zum Schluss über Magnesiumsulfat getrocknet. Das Lösungsmittel wird abgedampft, und der ölige Rückstand auf Kieselgel zuerst mit einem Gemisch von Chloroform (95 Teile) und Essigsäure-äthyl-ester (5 Teile) und schliesslich mit einem Gemisch von Chloroform (95 Teile) und Methanol (5 Teile) eluiert. Man erhält 2,1 g (90 %) 1-(3-Dodecylocycarbonyl-4-nitro-phenyl)-3,3-bis-(2-hydroxyäthyl)-triazen. Das Kernresonanzspektrum (in $CDCl_3$) und die Elementaranalyse bestätigen die chemische Struktur des Triazens.

Beispiel 5 : Herstellung des Triazens der Formel

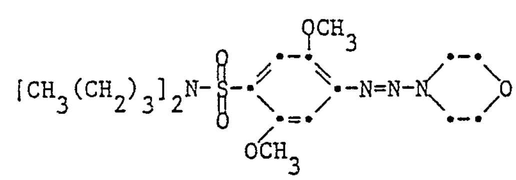

a) 19,5 g (0,1 m) 2,5-Dimethoxy-acetanilid werden bei 0 °C zu 250 ml Chlorosulfonsäure portionsweise zugegeben. Das Gemisch wird während 30 Minuten bei 0 °C und dann bei 40 °C während 3 Stunden gerührt. Das Gemisch wird danach in 1 500 ml Eiswasser gegossen. Die organische Phase wird mit Chloroform extrahiert. Das Extrakt wird über Magnesiumsulfat getrocknet und das Lösungsmittel abgedampft. Das Rohprodukt wird in einem Gemisch von 200 ml Hexan und 150 ml Essigsäure-äthylester umkristallisiert.

Man erhält 17 g (57,6 %) 2,5-Dimethoxy-acetanilide-4-sulfonsäurechlorid (Smp. 151-152 °C).

b) 6,5 g (0,05 m) Dibutylamin werden in 50 ml Methylenchlorid gelöst, 5,9 g (0,02 m) 2,5-Dimethoxy-acetanilide-4-sulfonsäurechlorid werden portionsweise hinzugefügt. Das Reaktionsgemisch wird während einer Stunde unter Rückfluss gekocht. Nach dem Abkühlen wird die Lösung mit verdünnter wässriger Salzsäure gewaschen und über Magnesiumsulfat getrocknet. Das Lösungsmittel wird abgedampft. Der Rückstand wird mit 60 ml Aethanol und 30 ml konzentrierter Salzsäure gemischt und bei

60 °C während 2 Stunden erhitzt. Das Gemisch wird mit 200 ml Wasser verdünnt und mit Natronlauge neutralisiert. Die ölige Schicht wird mit Methylenchlorid extrahiert und über Magnesiumsulfat getrocknet. Nach Entfernen des Lösungsmittels wird das ölige Produkt im Hochvakuum getrocknet. Man erhält 6,6 g (97 %) 2,5-Dimethoxy-4-(N,N-dibutylsulfonamido)-anilin (Smp. 44-45 °C).

c) 3,1 g (0,009 m) 2,5-Dimethoxy-4-(N,N-Dibutylsulfonamido)-anilin werden in einem Gemisch von 20 ml Essigsäure und 6,3 g (0,036 m) 50 %-iger wässriger Tetrafluoroborwasserstoffsäure gelöst. Bei Raumtemperatur werden 1,17 g (0,010 m) Isopentylnitrit zugetropft. Das Gemisch wird 30 Minuten gerührt. Die erhaltene Diazoniumlösung wird bei 10 °C zu einem Gemisch von 61 g Morpholin, 200 ml Chloroform und 200 ml Wasser getropft. Das Reaktionsgemisch wird bei 10 °C während einer Stunde gerührt. Die organische Schicht wird abgetrennt und zweimal mit Wasser gewaschen. Das Lösungsmittel wird abgedampft und das ölige Rohprodukt auf Kieselgel mit einem Gemisch von Trichloräthylen (85 Teile) und Essigsäureäthylester (15 Teile) eluiert.

Man erhält 3,9 g (98 %) N-[12,5-Dimethoxy-4-(N,N-dibutylsulfonamido)-phenyl-azo]-morpholin in Form eines Oels.

Das Kernresonanzspektrum (in $CDCl_3$) und die Elementaranalyse bestätigen die chemische Struktur des Triazens.

Beispiel 6 : Herstellung des Triazens der Formel

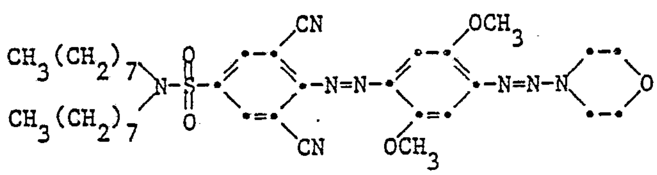

a) 5,4 g (0,01 m) 2,6-Dibrom-4-(N,N-dioctylsulfonamido)-anilin werden in 40 ml Chloroform in Gegenwart 2 g (0,021 m) Methansulfonsäure gelöst. Bei 5 °C werden 1,4 g (0,01 m) 93 %ige Nitrosylschwefelsäure zugefügt. Das Gemisch wird während 30 Minuten gerührt.

Die erhaltene Diazonium-lösung wird bei 5 °C zu einem Gemisch aus 1,7 g (0,011 m) 2,5-Dimethoxy-anilin, 1 g Methansulfonsäure, 40 ml Acetonitril und 20 ml Chloroform gegeben. 4,1 g (0,05 m) Natrium-acetat werden portionsweise hinzugefügt. Das Reaktionsgemisch wird eine Stunde bei 5 °C gerührt, danach in Wasser gegossen und mit Chloroform extrahiert. Die organische Phase wird mit Wasser gewaschen und über Magnesiumsulfat getrocknet. Nach Entfernen des Lösungsmittels erhält man 7,2 g (100 %) des Farbstoffes der Formel

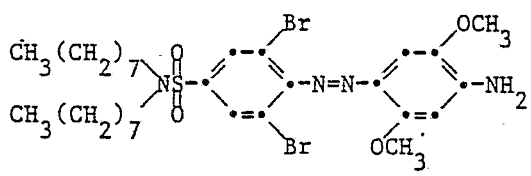

($\lambda_{max.}^{CHCH_3}$ = 424 nm; $\varepsilon$ = 17 700)

b) 7,0 g (0,097 m) dieses Farbstoffes werden in 30 ml N-Methyl-pyrrolidon gelöst. Dazu gibt man 5 g Kupfer (I)-cyanid. Das Gemisch wird bei 100 °C während 30 Minuten erhitzt. Nach dem Abkühlen wird das Reaktionsgemisch in einer Natriumrhodanidlösung während 15 Minuten gerührt. Der Niederschlag wird abgesaugt und mit Wasser gewaschen. Das Rohprodukt wird in 200 ml Chloroform gelöst und mit Magnesiumsulfat und Kieselgel gerührt.

Die Suspension wird abfiltriert und das Lösungsmittel wird abgedampft. Man erhält 5,4 g (91 %) des Farbstoffes der Formel

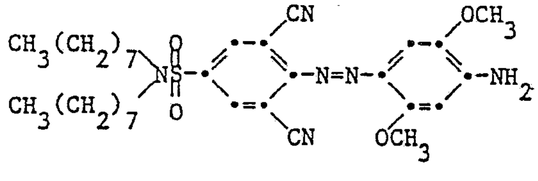

($\lambda_{max}^{CHCl_3}$= 538, $\varepsilon$ = 30 200)

c) 0,6 g (0,001 m) dieses Farbstoffes werden in 10 ml Methylenchlorid in Gegenwart von 0,2 g (0,002 m) Methansulfonsäure gelöst. 0,25 g (0,002 m) Isopentylnitrit werden zugefügt. Das Gemisch wird während 90 Minuten bei Raumtemperatur gerührt.

Die erhaltene Diazoniumlösung wird einem Gemisch von 0,45 g (0,005 m) Morpholin und 10 ml Methylenchlorid zugetropft. Das Gemisch wird eine Stunde gerührt und in 50 ml Wasser gegossen. Die organische Phase wird abgetrennt, dreimal mit Wasser gewaschen und über Magnesiumsulfat getrocknet. Das Lösungsmittel wird abgedampft und der Rückstand auf Kieselgel mit einem Gemisch von Trichloräthylen (8 Teile) und Essigsäure-aethyl-ester (2 Teile) eluiert.

Man erhält 350 mg (49,4 %) des Triazens der Formel

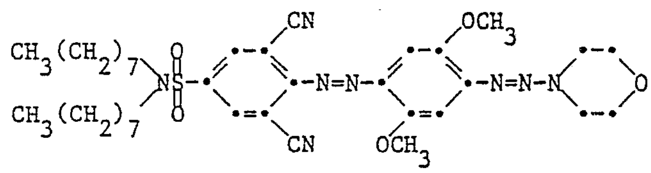

Analog wird das Triazen der Formel

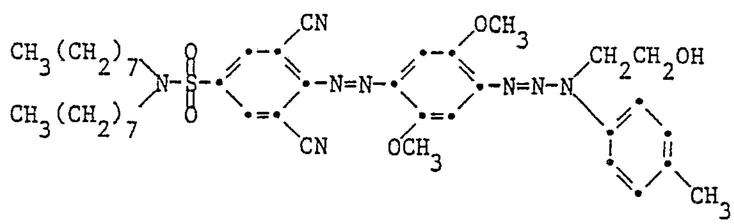

hergestellt. (Ausbeute : 32 %).

Beispiel 7 : Herstellung des Triazens der Formel

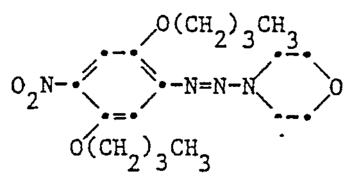

2,82 g (0,01 m) 2,5-Dibutoxy-4-nitro-anilin werden in einem Gemisch von 45 ml Essigsäure und 7,0 g (0,04 m) 50 %-iger wässriger Tetrafluoroborwasserstoffsäure suspendiert. 1,4 g (0,012 m) Isopentylnitrit werden zugetropft. Das Gemisch wird eine Stunde gerührt. Nach Zugabe von 10 ml Wasser wird der entstandene Niederschlag mit Wasser gewaschen und einem Gemisch von 6 g Morpholin und 30 ml Methanol portionsweise zugefügt. Die Suspension wird 30 Minuten gerührt und in 30 ml Wasser eingesetzt. Der Niederschlag wird abgesaugt. Das Rohprodukt wird in 25 ml Methanol umkristallisiert.

Man erhält 3,1 g (81,4 %) N-(2,5-Dibutoxy-4-nitro-phenyl-azo)-morpholin. (Smp. 79-81 °C).

Das Kernresonanzspektrum (in $CDCl_3$) und die Elementaranalyse bestätigen die chemische Struktur des Triazens.

Beispiel 8 : Herstellung der Kupplungskomponente der Formel

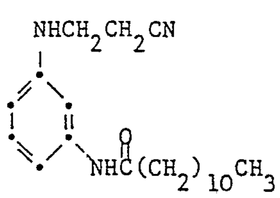

a) 21,6 g (0,2 mol) 1,3-Phenylendiamin, 10,6 g (0,2 mol) Acrylnitril, 5 g Essigsäure und 100 ml Wasser werden 6 Stunden bei 100 °C erhitzt. Das Reaktionsgemisch wird mit Natriumcarbonat neutralisiert und mit Methylenchlorid extrahiert. Der Extrakt wird über Magnesiumsulfat getrocknet. Das Lösungsmittel wird abgedampft, und der verbleibende ölige Rückstand unter Hochvakuum destilliert. Man erhält 16,2 g (50,2 %) 3-[N-(2-Cyanoäthyl)-amino]-anilin (Sdp. 160-164 °C/0,1 Torr).

b) 0,97 g (0,006 mol) 3-[N-(2-Cyanoäthyl)-amino]-anilin werden in 10 ml trockenen Toluol und 5 ml trockenem Dioxan gelöst. Dieser Lösung werden 0,67 g (0,006 6 mol) Triäthylamin zugefügt. 1,34 g (0,006 1 mol) Laurylsäurechlorid werden zugetropft. Das Reaktionsgemisch wird 3 Stunden gerührt. Der

Niederschlag von Triäthylamin-Hydrochlorid wird abfiltriert, und das Filtrat wird zur Trockne abgedampft. Der Rückstand wird zweimal in einem Gemisch von 10 ml Hexan und 2,5 ml Essigsäure-äthyl-ester umkristallisiert. Man erhält 0,9 g (44 %) 3-(Laurylamido)-N-(2-cyanoäthyl)-anilin (Smp. 101-103 °C). Das Kernresonanzspektrum (in $CDCl_3$) und die Elementaranalysebestätigen die chemische Struktur der Kupplungskomponente.

Beispiel 9 : Herstellung der Kupplungskomponente der Formel

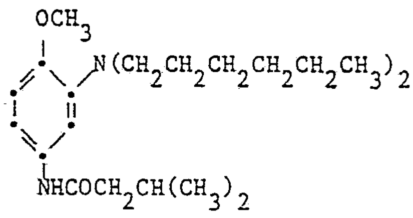

a) 84 g (0,5 m) 4-Amino-2-nitro-anisol werden in 500 ml trockenem Dioxan gelöst. 65 g (0,65 m) Calcium-carbonat werden zugefügt. Bei 15 °C werden sodann 66,3 g (0,55 m) Isovaleriansäurechlorid zugetropft. Die Suspension wird zwei Stunden bei Raumtemperatur gerührt und dann abgesaugt ; der feste Rückstand wird noch mit 300 ml Dioxan extrahiert ; die organischen Lösungen werden gesammelt und zur Trockne eingedampft. Das Rohprodukt wird in 250 ml Toluol in Gegenwart von Tierkohle umkristallisiert. Man erhält 86,9 g (69 %) 4-Isovalerylamido-2-nitro-anisol (Smp. 100-101 °C).

b) 86 g (0,34 m) 4-Isovalerylamido-2-nitro-anisol werden in 500 ml 2-Methoxy-äthanol gelöst, mit 2 g 10 %-igem Palladium-Kohlenstoff-Katalysator versetzt und unter Normaldruck hydriert. Nach beendeter Reaktion wird der Katalysator unter Stickstoff abfiltriert. Das Lösungsmittel wird abgedampft und das Rohprodukt wird in 300 ml Toluol in Gegenwart von Tierkohle umkristallisiert. Man erhält 48 g (64 %) 2-Amino-4-isovalerylamido-anisol (Smp. 111-113 °C).

c) unter Stickstoff werden 13,35 g (0,06 m) 2-Amino-4-isovalerylamidoanisol in 200 ml N,N-Dimethylacetamid gelöst. 10 g (0,25 m) Magnesiumoxid und 33 g (0,2 m) Hexylbromid werden zugefügt. Das Gemisch wird sechs Stunden bei 130 °C erhitzt und schliesslich abgekühlt. Die Suspension wird abgesaugt und das Filtrat wird in 100 ml Eiswasser gegossen. Das ölige Produkt wird mit Hexan extrahiert, die organische Lösung auf Magnesiumsulfat getrocknet und zuletzt das Lösungsmittel abgedampft. Der ölige Rückstand wird auf Kieselgel mit Chloroform eluiert. Man erhält 20,9 g (89 %) 2-Methoxy-5-isovalerylamido-N,N-dihexylanilin (Smp. 26-28 °C). Das Kernresonanzspektrum (in $CDCl_3$) und die Elementaranalyse bestätigen die chemische Struktur der Kupplungskomponente.

Andere Verbindungen können in analoger Weise hergestellt werden, z. B. :

2-Methoxy-5-isovalerylamido-N,N-dibutyl-anilin
(Ausbeute : 80 %) (Smp. 88-90 °C (aus Acetonitril)).
3-Isovalerylamido-N,N-dihexyl-anilin
(Ausbeute : 89 %) (Smp. 38-40 °C)
3-Isovalerylamido-N-decyl-anilin
(Ausbeute : 30 %) (Smp. 58-60 °C (aus Hexan-Toluol 3 : 1)).
3-Isovalerylamido-N-dodecyl-anilin
(Ausbeute : 56 %) (Smp. 71-73 °C (aus Hexan-Toluol 9 : 1))
2-Methoxy-5-isovalerylamido-N-decyl-anilin
(Ausbeute : 44 %) (Smp. 68-70 °C (aus Hexan-Toluol 7 : 3))

Beispiel 10 : Herstellung der Kupplungskomponente der Formel

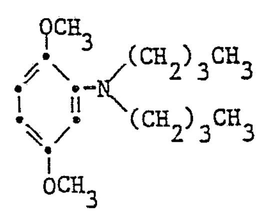

15,3 g (0,1 m) 2,5-Dimethoxy-anilin, 54,8 g (0,4 m) Butylbromid, 20,0 g (0,5 m) Magnesium-oxid und 260 ml N,N-Dimethyl-acetamid werden während 6 Stunden unter Stickstoff bei 130 °C gehalten. Das Reaktionsgemisch wird abgekühlt und der Niederschlag abgesaugt. Das Filtrat wird in 600 ml Eiswasser gegossen. Das ölige Produkt wird mit Aether extrahiert. Die organische Phase wird dreimal mit Wasser

gewaschen und mit Magnesiumsulfat getrocknet. Das Lösungsmittel wird abgedampft und der Rückstand wird unter Vakuum destilliert.

Man erhält 19,7 g (74,3 %) N,N-Dibutyl-2,5-dimethoxyanilin (Sdp. 114-118 °C/0,17 Torr).

Das Kernresonanzspektrum (in $CDCl_3$) und die Elementaranalyse bestätigen die chemische Struktur der Kupplungskomponente.

Beispiel 11 : Herstellung der Kupplungskomponente der Formel

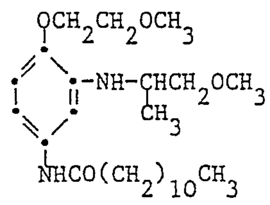

a) 23,8 g 3-Amino-4-(2-methoxy-äthoxy)-propionanilid (hergestellt gemäss Europäischer Patentschrift 0011048) werden mit 18,9 g Methoxyaceton kondensiert und gleichzeitig in Gegenwart eines Hydrierkatalysators (Platin auf Kohle) mit Wasserstoff reduziert. 28,4 g 2-(2-Methoxy-äthoxy)-5-propionamido-N-(2-methoxyisopropyl)-anilin werden in 91 % Ausbeute erhalten.

b) 9 g 2-(2-Methoxy-äthoxy)-5-propionamido-N-(2-methoxy-isopropyl)-anilin werden in 100 ml eines Gemisches von Methanol und konz. Salzsäure erhitzt. Die Lösung wird in Wasser eingerührt und mit 55 ml 30 %-iger Natronlauge neutralisiert. Die ölige Schicht wird mit Chloroform extrahiert. Der Extrakt wird über Kaliumcarbonat getrocknet und das Lösungsmittel vollständig abgedampft. Der Rückstand wird in 100 ml Dioxan gelöst. Dieser Lösung werden 1,5 g Kaliumcarbonat und schliesslich 6,7 g Laurylsäurechlorid zugefügt. Das Reaktionsgemisch wird 3 Stunden gerührt, unter Rückfluss gekocht und heiss filtriert. Das Produkt fällt beim Abkühlen aus und wird abgesaugt. Der Niederschlag wird in Essigsäure-äthyl-ester umkristallisiert. Man erhält 6,3 g 2-(2-Methoxy-äthoxy)-5-laurylamido-N-(2-methoxy-isopropyl)-anilin in 50 % Ausbeute (Smp. 148-151 °C).

Das Kernresonanzspektrum (in $CDCl_3$) und die Elementaranalyse bestätigen die chemische Struktur der Kupplungskomponente.

Beispiel 12 : Herstellung der Kupplungskomponente der Formel

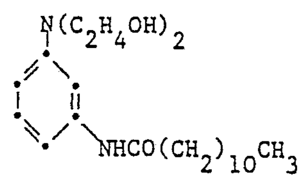

a) 20 g (0,145 m) 3-Nitro-anilin werden in 180 ml trockenen Dioxan gelöst, 17,4 g (0,174 m) Calciumcarbonat werden hinzugefügt. Bei 15 °C werden sodann 35 g (0,16 m) Laurylsäurechlorid in 20 ml Dioxan zugetropft. Die Suspension wird zwei Stunden bei Raumtemperatur gerührt und dann abgesaugt. Der feste Rückstand wird mit 100 ml Dioxan extrahiert und die erhaltene organische Lösung zur Trockne eingedampft. Das Produkt wird bei 50 °C unter Vakuum getrocknet.

Man erhält 45,8 g (98,5 %) 3-Nitro-laurylanilid (Smp. 69-70 °C).

b) 45,5 g (0,142 m) 3-Nitro-laurylanilid werden in 250 ml 2-Methoxyäthanol gelöst, mit 2 g eines 10 %-igen Hydrierkatalysators (Palladium auf Kohle) versetzt und unter Normaldruck hydriert. Nach beendeter Reaktion wird das Gemisch auf 90 °C erhitzt und der Katalysator unter Stickstoff heiss abfiltriert. Das Gemisch wird abgekühlt und das Produkt fällt aus. Der Niederschlag wird abgesaugt und getrocknet.

Man erhält 38,6 g (93,5 %) 3-Amino-laurylanilid (Smp. 90-91 °C).

c) 2,9 g (0,01 m) 3-Amino-laurylanilid und 0,9 g (0,021 m) Aethylenoxid in 25 ml Xylol werden bei 140 °C in einem Druckrohr während einer Nacht erhitzt. Das heisse Gemisch wird abfiltriert und dann bei 0 °C gekühlt. Der Niederschlag wird abgesaugt und getrocknet.

Man erhält 2,25 g (60 %) 3-N,N-bis-(hydroxyäthyl)-amino-laurylanilid (Smp. 96-98 °C).

Das Kernresonanzspektrum (in $CDCl_3$) und die Elementaranalyse bestätigen die chemische Struktur der Kupplungskomponente.

Beispiel 13 : 79,5 mg des Triazens der Formel

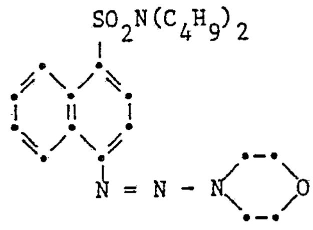

und 63,6 mg des Kupplers der Formel

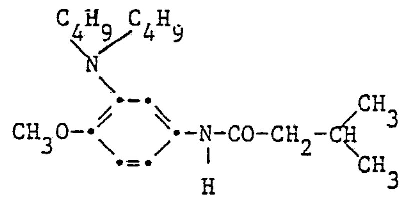

werden in 3 ml eines Gemisches von 9 Gewichtsteilen Aethylacetat und 1 Teil Trikresylphosphat gelöst. Diese ölige Lösung wird in eine Mischung aus 10,3 ml 6 %-iger Gelatinelösung und 2,0 ml einer 3 %-igen wässrigen Lösung von Dibutylnaphthalinsulfonsäure (Nekal BX®) unter Rühren eingetragen. Durch Behandlung mit Ultraschall wird die ölige Lösung in dem wässrigen Gemisch fein emulgiert.

2,5 ml dieser Emulsion werden nun mit 1,7 ml einer wässrigen Gelatinelösung, 5 ml Wasser, 0,8 ml einer Silberbromojodidemulsion, welche 22 g Silber pro kg enthält und 1 ml einer 1 %-igen Lösung der Verbindung

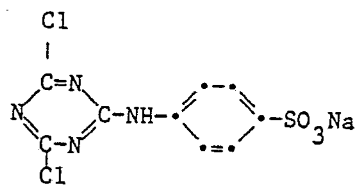

vermischt. Das Gemisch wird in gleichmässiger Schicht auf einen opaken Cellulosetriacetatträger vergossen, derart, dass nach dem Trocknen der Schicht auf einen $cm^2$ je 0,12 Mikromol Kuppler und Triazen und 0,7 Mikromol Silber entfallen.

Das getrocknete Material wird hinter einem Stufenkeil belichtet und anschliessend bei einer Temperatur von 32 °C wie folgt weiterverarbeitet :

1. Entwicklung : 1,5 Minuten

| | |
|---|---|
| Hydrochinon | 6,0 g |
| Phenidon (1-Phenyl-3-pyrazolidinon) | 0,25 g |
| Kaliumsulfit | 50 g |
| Aethylendiamin-tetraessigsäure (Na-Salz) | 2 g |
| Natriummetaborat | 7,1 g |
| Kaliumbromid | 1,0 |
| Benzotriazol | 0,1 g |
| Wasser ad | 1 Liter |

2. Wässerung : 0,5 Minuten

3. Kombinierte Farbbildung, Farb- und Silberbleichung 6 Minuten

| | |
|---|---|
| Trichloressigsäure | 70 g |
| Schwefelsäure 98 % | 15,5 g |
| Ascorbinsäure | 0,30 g |
| Natriumjodid | 2,8 |
| 4-Nitrophenol-2-sulfosäure | 1,8 g |
| 6-Methoxy-2,3-dimethylchinoxalin | 0,3 g |
| Wasser ad | 1 Liter |

4. Wässerung : 0,5 Minuten

5. Fixierung : 4 Minuten

| | |
|---|---|
| Ammoniumthiosulfat | 200 g |
| Kaliummetabisulfit | 25 g |
| Kaliumhydroxid 85 % | 11 g |
| Wasser ad | 1 Liter |

6. Wässerung : 4 Minuten

Man erhält nach dem Trocknen ein purpurfarbenes Keilpositiv mit einer Minimaldichte von 0,02 und einer Maximaldichte von 2,25 ($\lambda_{max.}$ = 540 nm)

Beispiel 14

Beispiel 13 wird wiederholt, wobei jedoch 44,5 mg des Triazens der Formel

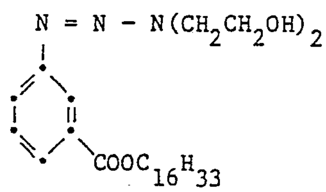

und 46,5 mg Kupplungskomponente der Formel

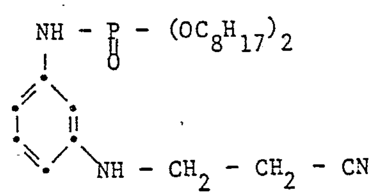

verwendet werden. Die Herstellung der Lösung im Aethylacetat-Trikesylphosphat-Gemisch und die Emulgierung in Gelatinelösung erfolgen wie in Beispiel 13.

Zur Herstellung der Beschichtungslösung verwendet man Gelatinelösung und Silberhalogenidemulsion in gleichen Mengenverhältnissen wie in Beispiel 13. Nach Beschichtung eines Cellulosetriacetatträgers und Trocknung, wie in Beispiel 13 erhält man eine Schicht, die pro $cm^2$ je 0,065 Mikromol Triazen und Kupplungskomponente und 0,7 Mikromol Silber enthält.

Das getrocknete Material wird hinter einem Stufenkeil belichtet und anschliessend bei einer Temperatur von 32 °C wie folgt verarbeitet :

1. Entwicklung : 1,5 Minuten
Gleich wie in Beispiel 13

2. Wässerung : 0,5 Minuten

3. Farbbildung : 6 Minuten
Perchlorsäure 0,5 molar = 50 g/Liter

4. Kombinierte Farb- und Silberbleichung : 5 Minuten

| | |
|---|---|
| Schwefelsäure 98 % | 51,5 g |
| Natriumjodid | 9,0 g |
| 4-Nitro-2-phenolsulfosäure (Dinatriumsalz) | 6,0 g |
| 6-Methoxy-2,3-dimethylchinoxalin | 1,0 g |
| Ascorbinsäure | 1,0 g |
| Wasser ad | 1 Liter |

5. Wässerung : 0,5 Minuten

6. Fixierbad : 4 Minuten
Gleich wie in Beispiel 13

7. Wässerung : 4 Minuten

Man erhält eine brillante gelbe Positivkopie des Keils mit einer Minimaldichte von 0,03 und einer Maximaldichte von 1,05 ($\lambda_{max.}$ = 437 nm)

Ein ähnliches Resultat erhält man, wenn man die Farbbildung und die Farb- und Silberbleichung wie beim Beispiel 13 in ein und demselben Bad durchführt.

Beispiel 15

Gemäss Beispiel 13 wird ein Träger mit einer Schicht der folgenden Zusammensetzung versehen :

| | |
|---|---|
| Triazen gemäss Beispiel 14 | 0,432 mmol/$m^2$ |
| Kupplungskomponente der Formel | |

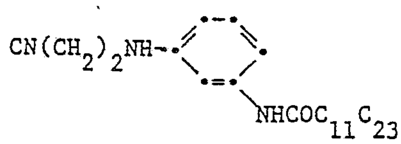

| | |
|---|---|
| Silberbromoiodid mit einem Silbergehalt von | 2,200 mmol/$m^2$ |
| Trikresylphosphat | 0,715 g/$m^2$ |
| Gelatine | 2,015 g/$m^2$ |

Dieses Material wird hinter einem Stufenkeil mit blauem Licht (20 lux. sec) belichtet und bei 32 °C wie folgt verarbeitet :

1. Entwicklung 1 Minute
Zusammensetzung des Entwicklerbades wie in Beispiel 13

2. Wässerung 0,5 Minute

3. Kombinierte Farbbildung, Farb- und Silberbleichung und Fixierung 3 Min.

| | |
|---|---|
| Schwefelsäure (98 %) | 15,5 g |
| Natriumiodid | 2,7 g |
| 6-Methoxy-2,3-dimethyl-chinoxalin | 0,3 g |
| Thioharnstoff | 75,0 g |
| Ascorbinsäure | 1,0 g |
| Trichloressigsäure | 70,0 g |
| Wasser ad | 1 Liter |

4. Wässerung 3 Minuten

Man erhält nach dem Trocknen ein gelbes Keilpositiv mit einer Minimaldichte von 0,02, einer Blaureflexionsdichte von 1,50 und einem Restsilbergehalt von weniger als 0,005 g/$m^2$.

Beispiel 16 : Ein photographisches Mehrschichtenmaterial (Tripack) wird hergestellt, indem man auf einen Triacetatträger nacheinander die folgenden Schichten aufbringt :

a) eine rotempfindliche Silberbromidemulsionsschicht mit einem Giessgewicht von 2 g/m Gelatine, 0,5 g/$m^2$ Silber und 112 mg/$m^2$ des Cyanfarbstoffes der Formel

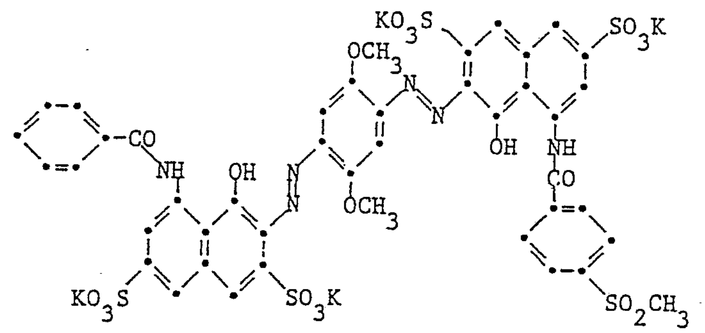

b) eine Gelatinfezwischenschicht mit einem Giessgewicht von 2,5 g/m$^2$,

c) eine grünempfindliche Schicht gemäss Beispiel 13,

d) eine Gelatinezwischenschicht gemäss (b),

e) eine blauempfindliche Schicht gemäss 15, wobei jedoch der Gehalt an Silber, Triazen und Kupplungskomponente um den Faktor 1,33 erhöht ist, und

f) eine Gelatineschutzschicht mit einem Giessgewicht von 1,25 g/m$^2$.

Das Material wird getrocknet und durch einen Stufenkeil mit blauem (20 Lux. Sekunden), grünem (20 Lux. Sekunden) und rotem Licht (30 Lux. Sekunden) belichtet. Die Verarbeitung des belichteten Materials erfolgt gemäss Beispiel 13. Man erhält einen Graukeil mit ausgewogenem Farbgleichgewicht, worin $D_{max}$ 2,1 und $D_{min}$ 0,05 beträgt.

**Patentansprüche**

1. Verfahren zur Herstellung photographischer Farbbilder nach dem Silberfarbbleichverfahren durch Belichtung, Silberentwicklung, Farbbildung, Farbbleichung, Silberbleichung und Fixierung eines photographischen Materials, welches auf einem transparenten oder opaken Träger mindestens eine Schicht mit einer lichtempfindlichen Silberhalogenidemulsion enthält, wobei die Farbbildung gegebenenfalls mit der Farbbleichung, Silberbleichung und Fixierung, und die Silberbleichung gegebenenfalls mit der Farbbleichung und/oder der Fixierung in einem einzigen Verarbeitungsbad gleichzeitig durchgeführt werden kann, dadurch gekennzeichnet, dass die lichtempfindliche(n) Silberhalogenidemulsionsschicht(en) oder (je) eine dieser Silberhalogenidemulsionsschicht(en) benachbarte Schicht ein öllösliches Triazen der Formel

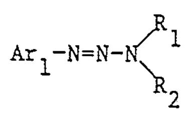 (1) oder 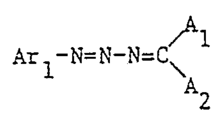 (2)

und eine öllösliche Kupplungskomponente der Formel

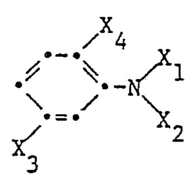

in Oel dispergiert enthält (enthalten), worin

$Ar_1$ gegebenenfalls substituiertes Aryl oder ein gegebenenfalls substituierter, aromatischer, heterocyclischer Rest,

$R_1$ Wasserstoff, gegebenenfalls substituiertes Alkyl mit 1 bis 6 Kohlenstoffatomen, $-(CH_2CH_2O)_r-L_1$ oder $-OL_1$, worin $L_1$ Alkyl mit 1 bis 12 Kohlenstoffatomen und r 1, 2 oder 3 ist, gegebenenfalls substituiertes Aryl, Hydroxyl oder ein Rest der Formel

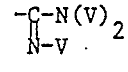

ist, worin V Wasserstoff oder Alkyl mit 1 bis 12 Kohlenstoffatomen ist,

$R_2$ gegebenenfalls substituiertes Alkyl mit 1 bis 6 Kohlenstoffatomen, $-(CH_2CH_2O)_r-L_1$, worin $L_1$

und r die oben angegebenen Bedeutungen haben, oder gegebenenfalls substituiertes Aryl ist, oder

$R_1$ und $R_2$ zusammen mit dem Stickstoffatom, an das sie gebunden sind, einen gegebenenfalls substituierten, gesättigten oder ungesättigten, gegebenenfalls ein weiteres Heteroatom enthaltenden 5-, 6- oder 7-gliedrigen Ring bilden,

$A_1$ und $A_2$ unabhängig voneinander ein Rest der Formel

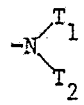

sind, worin $T_1$ und $T_2$ unabhängig voneinander Wasserstoff, gegebenenfalls substituiertes Alkyl mit 1 bis 12 Kohlenstoffatomen oder gegebenenfalls substituiertes Aryl sind,

$X_1$ und $X_2$ unabhängig voneinander Wasserstoff oder gegebenenfalls substituiertes Alkyl mit 1 bis 20 Kohlenstoffatomen sind,

$X_3$ Wasserstoff, gegebenenfalls substituiertes Alkyl mit 1 bis 6 Kohlenstoffatomen, gegebenenfalls substituiertes Alkoxy mit 1 bis 20 Kohlenstoffatomen, $—O(C_2H_4O)_n—H$, $—O(CH_2)_m—OH$, $—O—(CH_2)_m—OZ_1$ oder $—O—(C_2H_4O)_n—Z_1$, worin $Z_1$ Alkyl mit 1 bis 8 Kohlenstoffatomen und n eine ganze Zahl von 1 bis 6 und m 2, 3 oder 4 ist, gegebenenfalls substituiertes Aryloxy, Hydroxyl, Halogen, $—NHCO—Y_1$, $—NHCOH$, $—NHCO—OY_1$, $—NHP(O)(OY_1)_2$ oder $—NHSO_2—Y_1$, worin $Y_1$ gegebenenfalls substituiertes Alkyl mit 1 bis 16 Kohlenstoffatomen, $—O(CH_2)_m—OH$, $—O—(C_2H_4O)_n—H$, $—O—(CH_2)_m—OZ_1$ oder $—O—(C_2H_4O)_n—Z_1$, worin $Z_1$, m und n die oben angegebenen Bedeutungen haben, oder $Y_1$ gegebenenfalls substituiertes Aryl ist, und

$X_4$ Wasserstoff, gegebenenfalls substituiertes Alkyl mit 1 bis 12 Kohlenstoffatomen, $—O(C_2H_4O)_n—H$, $—O(CH_2)_m—OH$, $—O(CH_2)_m—OZ_1$ oder $—O—(C_2H_4O)_n—Z_1$, worin $Z_1$, m und n die oben angegebenen Bedeutungen haben, gegebenenfalls substituiertes Alkoxy mit 1 bis 16 Kohlenstoffatomen, gegebenenfalls substituiertes Aryloxy oder Halogen ist, und die Summe der Kohlenstoffatome in den Substituenten $X_1$, $X_2$, $X_3$ und $X_4$ mindestens 10 beträgt;

und das Material nach Entwickeln des Bildsilbers mit einer sauren Verarbeitungslösung behandelt wird, die einen zur Uebertragung von Kationen befähigten Phasentransferkatalysator enthält.

2. Verfahren nach Anspruch 1, dadurch gekennzeichnet, dass das Triazen der Formel

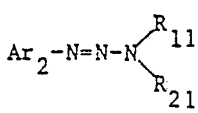 . (4) oder 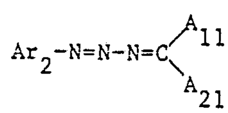 (5)

und die öllösliche Kupplungskomponente der Formel

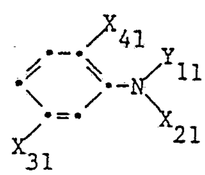 . (6)

entspricht, worin

$Ar_2$ gegebenenfalls substituiertes Phenyl, gegebenenfalls substituiertes Naphthyl oder ein gegebenenfalls substituierter, aromatischer, 1 bis 3 Heteroatome enthaltender Rest,

$R_{11}$ gegebenenfalls substituiertes Alkyl mit 1 bis 6 Kohlenstoffatomen, $—(CH_2CH_2O)_r—L_{11}$ oder $—OL_{11}$, worin $L_{11}$ Alkyl mit 1 bis 8 Kohlenstoffatomen und r 1, 2 oder 3 ist, gegebenenfalls substituiertes Phenyl oder Hydroxyl ist, und

$R_{21}$ gegebenenfalls substituiertes Alkyl mit 1 bis 6 Kohlenstoffatomen, $—(CH_2CH_2O)_r—L_{11}$, worin $L_{11}$ und r die oben angegebenen Bedeutungen haben, oder gegebenenfalls substituiertes Phenyl ist, oder $R_{21}$ und $R_{11}$ zusammen mit dem Stickstoffatom, an das sie gebunden sind, einen gegebenenfalls substituierten, gesättigten oder ungesättigten, gegebenenfalls ein weiteres Heteroatom enthaltenden 5-, 6- oder 7-gliedrigen Ring bilden, und

$A_{11}$ und $A_{21}$ unabhängig voneinander ein Rest der Formel

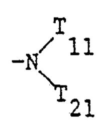

sind,

worin $T_{11}$ und $T_{21}$ unabhängig voneinander Wasserstoff, Alkyl mit 1 bis 12 Kohlenstoffatomen oder Phenyl sind,

$X_{11}$ und $X_{21}$ unabhängig voneinander Wasserstoff oder gegebenenfalls substituiertes Alkyl mit 1 bis 16 Kohlenstoffatomen sind,

$X_{31}$ Wasserstoff, gegebenenfalls substituiertes Alkyl mit 1 bis 4 Kohlenstoffatomen, gegebenenfalls substituiertes Alkoxy mit 1 bis 16 Kohlenstoffatomen, $O(C_2H_4O)_n$—H, —$O(CH_2)_m$—OH, —$O(CH_2)_m$—$OZ_{11}$ oder —O—$(C_2H_4O)_n$—$Z_{11}$, worin $Z_{11}$ Alkyl mit 1 bis 4 Kohlenstoffatomen ist und m und n die in Anspruch 1 angegebene Bedeutung haben, gegebenenfalls substituiertes Phenoxy, Hydroxyl, Halogen, —NHCO—$Y_2$, —NHCO—$OY_2$, —NHCOH, —NHP(O) $(OY_2)_2$ oder —$NHSO_2$—$Y_2$, worin $Y_2$ gegebenenfalls substituiertes Alkyl mit 1 bis 16 Kohlenstoffatomen, —$(C_2H_4O)_n$—H, —$(CH_2)_m$—OH, —$(CH_2)_m$—$OZ_{11}$ oder —$(C_2H_4O)_n$—$Z_{11}$, worin $Z_{11}$, n und m die oben angegebenen Bedeutungen haben, oder $Y_2$ gegebenenfalls substituiertes Phenyl ist, und

$X_{41}$ Wasserstoff, gegebenenfalls substituiertes Alkyl mit 1 bis 8 Kohlenstoffatomen, —$O(C_2H_4O)_n$—H, —$O(CH_2)_m$—OH, —$O(CH_2)_m$—$OZ_{11}$ oder —O—$(C_2H_4O)_n$—$Z_{11}$, worin $Z_{11}$, m und n die oben angegebenen Bedeutungen haben, gegebenenfalls substituiertes Alkoxy mit 1 bis 16 Kohlenstoffatomen, gegebenenfalls substituiertes Phenoxy oder Halogen ist, und die Summe der Kohlenstoffatomen in den Substituenten $X_{11}$, $X_{21}$, $X_{31}$ und $X_{41}$ mindestens 10 beträgt.

3. Verfahren nach Anspruch 2, dadurch gekennzeichnet, dass das Triazen der Formel

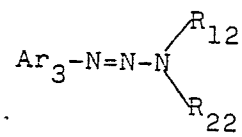

(7)

und die öllösliche Kupplungskomponente der Formel

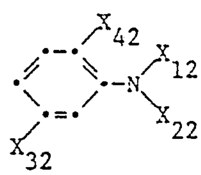

(8)

entspricht, worin

$Ar_3$ gegebenenfalls substituiertes Phenyl oder gegebenenfalls substituiertes Naphthyl,

$R_{12}$ gegebenenfalls mit Hydroxyl, Cyano, Methoxy, Carboxyl, Alkoxycarbonyl mit 2 bis 7 Kohlenstoffatomen, wobei der Alkoxyteil gegebenenfalls weiter substituiert ist, oder —$SO_3M$, worin M Wasserstoff, Ammonium oder ein Alkalimetall ist, substituiertes Alkyl mit 1 bis 6 Kohlenstoffatomen, —$(CH_2CH_2O)_r$—$L_{11}$ oder —$OL_{11}$, worin $L_{11}$ und r die in Anspruch 2 angegebenen Bedeutungen haben, gegebenenfalls mit Alkyl mit 1 bis 6 Kohlenstoffatomen, Halogen oder Alkoxy substituiertes Phenyl oder Hydroxyl ist, und

$R_{22}$ gegebenenfalls mit Hydroxyl, Cyano, Methoxy, Carboxyl, Alkoxycarbonyl mit 2 bis 7 Kohlenstoffatomen, wobei der Alkoxyteil gegebenenfalls weiter substituiert ist, oder —$SO_3M$, worin M Wasserstoff, Ammonium oder ein Alkalimetall ist, substituiertes Alkyl mit 1 bis 6 Kohlenstoffatomen, —$(CH_2CH_2O)_r$—$L_{11}$, worin $L_{11}$ und r die in Anspruch 2 angegebenen Bedeutungen haben, oder gegebenenfalls mit Alkyl mit 1 bis 6 Kohlenstoffatomen oder Halogen substituiertes Phenyl ist, oder $R_{22}$ und $R_{12}$ zusammen mit dem Stickstoffatom, an das sie gebunden sind, einen gegebenenfalls mit Alkyl mit 1 bis 6 Kohlenstoffatomen substituierten, gesättigten oder ungesättigten, gegebenenfalls ein Stickstoff- oder Sauerstoffatom als weiteres Heteroatom enthaltenden 5-, 6- oder 7-gliedrigen Ring bilden,

$X_{12}$ und $X_{22}$ unabhängig voneinander Wasserstoff, Alkyl mit 1 bis 16 Kohlenstoffatomen, Benzyl, Phenyläthyl oder ein Rest der Formel —$CHM_1$—$CH_2M_2$ sind, worin $M_1$ Wasserstoff oder Alkyl mit 1 bis 4 Kohlenstoffatomen und $M_2$ Cyano oder ein Rest der Formel —$OM_3$ oder —$CO_2M_3$, worin $M_3$ Wasserstoff, Alkyl mit 1 bis 16 Kohlenstoffatomen, gegebenenfalls mit Alkyl mit 1 bis 4 Kohlenstoffatomen substituiertes Phenyl oder ein Rest der Formel —$(C_2H_4O)_nM_4$ oder $(CH_2)_mOM_4$ ist, worin $M_4$ Wasserstoff oder gegebenenfalls substituiertes Alkyl mit 1 bis 4 Kohlenstoffatomen ist, und n und m die oben angegebenen Bedeutungen haben,

$X_{32}$ Wasserstoff, Alkyl mit 1 bis 4 Kohlenstoffatomen, Alkoxy mit 1 bis 16 Kohlenstoffatomen, —$O(C_2H_4O)_n$—H, —$O(CH_2)_m$—OH, —$O(C_2H_4O)_m$—$Z_{11}$ oder —$O(CH_2)_m$—$OZ_{11}$, worin $Z_{11}$ die in Anspruch 2 angegebene Bedeutung hat und n und m die oben angegebenen Bedeutungen haben, Phenoxy, Hydroxyl, Halogen, —NHCO—$Y_3$, —NHCOH, —NHCO—$OY_3$, —NHP(O) $(OY_3)_2$ oder —$NHSO_2$—$Y_3$, worin $Y_3$ Alkyl mit 1 bis 16 Kohlenstoffatomen, gegebenenfalls mit Alkyl mit 1 bis 4 Kohlenstoffatomen substituiertes Phenyl oder ein Rest der Formel —$(C_2H_4O)_n$—H, —$(CH_2)_m$—OH, —$(C_2H_4O)_n$—$Z_{11}$ oder $(CH_2)_m$—$OZ_{11}$ ist, worin $Z_{11}$, n und m die oben angegebenen Bedeutungen haben, und

$X_{42}$ Wasserstoff, Alkyl mit 1 bis 6 Kohlenstoffatomen, Halogen, Alkoxy mit 1 bis 8 Kohlenstoffatomen oder —$O(C_2H_4O)_n$—H, —$O(CH_2)_m$—OH, —$O(CH_2)_m$—$OZ_{11}$ oder —$O(C_2H_4O)_n$—$Z_{11}$ ist, worin $Z_{11}$, n und m die oben angegebenen Bedeutungen haben, oder gegebenenfalls substituiertes Phenoxy ist, und

wobei die Summe der Kohlenstoffatome in den Substituenten $X_{12}$, $X_{22}$, $X_{32}$ und $X_{42}$ mindestens 10 beträgt.

4. Verfahren nach Anspruch 3, dadurch gekennzeichnet, dass die öllösliche Kupplungskomponente der Formel (8) und das Triazen der Formel

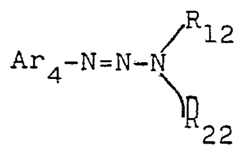

(9)

entspricht,
worin

$Ar_4$ der Formel

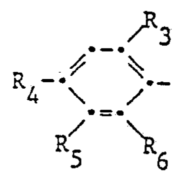

entspricht, worin $R_3$ und $R_6$ unabhängig voneinander Wasserstoff, gegebenenfalls substituiertes Alkyl oder Alkoxy mit je 1 bis 4 Kohlenstoffatomen, Carboxyl, Alkoxycarbonyl mit 2 bis 12 Kohlenstoffatomen, $—SO_2T_1$, $—SO_2N(T_1)_2$ oder $—SO_2NT_1T_2$, worin $T_1$ und $T_2$ die in Anspruch 1 angegebene Bedeutung haben, Halogen, Cyano oder Nitro sind, $R_4$ Wasserstoff, gegebenenfalls substituiertes Alkyl oder Alkoxy mit je 1 bis 20 Kohlenstoffatomen, Alkoxycarbonyl mit 2 bis 21 Kohlenstoffatomen, wobei der Alkoxyteil gegebenenfalls weiter substituiert ist, $—SO_2T_3$, $—SO_2N(T_3)_2$ oder $—SO_2NT_1T_3$, worin $T_3$ Alkyl mit 1 bis 20 Kohlenstoffatomen oder $—(CH_2)_p—OT_1$ ist, $T_1$ die oben angegebenen Bedeutungen hat und p 2, 3 oder 4 oder ist oder $R_4$ Halogen, Trifluormethyl, Cyano, Nitro oder ein Rest der Formel

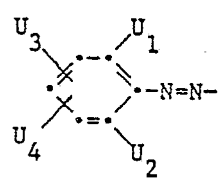

ist, worin $U_1$ und $U_2$ unabhängig voneinander Wasserstoff, $—SO_2U_5$, worin $U_5$ Alkyl mit 1 bis 4 Kohlenstoffatomen ist, Halogen, Cyano oder Nitro sind, $U_3$ Wasserstoff, Halogen oder Nitro und $U_4$ gegebenenfalls substituiertes Alkyl oder Alkoxy mit je 1 bis 20 Kohlenstoffatomen, $—SO_2T_3$, $—SO_2NT_1T_3$, worin $T_1$ und $T_3$ die oben angegebenen Bedeutungen haben, oder Alkoxycarbonyl mit 2 bis 21 Kohlenstoffatomen ist, wobei der Alkoxyteil gegebenenfalls weiter substituiert ist, $R_5$ Wasserstoff, gegebenenfalls substituiertes Alkyl oder Alkoxy mit je 1 bis 20 Kohlenstoffatomen, Alkoxycarbonyl mit 2 bis 21 Kohlenstoffatomen, wobei der Alkoxyteil gegebenenfalls weiter substituiert ist, $—SO_2T_3$, $—SO_2N(T_3)_2$ oder $—SO_2NT_1T_3$ ist, worin $T_1$ und $T_3$ die oben angegebenen Bedeutungen haben, oder $R_5$ zusammen mit $R_4$ einen Rest der Formel

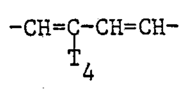

oder $R_5$ zusammen mit $R_6$ einen Rest der Formel

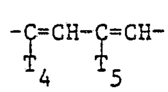

bildet, worin $T_4$ Wasserstoff, Nitro, $—SO_2T_3$, $—SO_2N(T_3)_2$ oder $—SO_2NT_1T_3$ und $T_5$ Wasserstoff, $—OT_3$, $—SO_2T_3$ oder $—SO_2NT_1T_3$ ist, worin $T_1$ und $T_3$ die oben angegebenen Bedeutungen haben, und worin die Summe der Kohlenstoffatome in den Substituenten $R_3$, $R_4$, $R_5$ und $R_6$ mindestens 8 beträgt, und $R_{12}$, $R_{22}$, sowie $X_{12}$, $X_{22}$, $X_{32}$ und $X_{42}$ die in Anspruch 3 angegebenen Bedeutungen haben.

5. Verfahren nach Anspruch 4, dadurch gekennzeichnet, dass die öllösliche Kupplungskomponente der Formel (8) und das Triazen der Formel

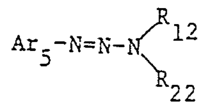 (10)

entspricht, worin

$Ar_5$ der Formel

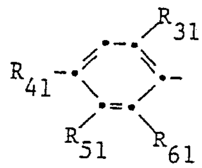

entspricht, worin $R_{31}$ und $R_{61}$ unabhängig voneinander Wasserstoff, Alkyl oder Alkoxy mit je 1 oder 2 Kohlenstoffatomen, Trifluormethyl, Carboxyl, Alkoxycarbonyl mit 2 bis 7 Kohlenstoffatomen, $—SO_2T_{11}$, $—SO_2N(T_{11})_2$ oder $—SO_2NT_{11}T_{21}$, worin $T_{11}$ und $T_{21}$ die in Anspruch 2 angegebenen Bedeutungen haben, Fluor, Chlor, Brom, Cyano oder Nitro sind, $R_{41}$ Wasserstoff, Alkyl mit 1 bis 20 Kohlenstoffatomen, Trifluormethyl, gegebenenfalls mit Alkoxy mit 1 bis 6 Kohlenstoffatomen substituiertes Alkoxy mit 1 bis 20 Kohlenstoffatomen, Alkoxycarbonyl mit 2 bis 21 Kohlenstoffatomen, wobei der Alkoxyteil gegebenenfalls mit Alkoxy mit 1 bis 6 Kohlenstoffatomen weiter substituiert ist, $—SO_2T_{31}$, $—SO_2N(T_{31})_2$ oder $—SO_2NT_{11}T_{31}$, worin $T_{31}$ Alkyl mit 1 bis 20 Kohlenstoffatomen oder $—(CH_2)_p—OT_{11}$ ist, worin $T_{11}$ die oben angegebenen Bedeutungen hat und p 2, 3 oder 4 ist, oder $R_4$ Chlor, Brom, Cyano, Nitro oder ein Rest der Formel

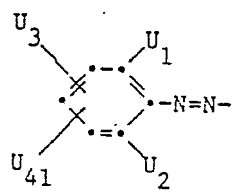

ist, worin $U_{41}$ Wasserstoff, Alkyl mit 1 bis 20 Kohlenstoffatomen, Trifluormethyl, gegebenenfalls mit Alkoxy mit 1 bis 6 Kohlenstoffatomen substituiertes Alkoxy mit 1 bis 20 Kohlenstoffatomen, $—SO_2T_{31}$, $—SO_2NT_{11}T_{31}$, worin $T_{11}$ und $T_{31}$ die oben angegebenen Bedeutungen haben, oder Alkoxycarbonyl mit 2 bis 21 Kohlenstoffatomen ist, wobei der Alkoxyteil gegebenenfalls mit Alkoxy mit 1 bis 6 Kohlenstoffatomen weiter substituiert ist, $R_{51}$ Wasserstoff, Alkyl mit 1 bis 20 Kohlenstofatomen, gegebenenfalls mit Alkoxy mit 1 bis 12 Kohlenstoffatomen, oder Hydroxyl substituiertes Alkoxy mit 1 bis 20 Kohlenstoffatomen, Alkoxycarbonyl mit 2 bis 21 Kohlenstoffatomen, wobei der Alkoxyteil gegebenenfalls mit Alkoxy mit 1 bis 12 Kohlenstoffatomen, weitersubstituiert ist, $—SO_2T_{31}$, $—SO_2N(T_{31})_2$ oder $—SO_2NT_{11}T_{31}$ ist, worin $T_{11}$ und $T_{31}$ die oben angegebenen Bedeutungen haben, oder $R_{51}$ zusammen mit $R_{41}$ einen Rest der Formel

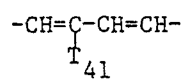

oder $R_{51}$ zusammen mit $R_{61}$ einen Rest der Formel

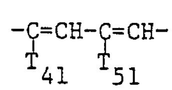

bildet, worin $T_{41}$ Wasserstoff, Nitro, $—SO_2T_{31}$, $—SO_2N(T_{31})_2$ oder $—SO_2NT_{11}T_{31}$ und $T_{51}$ Wasserstoff, $—OT_{31}$, $—SO_2T_{31}$ oder $—SO_2NT_{11}T_{31}$ ist, worin $T_{11}$ und $T_{31}$ die oben angegebenen Bedeutungen haben, und wobei die Summe der Kohlenstoffatome in den Substituenten $R_{31}$, $R_{41}$, $R_{51}$ und $R_{61}$ mindestens 10 beträgt, und $R_{12}$, $R_{22}$ sowie $U_1$, $U_2$ und $U_3$ die in Anspruch 4 angegebenen Bedeutungen haben.

6. Verfahren nach Anspruch 5, dadurch gekennzeichnet, dass das Triazen der Formel (10) und die öllösliche Kupplungskomponente der Formel

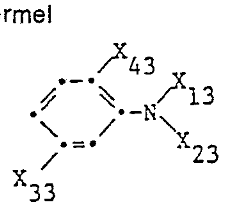 (11)

entspricht, worin

$X_{13}$ Alkyl mit 1 bis 16 Kohlenstoffatomen oder ein Rest der Formel $—CHM_1—CH_2M_2$, worin $M_1$ und $M_2$ die in Anspruch 3 angegebenen Bedeutungen haben,

$X_{23}$ Wasserstoff, Alkyl mit 1 bis 16 Kohlenstoffatomen, ein Rest der Formel $—CHM_1—CH_2M_2$, worin $M_1$ und $M_2$ die oben angegebenen Bedeutungen haben, Benzyl oder Phenyläthyl,

$X_{33}$ Wasserstoff, Alkyl mit 1 bis 4 Kohlenstoffatomen, Alkoxy mit 1 bis 12 Kohlenstoffatomen, $—O(C_2H_4O)_n—Z_{11}$ oder $—O(CH_2)_m—OZ_{11}$, worin $Z_{11}$, n und m die in Anspruch 3 angegebenen Bedeutungen haben, Phenoxy, Hydroxyl, Chlor, Brom, $—NHCO—Y_3$, —NHCOH, $—NHCO—OY_3$, $—NHP(O)(OY_3)_2$ oder $—NHSO_2—Y_3$, worin $Y_3$ die in Anspruch 3 genannten Bedeutungen hat, $X_{43}$ Wasserstoff, Alkyl mit 1 bis 4 Kohlenstoffatomen, Chlor, Brom, Alkoxy mit 1 bis 8 Kohlenstoffatomen oder $—O—(CH_2CH_2O)_n—Z_{11}$ ist, worin $Z_{11}$ und n die oben angegebene Bedeutung haben, und wobei die Summe der Kohlenstoffatome in den Substituenten $X_{13}$, $X_{23}$, $X_{33}$ und $X_{43}$ mindestens 10 beträgt.

7. Verfahren nach Anspruch 6, dadurch gekennzeichnet, dass das Triazen der Formel

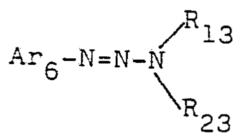 (12)

und die öllösliche Komponente der Formel

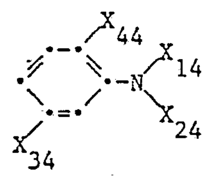 (13)

entspricht, wobei

$Ar_6$ der Formel

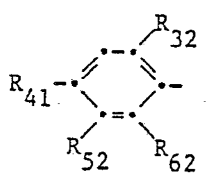

entspricht, worin $R_{32}$ und $R_{62}$ unabhängig voneinander Wasserstoff, Methyl, Methoxy, Trifluormethyl, Alkoxycarbonyl mit 2 bis 4 Kohlenstoffatomen, $—SO_2T_{13}$, $—SO_2N(T_{13})_2$ oder $—SO_2NT_{13}T_{23}$, worin $T_{13}$ und $T_{23}$ unabhängig voneinander Wasserstoff oder Alkyl mit 1 oder 2 Kohlenstoffatomen sind, Fluor, Chlor, Brom, Cyano oder Nitro sind, $R_{52}$ Wasserstoff, Alkyl mit 1 bis 20 Kohlenstoffatomen, $—OT_{31}$, $—CO—OT_{31}$ oder $—SO_2T_{31}$, worin $T_{31}$ die in Anspruch 5 angegebenen Bedeutungen hat, oder $R_{52}$ zusammen mit $R_{41}$ einen Rest der Formel

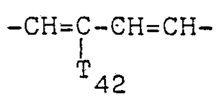

oder $R_{52}$ zusammen mit $R_{62}$ einen Rest der Formel

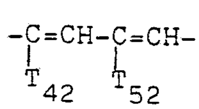

bildet, worin $T_{42}$ Wasserstoff, Nitro, $—SO_2T_{32}$ oder $—SO_2N(T_{32})_2$ und $T_{52}$ Wasserstoff, $—OT_{32}$, $—SO_2T_{32}$ oder $—SO_2NT_{12}T_{32}$ ist, worin $T_{32}$ Alkyl mit 1 bis 10 Kohlenstoffatomen oder $—(CH_2)_p—OT_{12}$, und p und $T_{12}$ sowie $R_{41}$ die in Anspruch 5 angegebenen Bedeutungen haben, und wobei die Summe der Kohlenstoffatome in den Substituenten $R_{32}$, $R_{41}$, $R_{52}$ und $R_{62}$ mindestens 10 beträgt,

$R_{13}$ Alkyl mit 1 bis 4 Kohlenstoffatomen, gegebenenfalls mit Hydroxyl, Cyano, Carboxyl, $—COOCH_3$ oder Methoxy substituiertes Alkyl mit 1 oder 2 Kohlenstoffatomen, $—(CH_2CH_2O)_r—L_{12}$ oder $—OL_{12}$,

worin $L_{12}$ Alkyl mit 1 bis 6 Kohlenstoffatomen und r die in Anspruch 2 angegebenen Bedeutungen hat, Phenyl, Tolyl, Chlorphenyl, Bromphenyl oder Methoxyphenyl,

$R_{23}$ Alkyl mit 1 bis 4 Kohlenstoffatomen, gegebenenfalls mit Hydroxyl, Cyano, Carboxyl, $-COOCH_3$ oder Methoxy substituiertes Alkyl mit 1 oder 2 Kohlenstoffatomen, $-(CH_2CH_2O)_r-L_{12}$, worin $L_{12}$ und r die oben angegebenen Bedeutungen haben, Phenyl, Tolyl, Chlorphenyl oder Bromphenyl ist, oder $R_{23}$ und $R_{13}$ einen Rest der Formel $-(CH_2)_4-$, $-(CH_2)_5-$, $-C_2H_4-O-C_2H_4-$, $-C_2H_4-NH-C_2H_4-$, $-C_2H_4-N(CH_3)-C_2H_4-$, $-CH=N-CH=CH-$ oder $-CH=CH-CH=CH-$ bilden, und

$X_{14}$ Alkyl mit 1 bis 16 Kohlenstoffatomen oder ein Rest der Formel $-CHM_{11}-CH_2M_{21}$, worin $M_{11}$ Wasserstoff, Methyl oder Aethyl und $M_{21}$ Cyano oder $-OM_{31}$ ist, worin $M_{31}$ Wasserstoff, Alkyl mit 1 bis 6 Kohlenstoffatomen oder gegebenenfalls mit Alkyl mit 1 bis 4 Kohlenstoffatomen substituiertes Phenyl ist,

$X_{24}$ Wasserstoff, Alkyl mit 1 bis 16 Kohlenstoffatomen oder Benzyl, $X_{34}$ Wasserstoff, Alkyl mit 1 bis 4 Kohlenstoffatomen, Phenoxy, Chlor, Hydroxyl, Alkoxy mit 1 bis 6 Kohlenstoffatomen, $-O-CH_2CH_2-OH$, $-O-CH_2CH_2-OZ_{11}$, $-NHCO-Y_4$, $-NHCOH$, $-NHCO-CH_2CH_2-OH$, $-NHCO-CH_2CH_2-OZ_{11}$, $-NHP(O)(OY_4)_2$, $-NHP(O)(OC_6H_4Y_5)_2$ oder $-NHSO_2Y_4$, worin $Y_4$ Alkyl mit 1 bis 16 Kohlenstoffatomen, $Y_5$ Wasserstoff oder Alkyl mit 1 bis 6 Kohlenstoffatomen und $Z_{11}$ die in Anspruch 6 angegebenen Bedeutungen hat, und

$X_{44}$ Wasserstoff, Alkyl oder Alkoxy mit je 1 bis 4 Kohlenstoffatomen oder $-O-(CH_2CH_2O)_n-Z_{11}$ ist, worin $Z_{11}$ und n die in Anspruch 6 angegebenen Bedeutungen haben, und wobei die Summe der Kohlenstoffatome in den Substituenten $X_{14}$, $X_{24}$, $X_{34}$ und $X_{44}$ mindestens 10 beträgt.

8. Verfahren nach Anspruch 2, dadurch gekennzeichnet, dass die öllösliche Kupplungskomponente der Formel (8) und das Triazen der Formel

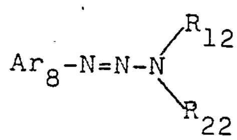

(15)

entspricht, worin

$Ar_8$ ein Rest der Formel

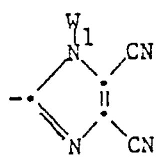

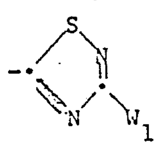

oder

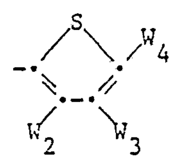

ist, worin

$W_1$ gegebenenfalls substituiertes Alkyl mit 6 bis 18 Kohlenstoffatomen oder gegebenenfalls substituiertes Phenyl, $W_2$ Alkoxycarbonyl mit 2 bis 25 Kohlenstoffatomen, Nitro oder Cyano ist, und $W_3$ und $W_4$ unabhängig voneinander Wasserstoff oder Alkyl mit 1 bis 6 Kohlenstoffatomen sind, oder

$W_3$ zusammen mit $W_4$ einen Rest der Formel

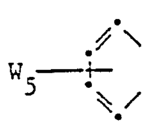

,

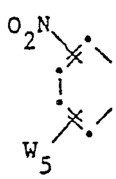

oder

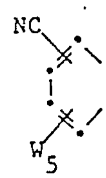

bildet, worin

$W_5$ Alkyl mit 6 bis 18 Kohlenstoffatomen bedeutet, und wobei die Summe der Kohlenstoffatome in den Substituenten $W_1$, $W_2$, $W_3$ und $W_4$ mindestens 8 beträgt und

$R_{12}$ und $R_{22}$ die in Anspruch 3 angegebenen Bedeutungen haben.

9. Verfahren nach Anspruch 1, dadurch gekennzeichnet, dass das Material in mindestens einer Silberhalogenidemulsionsschicht oder in je einer benachbarten Schicht ein öllösliches Triazen der Formel (1) oder (2) und eine öllösliche Kupplungskomponente der Formel (3) enthält, wobei Triazen und Kupplungskomponente in einem mit Wasser nicht mischbaren Lösungsmittel oder Lösungsmittelgemisch gelöst in feinverteilter Form der (den) lichtempfindlichen Silberhalogenidemulsionsschicht(en) oder in einer dieser (diesen) benachbarten Schicht eingearbeitet vorliegen, und das Material nach Belichtung und Entwicklung des Bildsilbers mit einem wässrigen Verarbeitungsbad behandelt wird, das zur Farbbildung

a) eine starke Säure und

b) einen Kationenphasentransferkatalysator,

gegebenenfalls zur gleichzeitigen Farbbleichung

c) einen Liganden, der Silberkomplexe bildet,
d) einen Farbbleichkatalysator und
e) ein Oxidationsschutzmittel,
gegebenenfalls zur gleichzeitigen Silberbleichung
f) ein Oxidationsmittel und
gegebenenfalls zur gleichzeitigen Fixierung
g) ein Silberhalogenidlösungsmittel
enthält.

10. Verfahren nach Anspruch 9, dadurch gekennzeichnet, dass der Kationenphasentransferkatalysator eine starke anorganische Säure, eine perhalogenierte aliphatische Säure, eine mit 1 oder 2 Alkyl- oder Alkoxygruppen mit je 1 bis 12 Kohlenstoffatomen substituierte Benzolsulfonsäure, mit 1 bis 3 Halogenatomen substituierte Benzolsulfonsäure, gegebenenfalls halogenierte Alkylsulfonsäure mit 1 bis 12 Kohlenstoffatomen, Monoalkylschwefelsäure mit 1 bis 12 Kohlenstoffatomen oder ein Alkali- oder Ammoniumsalz dieser Säuren ist.

11. Verfahren nach Anspruch 10, dadurch gekennzeichnet, dass der Kationenphasentransferkatalysator in Mengen von 10 bis 200 g, vorzugsweise 10 bis 100 g pro Liter Verarbeitungslösung eingesetzt wird.

12. Photographisches Material für das Verfahren zur Herstellung photographischer Farbbilder nach einem der Ansprüche 1 bis 11, dadurch gekennzeichnet, dass es auf einem opaken oder transparenten Träger mindestens eine Silberhalogenidemulsionsschicht und in der gleichen oder benachbarten Schicht eine Dispersion des öllöslichen Triazens der Formel (1) oder (2) und der öllöslichen Kupplungskomponente der Formel (3) in einem mit Wasser nicht mischbaren Lösungsmittel oder Lösungsmittelgemisch enthält.

13. Zubereitung zur Verarbeitung des photographischen Materials nach Anspruch 12, dadurch gekennzeichnet, dass sie
a) Alkyl- oder Arylsulfonsäuren, Schwefelsäure, Sulfaminsäure oder gegebenenfalls auch Gemische dieser Säuren,
b) Trifluor- oder Trichloressigsäure oder Perchlorsäure,
gegebenenfalls c) einen Liganden, der Silberkomplexe bildet,
d) einen Farbbleichkatalysator,
e) ein Oxidationsschutzmittel
gegebenenfalls f) ein Oxidationsmittel
und gegebenenfalls g) ein Silberhalogenidlösungsmittel enthält.

**Claims**

1. A process for the production of a photographic colour image by the silver dye bleach process, by exposure, silver development, colour formation, dye bleaching, silver bleaching and fixing of a photographic material which, on a transparent or opaque base, contains at least one layer with a light-sensitive silver halide emulsion, if appropriate, the colour formation being carried out simultaneously with the dye bleaching, silver bleaching and fixing and, if appropriate, the silver bleaching being carried out simultaneously with the dye bleaching and/or fixing in a single processing bath, wherein the light-sensitive silver halide emulsion layer or layers, or a layer or layers adjacent to said silver halide emulsion layer or layers contains or contain, dispersed in oil, an oil-soluble triazene of the formula

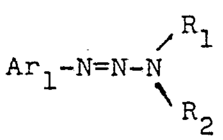 (1) or 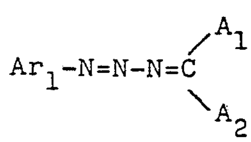, (2)

and an oil-soluble coupling component of the formula

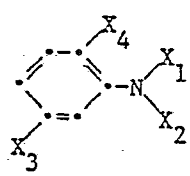 (3)

in which
$Ar_1$ is substituted or unsubstituted aryl or a substituted or unsubstituted aromatic heterocyclic radical,
$R_1$ is hydrogen, substituted or unsubstituted alkyl having 1 to 6 carbon atoms, $-(CH_2CH_2O)_r-L_1$ or

—$OL_1$, in which $L_1$ is alkyl having 1 to 12 carbon atoms and r is 1, 2 or 3, or $R_1$ is substituted or unsubstituted aryl, hydroxyl or a radical of the formula

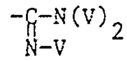

in which V is hydrogen or alkyl having 1 to 12 carbon atoms,

$R_2$ is substituted or unsubstituted alkyl having 1 to 6 carbon atoms, —$(CH_2CH_2O)_r$—$L_1$, with $L_1$ and r being as defined above, or substituted or unsubstituted aryl, or

$R_1$ and $R_2$, conjointly with the nitrogen atom to which they are bonded, form a substituted or unsubstituted, saturated or unsaturated 5-membered, 6-membered or 7-membered ring which may contain a further hetero-atom,

$A_1$ and $A_2$ independently of each other are an amino group of the formula

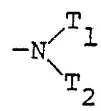

in which $T_1$ and $T_2$ independently of each other are hydrogen, substituted or unsubstituted alkyl having 1 to 12 carbon atoms or substituted or unsubstituted aryl,

$X_1$ and $X_2$ independently of each other are hydrogen or substituted or unsubstituted alkyl having 1 to 20 carbon atoms,

$X_3$ is hydrogen, substituted or unsubstituted alkyl having 1 to 6 carbon atoms, substituted or unsubstituted alkoxy having 1 to 20 carbon atoms, —$O(C_2H_4O)_n$—H, —$O(CH_2)_m$—OH, —O—$(CH_2)_m$—$OZ_1$ or —O—$(C_2H_4O)_n$—$Z_1$, in which $Z_1$ is alkyl having 1 to 8 carbon atoms, n is an integer from 1 to 5, and m is 2, 3 or 4, or $X_3$ is substituted or unsubstituted aryloxy, hydroxyl, halogen, —NHCO—$Y_1$, —NHCOH, —NHCO—$OY_1$, —$NHP(O)(OY_1)_2$ or —$NHSO_2$—$Y_1$, in which $Y_1$ is substituted or unsubstituted alkyl having 1 to 16 carbon atoms, —$O(CH_2)_m$—OH, —O—$(C_2H_4O)_n$—H, —O—$(CH_2)_m$—$OZ_1$ or —O—$(C_2H_4O)_n$—$Z_1$, with $Z_1$, m and n being as defined above, or $Y_1$ is substituted or unsubstituted aryl, and

$X_4$ is hydrogen, substituted or unsubstituted alkyl having 1 to 12 carbon atoms, —$O(C_2H_4O)_n$—H, —$O(CH_2)_m$—OH, —$O(CH_2)_m$—$OZ_1$ or —O—$(C_2H_4O)_n$—$Z_1$, with $Z_1$, m and n being as defined above, or $X_4$ is substituted or unsubstituted alkoxy having 1 to 16 carbon atoms, substituted or unsubstituted aryloxy or halogen, and the total of the carbon atoms in the substituents $X_1$, $X_2$, $X_3$ and $X_4$ is at least 10 ; and,

after development of the image silver, the material is treated with an acid processing solution which contains a phase transfer catalyst capable of transferring cations.

2. A process according to claim 1, wherein the triazene is of the formula

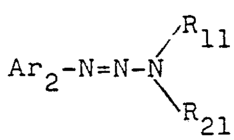 (4) or 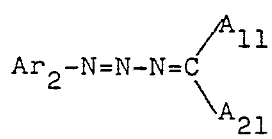 (5)

and the oil-soluble coupling component is of the formula

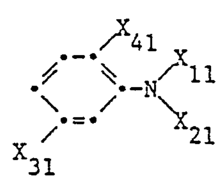 (6)

in which

$Ar_2$ is substituted or unsubstituted phenyl, substituted or unsubstituted naphthyl or a substituted or unsubstituted, aromatic radical containing 1 to 3 hetero-atoms,

$R_{11}$ is substituted or unsubstituted alkyl having 1 to 6 carbon atoms, —$(CH_2CH_2O)_r$—$L_{11}$ or —$OL_{11}$, in which $L_{11}$ is alkyl having 1 to 8 carbon atoms and r is 1, 2, or 3, or is substituted or unsubstituted phenyl, or hydroxyl,

$R_{21}$ is substituted or unsubstituted alkyl having 1 to 6 carbon atoms, —$(CH_2CH_2O)_r$—$L_{11}$, with $L_{11}$ and r being as defined above, or is substituted or unsubstituted phenyl, or $R_{21}$ and $R_{11}$, conjointly with the nitrogen atom to which they are bonded, form a substituted or unsubstituted, saturated or

unsaturated 5-membered, 6-membered or 7-membered ring which may contain a further hetero-atom,

$A_{11}$ and $A_{21}$ independently of each other are an amino group of the formula

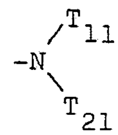

in which $T_{11}$ and $T_{21}$ independently of each other are hydrogen, alkyl having 1 to 12 carbon atoms or phenyl,

$X_{11}$ and $X_{21}$ independently of each other are hydrogen or substituted or unsubstituted alkyl having 1 to 16 carbon atoms,

$X_{31}$ is hydrogen, substituted or unsubstituted alkyl having 1 to 4 carbon atoms, substituted or unsubstituted alkoxy having 1 to 16 carbons atoms, $-O(C_2H_4O)_n-H$, $-O(CH_2)_m-OH$, $-O(CH_2)_m-OZ_{11}$ or $-O-(C_2H_4O)_n-Z_{11}$, in which $Z_{11}$ is alkyl having 1 to 4 carbon atoms and m and n are as defined in claim 1, or $X_{31}$ is substituted or unsubstituted phenoxy, hydroxyl, halogen, $-NHCO-Y_2$, $-NHCO-OY_2$, $-NHCOH$, $-NHP(O)(OY_2)_2$ or $-NHSOO_2-Y_2$, in which $Y_2$ is substituted or unsubstituted alkyl having 1 to 16 carbon atoms, $-(C_2H_4O)_n-H$, $-(CH_2)_m-OH$, $-(CH_2)_m-OZ_{11}$ or $-(C_2H_4O)_n-Z_{11}$, with $Z_{11}$, n and m being as defined above, or $Y_2$ is substituted or unsubstituted phenyl, and

$X_{41}$ is hydrogen, substituted or unsubstituted alkyl having 1 to 8 carbon atoms, $-O(C_2H_4O)_n-H$, $-O(CH_2)_m-OH$, $-O(CH_2)_m-OZ_{11}$ or $-O-(C_2H_4O)_n-Z_{11}$, with $Z_{11}$, m and n being as defined above, or $X_{41}$ is substituted or unsubstituted alkoxy having 1 to 16 carbon atoms, substituted or unsubstituted phenoxy or halogen, the total of the carbon atoms in the substituents $X_{11}$, $X_{21}$, $X_{31}$ and $X_{41}$ being at least 10.

3. A process according to claim 2, wherein the triazene is of the formula

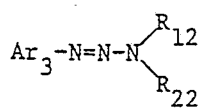

(7)

and the oil-soluble coupling component is of the formula

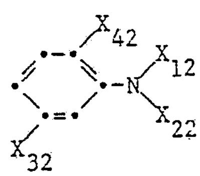

(8)

in which

$Ar_3$ is substituted or unsubstituted phenyl or substituted or unsubstituted naphthyl,

$R_{12}$ is alkyl having 1 to 6 carbon atoms, which is unsubstituted or substituted by hydroxyl, cyano, methoxy, carboxyl, alkoxycarbonyl having 2 to 7 carbon atoms, in which the alkoxy moiety can be further substituted, or by $-SO_3M$, in which M is hydrogen, ammonium or an alkali metal, or $R_{12}$ is $-(CH_2CH_2O)_r-L_{11}$ or $-OL_{11}$, in which $L_{11}$ and r are as defined in claim 2, or phenyl which is unsubstituted or substituted by alkyl having 1 to 6 carbon atoms or by halogen or alkoxy or is hydroxyl,

$R_{22}$ is alkyl having 1 to 6 carbon atoms, which is unsubstituted or substituted by hydroxyl, cyano, methoxy, carboxyl, alkoxycarbonyl having 2 to 7 carbon atoms, in which the alkoxy moiety can be further substituted, or by $-SO_3M$, in which M is hydrogen, ammonium or an alkali metal, or $R_{22}$ is $-(CH_2CH_2O)_r-L_{11}$, in which $L_{11}$ and r are as defined in claim 2, or phenyl which is unsubstituted or substituted by alkyl having 1 to 6 carbon atoms or halogen, or $R_{22}$ and $R_{12}$, conjointly with the nitrogen atom to which they are bonded, form a saturated or unsaturated 5-membered, 6-membered or 7-membered ring which is unsubstituted or substituted by alkyl having 1 to 6 carbon atoms and may contain a nitrogen atom or oxygen atom as a further hetero-atom,

$X_{12}$ and $X_{22}$ independently of each other are hydrogen, alkyl having 1 to 16 carbon atoms, benzyl, phenylethyl or a radical of the formula $-CHM_1-CH_2M_2$, in which $M_1$ is hydrogen or alkyl having 1 to 4 carbon atoms and $M_2$ is cyano or a radical of the formula $-OM_3$ or $-CO_2M_3$, in which $M_3$ is hydrogen, alkyl having 1 to 16 carbon atoms, phenyl which is unsubstituted or substituted by alkyl having 1 to 4 carbon atoms, or a radical of the formula $-(C_2H_4O)_n-M_4$ or $(-CH_2)_m-OM_4$, in which $M_4$ is hydrogen or substituted or unsubstituted alkyl having 1 to 4 carbon atoms, and n and m are as defined above,

$X_{32}$ is hydrogen, alkyl having 1 to 4 carbon atoms, alkoxy having 1 to 16 carbon atoms,

$—O(C_2H_4O)_n—H$, $—O(CH_2)_m—OH$, $—O(C_2H_4O)_m—Z_{11}$ or $—O(CH_2)_m—OZ_{11}$, in which $Z_{11}$ is as defined in claim 2 and n and m are as defined above, or $X_{32}$ is phenoxy, hydroxyl, halogen, $—NHCO—Y_3$, $—NHCOH$, $—NHCO—OY_3$, $—NHP(O)(OY_3)_2$ or $—NHSO_2—Y_3$, in which $Y_3$ is alkyl having 1 to 16 carbon atoms, phenyl which is unsubstituted or substituted by alkyl having 1 to 4 carbon atoms or is a radical of the formula $—(C_2H_4O)_n—H$, $—(CH_2)_m—OH$, $—(C_2H_4O)_n—Z_{11}$ or $—(CH_2)_m—OZ_{11}$, with $Z_{11}$, n and m being as defined above, and

$X_{42}$ is hydrogen, alkyl having 1 to 6 carbon atoms, halogen, alkoxy having 1 to 8 carbon atoms, or $—O(C_2H_4O)_n—H$, $—O(CH_2)_m—OH$, $—O(CH_2)_m—OZ_{11}$ or $—O(C_2H_4O)_n—Z_{11}$, n and m being as defined above, or $X_{42}$ is substituted or unsubstituted phenoxy, the total of the carbon atoms in the substitutents $X_{12}$, $X_{22}$, $X_{32}$ and $X_{42}$ being at least 10.

4. A process according to claim 3, wherein the oil-soluble coupling component is of the formula (8) and the triazene is of the formula

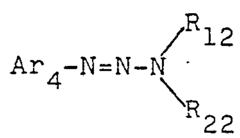

(9)

in which

$Ar_4$ is of the formula

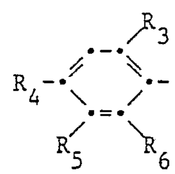

in which $R_3$ and $R_6$ independently of each other are hydrogen, substituted or unsubstituted alkyl or alkoxy each having 1 to 4 carbon atoms, carboxyl, alkoxycarbonyl having 2 to 12 carbon atoms, $—SO_2T_1$, $—SO_2N(T_1)_2$ or $—SO_2NT_1T_2$, in which $T_1$ and $T_2$ are as defined in claim 1, halogen, cyano or nitro, $R_4$ is hydrogen, substituted or unsubstituted alkyl or alkoxy each having 1 to 20 carbon atoms, alkoxycarbonyl having 2 to 21 carbon atoms, in which the alkoxy moiety can be further substituted, $—SO_2T_3$, $—SO_2N(T_3)_2$ or $—SO_2NT_1T_3$, in which $T_3$ is alkyl having 1 to 20 carbon atoms or $—(CH_2)_p—OT_1$, $T_1$ is as defined above and p is 2, 3 or 4, or $R_4$ is halogen, trifluoromethyl, cyano, nitro or a radical of the formula

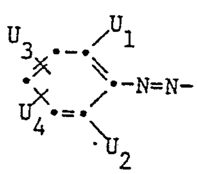

in which $U_1$ and $U_2$ independently of each other are hydrogen, $—SO_2U_5$, in which $U_5$ is alkyl having 1 to 4 carbon atoms, or are halogen, cyano or nitro, $U_3$ is hydrogen, halogen or nitro and $U_4$ is substituted or unsubstituted alkyl or alkoxy each having 1 to 20 carbon atoms, $—SO_2T_3$, $—SO_2NT_1T_3$, in which $T_1$ and $T_3$ are as defined above, or alkoxycarbonyl having 2 to 21 carbon atoms, in which the alkoxy moiety can be further substituted, $R_5$ is hydrogen, substituted or unsubstituted alkyl or alkoxy each having 1 to 20 carbon atoms, alkoxycarbonyl having 2 to 21 carbon atoms, in which the alkoxy moiety can be further substituted, $—SO_2T_3$, $—SO_2N(T_3)_2$ or $—SO_2NT_1T_3$, in which $T_1$ and $T_3$ are as defined above, or $R_5$ conjointly with $R_4$ forms a radical of the formula

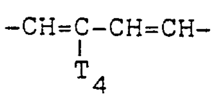

or $R_5$ conjointly with $R_6$ forms a radical of the formula

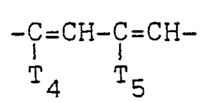

in which $T_4$ is hydrogen, nitro, $—SO_2T_3$, $—SO_2N(T_3)_2$ or $—SO_2NT_1T_3$ and $T_5$ is hydrogen, $—OT_3$, $—SO_2T_3$ or $—SO_2NT_1T_3$, $T_1$ and $T_3$ being as defined above, the total of the carbon atoms in the

substitutents $R_3$, $R_4$, $R_5$ and $R_6$ being at least 8, and $R_{12}$, $R_{22}$ as well as $X_{12}$, $X_{22}$, $X_{32}$ and $X_{42}$ being as defined in claim 3.

5. A process according to claim 4, wherein the oil-soluble coupling component is of the formula (8) and the triazene is of the formula

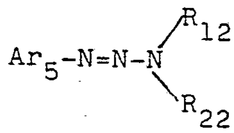

(10)

in which $Ar_5$ is of the formula

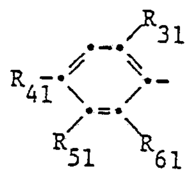

in which $R_{31}$ and $R_{61}$ independently of each other are hydrogen, alkyl or alkoxy each having 1 or 2 carbon atoms, trifluoromethyl, carboxyl, alkoxycarbonyl having 2 to 7 carbon atoms, $-SO_2T_{11}$, $-SO_2N(T_{11})_2$ or $-SO_2NT_{11}T_{21}$, in which $T_{11}$ and $T_{21}$ are as defined in claim 2, or are fluorine, chlorine, bromine, cyano or nitro, $R_{41}$ is hydrogen, alkyl having 1 to 20 carbon atoms, trifluoromethyl, alkoxy having 1 to 20 carbon atoms, which is unsubstituted or substituted by alkoxy having 1 to 6 carbon atoms, or is alkoxycarbonyl having 2 to 21 carbon atoms, in which the alkoxy moiety is unsubstituted or substituted further by alkoxy having 1 to 6 carbon atoms, or is $-SO_2T_{31}$, $-SO_2N(T_{31})_2$ or $-SO_2NT_{11}T_{31}$, in which $T_{31}$ is alkyl having 1 to 20 carbon atoms or $-(CH_2)_p-OT_{11}$, in which $T_{11}$ is as defined above and p is 2, 3 or 4, or $R_4$ is chlorine, bromine, cyano, nitro or a radical of the formula

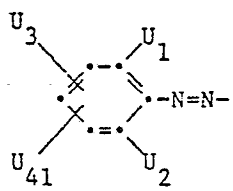

in which $U_{41}$ is hydrogen, alkyl having 1 to 20 carbon atoms, trifluoromethyl, alkoxy having 1 to 20 carbon atoms, which is unsubstituted or substituted by alkoxy having 1 to 6 carbon atoms, or is $-SO_2T_{31}$, $-SO_2NT_{11}T_{31}$, in which $T_{11}$ and $T_{31}$ are as defined above, or is alkoxycarbonyl having 2 to 21 carbon atoms in which the alkoxy moiety is unsubstituted or further substituted by alkoxy having 1 to 6 carbon atoms, $R_{51}$ is hydrogen, alkyl having 1 to 20 carbon atoms, alkoxy having 1 to 20 carbon atoms, which is unsubstituted or substituted by alkoxy having 1 to 12 carbon atoms or by hydroxyl, or is alkoxycarbonyl having 2 to 21 carbon atoms in which the alkoxy moiety is unsubstituted or further substituted by alkoxy having 1 to 12 carbon atoms, or is $-SO_2T_{31}$, $-SO_2N(T_{31})_2$ or $-SO_2NT_{11}T_{31}$, in which $T_{11}$ and $T_{31}$ are as defined above, or $R_{51}$ conjointly with $R_{41}$ forms a radical of the formula

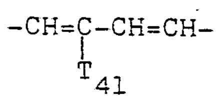

or $R_{51}$ conjointly with $R_{61}$ forms a radical of the formula

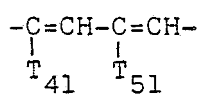

in which $T_{41}$ is hydrogen, nitro, $-SO_2T_{31}$, $-SO_2N(T_{31})_2$ or $-SO_2NT_{11}T_{31}$ and $T_{51}$ is hydrogen, $-OT_{31}$, $-SO_2T_{31}$ or $-SO_2NT_{11}T_{31}$, in which $T_{11}$ and $T_{31}$ are as defined above, the total of the carbon atoms in the substituents $R_{31}$, $R_{41}$, $R_{51}$ and $R_{61}$ being at least 10, and $R_{12}$, $R_{22}$ as well as $U_1$, $U_2$ and $U_3$ being as defined in claim 4.

6. A process according to claim 5, wherein the triazene is of the formula (10) and the oil-soluble coupling component is of the formula

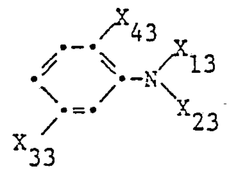

(11)

in which

$X_{13}$ is alkyl having 1 to 16 carbon atoms or a radical of the formula $-CHM_1-CH_2M_2$, in which $M_1$ and $M_2$ are as defined in claim 3, $X_{23}$ is hydrogen, alkyl having 1 to 16 carbon atoms, a radical of the formula $-CHM_1-CH_2M_2$, in which $M_1$ and $M_2$ are as defined above, or is benzyl or phenylethyl,

$X_{33}$ is hydrogen, alkyl having 1 to 4 carbon atoms, alkoxy having 1 to 12 carbon atoms, $-O(C_2H_4O)_n-Z_{11}$ or $-O(CH_2)_m-OZ_{11}$, in which $Z_{11}$, n and m are as defined in claim 3, or is phenoxy, hydroxyl, chlorine, bromine, $-NHCO-Y_3$, $-NHCOH$, $-NHCO-OY_3$, $-NHP(O)(OY_3)_2$ or $-NHSO_2-Y_3$, in which $Y_3$ is as defined in claim 3, and

$X_{43}$ is hydrogen, alkyl having 1 to 4 carbon atoms, chlorine, bromine, alkoxy having 1 to 8 carbon atoms or $-O-(CH_2CH_2O)_n-Z_{11}$, in which $Z_{11}$ and n are as defined above, the total of the carbon atoms in the substituents $X_{13}$, $X_{23}$, $X_{33}$ and $X_{43}$ being at least 10.

7. A process according to claim 6, wherein the triazene is of the formula

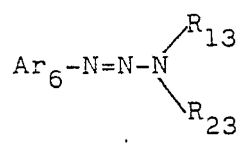

(12)

and the oil-soluble coupling component is of the formula

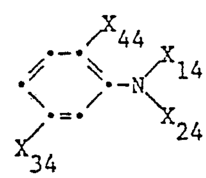

(13)

in which $Ar_6$ is of the formula

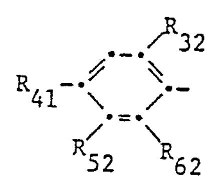

in which $R_{32}$ and $R_{62}$ independently of each other are hydrogen, methyl, methoxy, trifluoromethyl, alkoxycarbonyl having 2 to 4 carbon atoms, $-SO_2T_{13}$, $-SO_2N(T_{13})_2$ or $-SO_2NT_{13}T_{23}$, in which $T_{13}$ and $T_{23}$ independently of each other are hydrogen or alkyl having 1 or 2 carbon atoms, or $R_{32}$ and $R_{62}$ are fluorine, chlorine, bromine, cyano or nitro, $R_{52}$ is hydrogen, alkyl having 1 to 20 carbon atoms, $-OT_{31}$, $-CO-OT_{31}$ or $-SO_2T_{31}$, in which $T_{31}$ is as defined in claim 5 or $R_{52}$ conjointly with $R_{41}$ forms a radical of the formula

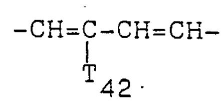

or $R_{52}$ conjointly with $R_{62}$ forms a radical of the formula

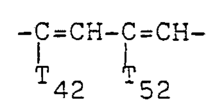

in which $T_{42}$ is hydrogen, nitro, $-SO_2T_{32}$ or $-SO_2N(T_{32})_2$ and $T_{52}$ is hydrogen, $-OT_{32}$, $-SO_2T_{32}$, or $-SO_2NT_{12}T_{32}$, in which $T_{32}$ is alkyl having 1 to 10 carbon atoms or $-(CH_2)_p-OT_{12}$ and p and $T_{12}$ as well as $R_{41}$ are as defined in claim 5, the total of the carbon atoms in the substituents $R_{32}$, $R_{41}$, $R_{52}$ and $R_{62}$ being at least 10,

$R_{13}$ is alkyl having 1 to 4 carbon atoms, alkyl having 1 or 2 carbon atoms, which is unsubstituted or substituted by hydroxyl, cyano, carboxyl, $—COOCH_3$ or methoxy, or is $—(CH_2CH_2O)_r—L_{12}$ or $—OL_{12}$, in which $L_{12}$ is alkyl having 1 to 6 carbon atoms and r is as defined in claim 2, or $R_{13}$ is phenyl, tolyl, chlorophenyl, bromophenyl or methoxyphenyl,

$R_{23}$ is alkyl having 1 to 4 carbon atoms, alkyl having 1 or 2 carbon atoms, which is unsubstituted or substituted by hydroxyl, cyano, carboxyl, $—COOCH_3$ or methoxy, or is $—(CH_2CH_2O)_r—L_{12}$, in which $L_{12}$ and r are as defined above, or $R_{23}$ is phenyl, tolyl, chlorophenyl or bromophenyl, or $R_{23}$ and $R_{13}$ form a radical of the formula $—(CH_2)_4—$, $—(CH_2)_5—$, $—C_2H_4—O—C_2H_4—$, $—C_2H_4—NH—C_2H_4—$, $—C_2H_4—N(CH_3)—C_2H_4—$, $—CH{=}N—CH{=}CH—$ or $—CH{=}CH—CH{=}CH—$, and

$X_{14}$ is alkyl having 1 to 16 carbon atoms or a radical of the formula $—CHM_{11}—CH_2M_{21}$, in which $M_{11}$ is hydrogen, methyl or ethyl and $M_{21}$ is cyano or $—OM_{31}$, in which $M_{31}$ is hydrogen, alkyl having 1 to 6 carbon atoms or phenyl which is unsubstituted or substituted by alkyl having 1 to 4 carbon atoms,

$X_{24}$ is hydrogen, alkyl having 1 to 16 carbon atoms or benzyl,

$X_{34}$ is hydrogen, alkyl having 1 to 4 carbon atoms, phenoxy, chlorine, hydroxyl, alkoxy having 1 to 6 carbon atoms, $—C—CH_2CH_2—OH$, $—O—CH_2CH_2OZ_{11}$, $—NHCO—Y_4$, $—NHCOH$, $—NHCO—CH_2CH_2—OH$, $—NHCO—CH_2CH_2—OZ_{11}$, $NHP(O)(OY_4)_2$, $—NHP(O)(OC_6H_4Y_5)_2$ or $—NHSO_2Y_4$, in which $Y_4$ is alkyl having 1 to 16 carbon atoms, $Y_5$ is hydrogen or alkyl having 1 to 6 carbon atoms and $Z_{11}$ is as defined in claim 6, and

$X_{44}$ is hydrogen, alkyl or alkoxy each having 1 to 4 carbon atoms or $—O—(CH_2CH_2O)_n—Z_{11}$, in which $Z_{11}$ and n are as defined in claim 6, the total of the carbon atoms in the substituents $X_{14}$, $X_{24}$, $X_{34}$ and $X_{44}$ being at least 10.

8. A process according to claim 2, wherein the oil-soluble coupling compound is of the formula (8) and the triazene is of the formula

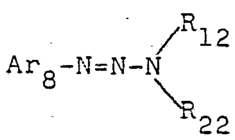

(15)

in which $Ar_8$ is a radical of the formula

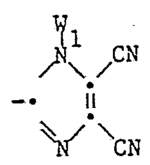

,

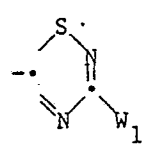

or

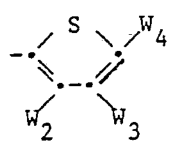

in which $W_1$ is substituted or unsubstituted alkyl having 6 to 18 carbon atoms or substituted or unsubstituted phenyl, $W_2$ is alkoxycarbonyl having 2 to 25 carbon atoms, nitro or cyano, and $W_3$ and $W_4$ independently of each other are hydrogen or alkyl having 1 to 6 carbon atoms, or $W_3$ conjointly with $W_4$ forms a radical of the formula

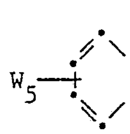

,

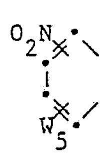

or

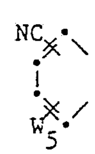

in which

$W_5$ is alkyl having 6 to 18 carbon atoms, the total of the carbon atoms in the substituents $W_1$, $W_2$, $W_3$ and $W_4$ being at least 8, and

$R_{12}$ and $R_{22}$ being as defined in claim 3.

9. A process according to claim 1, wherein the material, in at least one silver halide emulsion layer, or in one adjacent layer, contains an oil-soluble triazene of the formula (1) or (2) and an oil-soluble coupling component of the formula (3), the triazene and the coupling component having been incorporated, in solution in a water-immiscible solvent or solvent mixture, in a finely divided form into the light-sensitive silver halide emulsion layer or layers or into a layer adjacent to the latter, and, after exposure and development of the image silver, the material is treated with an aqueous processing bath which, for dye formation, contains

a) a strong acid,
b) a cation phase transfer catalyst, optionally for a simultaneous dye bleaching
c) a ligand which forms silver complexes,
d) a dye bleach catalyst,
e) an antioxidising agent, optionally for a simultaneous silver bleaching
f) an oxidising agent and optionally for simultaneous fixing
g) a solvent for silver halide.

10. A process according to claim 9, wherein the cation phase transfer catalyst is a strong inorganic acid, a perhalogenated aliphatic acid, a benzenesulfonic acid which is substituted by 1 or 2 alkyl or alkoxy groups each having 1 to 12 carbon atoms, a benzenesulfonic acid substituted by 1 to 3 halogen atoms, an alkylsulfonic acid which may be halogenated, having 1 to 12 carbon atoms, a monoalkylsulfuric acid having 1 to 12 carbon atoms, or an alkali metal salt or ammonium salt of these acids.

11. A process according to claim 10, wherein the cation phase transfer catalyst is employed in quantities of 10 to 200 g, preferably 10 to 100 g, per litre of processing solution.

12. A photographic material for the process for the production of photographic colour images according to any one of claims 1 to 11, which material contains, in an opaque or transparent base, at least one silver halide emulsion layer and, in the same layer or in an adjacent layer, a dispersion of the oil-soluble triazene of the formula (1) or (2) and of the oil-soluble component of the formula (3) in a water-immiscible solvent or silvent mixture.

13. A formulation for processing the photographic material according to claim 12, which contains
a) an alkylsulfonic or arylsulfonic acid, sulfuric acid, sulfamic acid or optionally a mixture of these acids,
b) trifluoroacetic or trichloroacetic or perchloric acid, optionally
c) a ligand which forms silver complexes,
d) a dye bleach catalyst,
e) an anti-oxidising agent, optionally
f) an oxidising agent and optionally
g) a solvent for silver halide.

**Revendications**

1. Procédé de production d'images photographiques en couleurs par le procédé de blanchiment des couleurs à l'argent par exposition, développement de l'argent, formation de couleurs, blanchiment des couleurs, blanchiment de l'argent et fixage d'une matière photographique, qui contient sur un support transparent ou opaque au moins une couche contenant une émulsion d'halogénure d'argent photosensible, la formation de couleurs pouvant être effectuée le cas échéant en même temps que le blanchiment des couleurs, le blanchiment de l'argent et le fixage, et le blanchiment de l'argent pouvant être effectué le cas échéant en même temps que le blanchiment des couleurs et/ou le fixage dans un seul bain de traitement, caractérisé par le fait que la ou les couche(s) d'émulsion d'halogénure d'argent photosensible(s) ou une couche ou chacune des couches voisine(s) de cette couche ou de ces couches d'émulsion d'halogénure d'argent contient (contiennent) dispersés dans de l'huile un triazène oléosoluble répondant aux formules

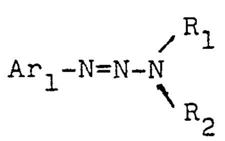 (1) ou 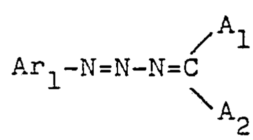 (2)

et un constituant de copulation oléosoluble répondant à la formule

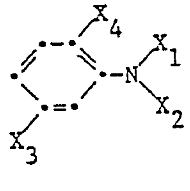 (3)

où

$Ar_1$ est un aryle éventuellement substitué où un radical aromatique, hétérocyclique éventuellement substitué,

$R_1$ est l'hydrogène, un radical alkyle en $C_1$-$C_6$ éventuellement substitué, $-(CH_2CH_2O)_r-L_1$, ou

—$OL_1$, où $L_1$ est un radical alkyle en $C_1$-$C_{12}$ et r est 1, 2 ou 3, un aryle éventuellement substitué, un hydroxyle ou un radical répondant à la formule

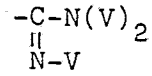

où V est l'hydrogène ou un radical alkyle en $C_1$-$C_{12}$,

$R_2$ est un radical alkyle en $C_1$-$C_6$ éventuellement substitué, —$(CH_2CH_2O)_r$—$L_1$, où $L_1$ et r ont les significations indiquées ci-dessus, ou un radical aryle éventuellement substitué, ou

$R_1$ et $R_2$ forment avec l'atome d'azote auquel ils sont liés un cycle à 5, 6 ou 7 maillons, éventuellement substitué, saturé ou insaturé, contenant le cas échéant un autre hétéro-atome,

$A_1$ et $A_2$ sont indépendamment l'un de l'autre un radical répondant à la formule

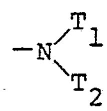

dans laquelle $T_1$ et $T_2$ sont indépendamment l'un de l'autre l'hydrogène, un radical alkyle en $C_1$-$C_{12}$ éventuellement substitué ou un radical aryle éventuellement substitué,

$X_1$ et $X_2$ sont indépendamment l'un de l'autre l'hydrogène ou un radical alkyle en $C_1$-$C_{20}$ éventuellement substitué,

$X_3$ est l'hydrogène, un radical alkyle en $C_1$-$C_6$ éventuellement substitué, un radical alcoxy en $C_1$-$C_{20}$ éventuellement substitué, —$O(C_2H_4O)_n$—H, —$O(CH_2)_m$—OH, —O—$(CH_2)_m$—$OZ_1$ ou —O—$(C_2H_4O)_n$—$Z_1$ où $Z_1$ est un radical alkyle en $C_1$-$C_8$ et n un nombre entier de 1 à 5, et m est 2, 3 ou 4, un radical aryloxy éventuellement substitué, un hydroxyle, un halogène, —NHCO—$Y_1$, —NHCOH, —NHCO—$OY_1$, —NHP(O)$(OY_1)_2$ ou —$NHSO_2$—$Y_1$, où $Y_1$ est un radical alkyle en $C_1$-$C_{16}$ éventuellement substitué, —$O(CH_2)_m$—OH, —O—$(C_2H_4O)_n$—H, —O—$(CH_2)_m$—$OZ_1$ ou —O—$(C_2H_4O)_n$—$Z_1$, où $Z_1$, m et n ont les significations indiquées ci-dessus, ou $Y_1$ est un radical aryle éventuellement substitué, et

$X_4$ est l'hydrogène, un radical alkyle en $C_1$-$C_{12}$ éventuellement substitué, —$O(C_2H_4O)_n$—H, —$O(CH_2)_m$—OH, —$O(CH_2)_m$—$OZ_1$ ou —O—$(C_2H_4O)_n$—$Z_1$, où $Z_1$, m et n ont les significations indiquées ci-dessus, un radical alcoxy en $C_1$-$C_{16}$ éventuellement substitué, un radical aryloxy éventuellement substitué ou un halogène, et la somme des atomes de carbone dans les substituants $X_1$, $X_2$, $X_3$ et $X_4$ est d'au moins 10 ;

et en ce que la matière est traitée après développement de l'argent de l'image par une solution de traitement acide, qui contient un catalyseur de transfert de phase, capable de transférer des cations.

2. Procédé selon la revendication 1, caractérisé par le fait que le triazène répond aux formules :

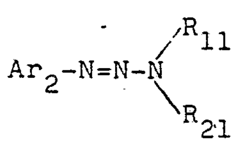 (4) ou 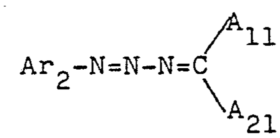 (5)

et le constituant de copulation oléosoluble répond à la formule :

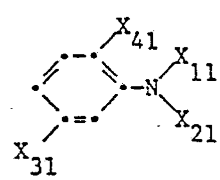 (6)

dans lesquelles

$Ar_2$ est un radical phényle éventuellement substitué, un radical naphtyle éventuellement substitué ou un radical aromatique éventuellement substitué contenant 1 à 3 hétéro-atomes,

$R_{11}$ est un radical alkyle en $C_1$-$C_6$ éventuellement substitué, —$(CH_2CH_2O)_r$—$L_{11}$ ou —$OL_{11}$, où $L_{11}$ est un radical alkyle en $C_1$-$C_8$, et r est 1, 2 ou 3, un radical phényle éventuellement substitué ou un hydroxyle, et

$R_{21}$ est un radical alkyle en $C_1$-$C_6$ éventuellement substitué, —$(CH_2CH_2O)_r$—$L_{11}$, où $L_{11}$ et r ont les significations indiquées ci-dessus, ou un radical phényle éventuellement substitué, ou bien $R_{21}$ et $R_{11}$ forment avec l'atome d'azote auquel ils sont liés un cycle à 5, 6 ou 7 maillons éventuellement substitué, saturé ou insaturé, contenant éventuellement un autre hétéro-atome, et

$A_{11}$ et $A_{21}$ sont indépendamment l'un de l'autre un radical répondant à la formule

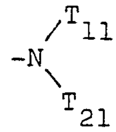

dans laquelle $T_{11}$ et $T_{21}$ sont indépendamment l'un de l'autre l'hydrogène, un radical alkyle en $C_1$-$C_{12}$ ou un phényle,

$X_{11}$ et $X_{21}$ sont indépendamment l'un de l'autre l'hydrogène ou un radical alkyle en $C_1$-$C_{16}$ éventuellement substitué,

$X_{31}$ est l'hydrogène, un radical alkyle en $C_1$-$C_4$ éventuellement substitué, un radical alcoxy en $C_1$-$C_{16}$ éventuellement substitué, $O(C_2H_4O)_n$—H, —$O(CH_2)_m$—OH, —$O(CH_2)_m$—$OZ_{11}$ ou —O—$(C_2H_4O)_n$—$Z_{11}$, où $Z_{11}$ est un radical alkyle en $C_1$-$C_4$ et m et n ont la signification indiquée dans la revendication 1, un radical phénoxy éventuellement substitué, un hydroxyle, un halogène, —NHCO—$Y_2$, —NHCO—$OY_2$, —NHCOH—, $NHP(O)(OY_2)_2$ ou —$NHSO_2$-$Y_2$, où $Y_2$ est un radical alkyle en $C_1$-$C_{16}$ éventuellement substitué, —$(C_2H_4O)_n$—H, —$(CH_2)_m$—OH, —$(CH_2)_m$—$OZ_{11}$ ou —$(C_2H_4O)_n$—$Z_{11}$, où $Z_{11}$, n et m ont les significations indiquées ci-dessus, ou $Y_2$ est un radical phényle éventuellement substitué, et

$X_{41}$ est l'hydrogène, un radical alkyle en $C_1$-$C_8$ éventuellement substitué, —$O(C_2H_4O)_n$—H, —$O(CH_2)_m$—OH, —$O(CH_2)_m$—$OZ_{11}$ ou —O—$(C_2H_4O)_n$-$Z_{11}$, où $Z_{11}$, m et n ont les significations indiquées ci-dessus, un radical alcoxy en $C_1$-$C_{16}$ éventuellement substitué, un radical phénoxy éventuellement substitué ou un halogène, et la somme des atomes de carbone dans les substituants $X_{11}$, $X_{21}$, $X_{31}$ et $X_{41}$ est d'au moins 10.

3. Procédé selon la revendication 2, caractérisé par le fait que le triazène répond à la formule :

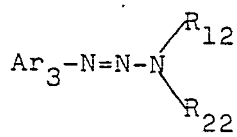

(7)

et le constituant de copulation oléosoluble répond à la formule

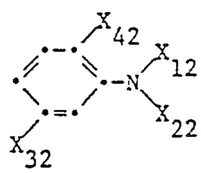

(8)

dans laquelle

$Ar_3$ est un radical phényle éventuellement substitué ou un radical naphtyle éventuellement substitué,

$R_{12}$ est un radical alkyle en $C_1$-$C_6$ éventuellement substitué par un hydroxyle, un groupe cyano, méthoxy, carboxyle, alcoxycarbonyle en $C_2$-$C_7$, la partie alcoxy pouvant le cas échéant être encore substituée, ou —$SO_3M$, où M est l'hydrogène, l'ammonium ou un métal alcalin, —$(CH_2CH_2O)_r$—$L_{11}$ ou —$OL_{11}$, où $L_{11}$ et r ont les significations indiquées dans la revendication 2, un radical phényle substitué le cas échéant par un alkyle en $C_1$-$C_6$, un halogène ou un alcoxy, ou un radical hydroxyle, et

$R_{22}$ est un radical alkyle en $C_1$-$C_6$ éventuellement substitué par un hydroxyle, un groupe cyano, méthoxy, carboxyle, alcoxycarbonyle en $C_2$-$C_7$, la partie alcoxy pouvant le cas échéant être encore substituée, ou —$SO_3M$, où M est l'hydrogène, l'ammonium ou un métal alcalin, —$(CH_2CH_2O)_r$—$L_{11}$, où $L_{11}$ et r ont les significations indiquées dans la revendication 2, ou un phényle substitué le cas échéant par un alkyle en $C_1$-$C_6$ ou un halogène, ou bien $R_{22}$ et $R_{12}$ forment avec l'atome d'azote auquel ils sont liés un cycle à 5, 6 ou 7 maillons substitué le cas échéant par un radical alkyle en $C_1$-$C_6$, saturé ou non saturé, contenant le cas échéant un atome d'azote ou d'oxygène comme autre hétéro-atome,

$X_{12}$ et $X_{22}$ sont indépendamment l'un de l'autre l'hydrogène, un radical alkyle en $C_1$-$C_{16}$, un radical benzyle, un radical phényléthyle ou un radical répondant à la formule —$CHM_1$—$CH_2M_2$, où $M_1$ est l'hydrogène ou un radical alkyle en $C_1$-$C_4$ et $M_2$ est un radical cyano ou un radical répondant à la formule —$OM_3$ ou —$CO_2M_3$, où $M_3$ est l'hydrogène, un alkyle en $C_1$-$C_{16}$, un phényle substitué le cas échéant par un alkyle en $C_1$-$C_4$ ou un radical répondant aux formules —$(C_22H_4O)_nM_4$ ou —$(CH_2)_m$—$OM_4$, où $M_4$ est l'hydrogène ou un radical alkyle en $C_1$-$C_4$ éventuellement substitué, et n et m ont les significations indiquées ci-dessus,

$X_{32}$ est l'hydrogène, un radical alkyle en $C_1$-$C_4$, un radical alcoxy en $C_1$-$C_{16}$, —$O(C_2H_4O)_n$—H, —$O(CH_2)_m$—OH, —$O(C_2H_4O)_m$—$Z_{11}$ ou —$O(CH_2)_m$—$OZ_{11}$, où $Z_{11}$ a la signification indiquée dans la revendication 2, et n et m ont les significations indiquées ci-dessus, un radical phénoxy, un hydroxyle, un halogène, —NHCO—$Y_3$, —NHCOH, —NHCO—$OY_3$, —$NHP(O)(OY_3)_2$ ou —$NHSO_2$—$Y_3$, où $Y_3$ est un

radical alkyle en $C_1$-$C_{16}$, un radical phényle substitué le cas échéant par un radical alkyle en $C_1$-$C_4$, ou un radical répondant aux formules $-(C_2H_4O)_n-H$, $-(CH_2)_m-OH$, $-(C_2H_4O)_n-Z_{11}$ ou $-(CH_2)_m-OZ_{11}$, où $Z_{11}$, n et m ont les significations indiquées ci-dessus, et

$X_{42}$ est l'hydrogène, un radical alkyle en $C_1$-$C_6$, un halogène, un radical alcoxy en $C_1$-$C_8$ ou $-O(C_2H_4O)_n-H$, $-O(CH_2)_m-OH$, $-O(CH_2)_m-OZ_{11}$ ou $-O(C_2H_4O)_n-Z_{11}$, où $Z_{11}$, n et m ont les significations indiquées ci-dessus, ou un radical phénoxy éventuellement substitué, et la somme des atomes de carbone dans les substituants $X_{12}$, $X_{22}$, $X_{32}$ et $X_{42}$ étant d'au moins 10.

4. Procédé selon la revendication 3, caractérisé par le fait que le constituant de copulation oléosoluble répond à la formule (8) et le triazène à la formule :

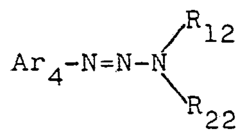
(9)

dans laquelle,

$Ar_4$ répond à la formule

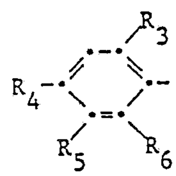

dans laquelle $R_3$ et $R_6$ sont indépendamment l'un de l'autre l'hydrogène, un alkyle ou un alcoxy chacun en $C_1$-$C_4$ éventuellement substitué, un carboxyle, un alcoxycarbonyle en $C_2$-$C_{12}$, $-SO_2T_1$, $-SO_2N(T_1)_2$ ou $-SO_2NR_1T_2$, où $T_1$ et $T_2$ ont les significations indiquées dans la revendication 1, un halogène, un cyano ou un nitro ; $R_4$ est l'hydrogène, un radical alkyle ou alcoxy chacun en $C_1$-$C_{20}$ éventuellement substitué, un radical alcoxycarbonyle en $C_2$-$C_{21}$, la partie alcoxy étant le cas échéant encore substituée, $-SO_2T_3$, $-SO_2N(T_3)_2$ ou $-SO_2NT_1T_3$, où $T_3$ est un radical alkyle en $C_1$-$C_{20}$ ou $-(CH_2)_p-OT_1$, $T_1$ a les significations indiquées ci-dessus, et p est 2, 3 ou 4, ou $R_4$ est un halogène, un groupe trifluorométhyle, un groupe cyano, un groupe nitro ou un radical répondant à la formule :

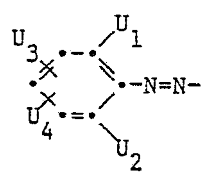

dans laquelle $U_1$ et $U_2$ sont indépendamment l'un de l'autre l'hydrogène, $-SO_2U_5$, où $U_5$ est un radical alkyle en $C_1$-$C_4$, un halogène, un radical cyano ou nitro, $U_3$ est l'hydrogène, un halogène ou un nitro, et $U_4$ un radical alkyle ou alkoxy chacun en $C_1$-$C_{20}$ éventuellement substitué, $-SO_2T_3$, $-SO_2NT_1T_3$, où $T_1$ et $T_3$ ont les significations indiquées ci-dessus, ou un radical alcoxycarbonyle en $C_2$-$C_{21}$, la partie alcoxy pouvant le cas échéant être encore substituée ; $R_5$ est l'hydrogène, un radical alkyle ou alcoxy chacun en $C_1$-$C_{20}$ éventuellement substitué, un radical alcoxycarbonyle en $C_2$-$C_{21}$, la partie alcoxy pouvant le cas échéant être encore substituée, $-SO_2T_3$, $-SO_2N(T_3)_2$ ou $-SO_2NT_1T_3$, où $T_1$ et $T_3$ ont les significations indiquées ci-dessus, ou $R_5$ forme avec $R_4$ un radical répondant à la formule

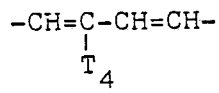

ou $R_5$ forme avec $R_6$ un radical répondant à la formule

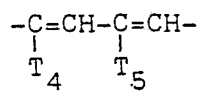

dans lesquelles $T_4$ est l'hydrogène, un groupe nitro, $-SO_2T_3$, $-SO_2N(T_3)_2$ ou $-SO_2NT_1T_3$, et $T_5$ est l'hydrogène, $-OT_3$, $-SO_2T_3$ ou $-SO_2NT_1T_3$, où $T_1$ et $T_3$ ont les significations indiquées ci-dessus, et

où la somme des atomes de carbone dans les substituants $R_3$, $R_4$, $R_5$ et $R_6$ est d'au moins 8, et $R_{12}$, $R_{22}$ ainsi que $X_{12}$, $X_{22}$, $X_{32}$ et $X_{42}$ ont les significations indiquées dans la revendication 3.

5. Procédé selon la revendication 4, caractérisé par le fait que le constituant de copulation oléosoluble répond à la formule (8) et le triazène à la formule :

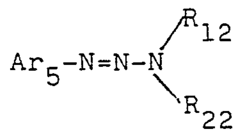

(10)

dans laquelle

$Ar_5$ répond à la formule

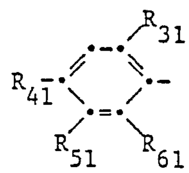

dans laquelle $R_{31}$ et $R_{61}$ sont indépendamment l'un de l'autre l'hydrogène, un radical alkyle ou alcoxy chacun en $C_1$ ou $C_2$, un radical trifluorométhyle, carboxyle, alcoxycarbonyle en $C_2$-$C_7$, $—SO_2T_{11}$, $—SO_2N(T_{11})_2$ ou $—SO_2NT_{11}T_{21}$, où $T_{11}$ et $T_{21}$ ont les significations indiquées dans la revendication 2, le fluor, le chlore, le brome, un radical cyano ou nitro ; $R_{41}$ est l'hydrogène, un radical alkyle en $C_1$-$C_{20}$, un radical trifluorométhyle, un alcoxy en $C_1$-$C_{20}$ éventuellement substitué par un alcoxy en $C_1$-$C_6$, un radical alcoxycarbonyle en $C_2$-$C_{21}$, la partie alcoxy étant éventuellement substituée en outre par un alcoxy en $C_1$-$C_6$, $—SO_2T_{31}$, $—SO_2N(T_{31})_2$ ou $—SO_2NT_{11}T_{31}$ où $T_{31}$ est un radical alkyle en $C_1$-$C_{20}$ ou $—(CH_2)_p—OT_{11}$, où $T_{11}$ a les significations indiquées ci-dessus, et p est 2, 3 ou 4, ou $R_4$ est le chlore, le brome, un groupe cyano ou nitro ou un radical répondant à la formule

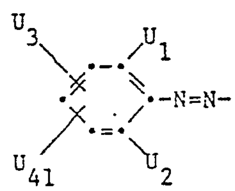

dans laquelle $U_{41}$ est l'hydrogène, un radical alkyle en $C_1$-$C_{20}$, un radical trifluorométhyle, un radical alcoxy en $C_1$-$C_{20}$ éventuellement substitué par un radical alcoxy en $C_1$-$C_6$, $—SO_2T_{31}$, $—SO_2NT_{11}T_{31}$, où $T_{11}$ et $T_{31}$ ont les significations indiquées ci-dessus, ou un radical alcoxycarbonyle en $C_2C_{21}$, la partie alcoxy étant éventuellement encore substituée par un alcoxy en $C_1$-$C_6$ ; $R_{51}$ est l'hydrogène, un radical alkyle en $C_1$-$C_{20}$, un radical alcoxy en $C_1$-$C_{20}$ éventuellement substitué par un radical alcoxy en $C_1$-$C_{12}$ ou un hydroxyle, un alcoxycarbonyle en $C_2$-$C_{21}$, la partie alcoxy pouvant le cas échéant être encore substituée par un alcoxy en $C_1$-$C_{12}$, $—SO_2T_{31}$, $—SO_2N(T_{31})_2$ ou $—SO_2NT_{11}T_{31}$, où $T_{11}$ et $T_{31}$ ont les significations indiquées ci-dessus, ou bien $R_{51}$ forme avec $R_{41}$ un radical répondant à la formule

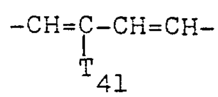

ou bien $R_{51}$ avec $R_{61}$ forment un radical répondant à la formule

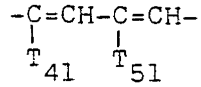

où $T_{41}$ est l'hydrogène, un groupe nitro, $—SO_2T_{31}$, $—SO_2N(T_{31})_2$ ou $—SO_2NT_{11}T_{31}$, et $T_{51}$ est l'hydrogène, $—OT_{31}$, $—SO_2T_{31}$ ou $—SO_2NT_{11}T_{31}$ où $T_{11}$ et $T_{31}$ ont les significations indiquées ci-dessus, la somme des atomes de carbone dans les substituants $R_{31}$, $R_{41}$, $R_{51}$ et $R_{61}$ étant d'au moins 10, et $R_{12}$, $R_{22}$ ainsi que $U_1$, $U_2$ et $U_3$ ayant les significations indiquées dans la revendication 4.

6. Procédé selon la revendication 5, caractérisé par le fait que le triazène répond à la formule (10) et le constituant de copulation oléosoluble à la formule

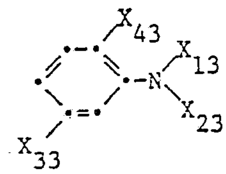

(11)

dans laquelle

$X_{13}$ est un radical alkyle en $C_1$-$C_{16}$ ou un radical répondant à la formule $-CHM_1-CH_2M_2$ et $M_2$ ont les significations indiquées dans la revendication 3,

$X_{23}$ est l'hydrogène, un radical alkyle en $C_1$-$C_{16}$, un radical répondant à la formule $-CHM_1-CH_2M_2$ où $M_1$ et $M_2$ ont les significations indiquées ci-dessus, un radical benzyle ou phényléthyle,

$X_{33}$ est l'hydrogène, un radical alkyle en $C_1$-$C_4$, un radical alcoxy en $C_1$-$C_{12}$, $-O(C_2H_4O)_n-Z_{11}$ ou $-O(CH_2)_m-OZ_{11}$, où $Z_{11}$, n et m ont les significations indiquées dans la revendication 3, un radical phénoxy, un hydroxyle, le chlore, le brome, $-NHCO-Y_3$, $-NHCOH$, $-NHCO-OY_3$, $NHP(O)(OY_3)_2$ ou $-NHSO_2-Y_3$, où $Y_3$ a les significations indiquées dans la revendication 3,

$X_{43}$ est l'hydrogène, un radical alkyle en $C_1$-$C_4$, le chlore, le brome, un radical alcoxy en $C_1$-$C_8$ ou $-O-(CH_2CH_2O)_n-Z_{11}$, où $Z_{11}$ et n ont la signification indiquée ci-dessus, et la somme des atomes de carbone dans les substituants $X_{13}$, $X_{23}$, $X_{33}$ et $X_{43}$ étant d'au moins 10.

7. Procédé selon la revendication 6, caractérisé par le fait que le triazène répond à la formule :

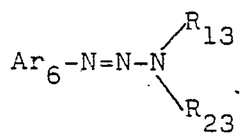

(12)

et le constituant oléosoluble à la formule :

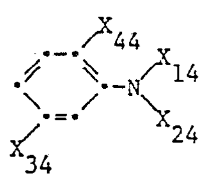

(13)

$Ar_6$ répondant à la formule

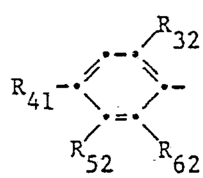

dans laquelle $R_{32}$ et $R_{62}$ désignent indépendamment l'un de l'autre l'hydrogène, un radical méthyle, méthoxy, trifluorométhyle, alcoxycarbonyle en $C_2$-$C_4$, $-SO_2T_{13}$, $-SO_2N(T_{13})_2$ ou $-SO_2NT_{13}T_{23}$, où $T_{13}$ et $T_{23}$ désignent indépendamment l'un de l'autre l'hydrogène ou un radical alkyle en $C_1$-$C_2$, le fluor, le chlore, le brome, un groupe cyano ou nitro ; $R_{52}$ est l'hydrogène, un radical alkyle en $C_1$-$C_{20}$, $-OT_{31}$, $-CO-OT_{31}$ ou $-SO_2T_{31}$ où $T_{31}$ a les significations indiquées dans la revendication 5, ou bien $R_{52}$ forme avec $R_{41}$ un radical répondant à la formule

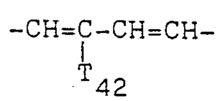

ou $R_{52}$ forme avec $R_{62}$ un radical répondant à la formule

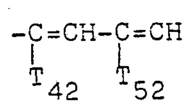

où $T_{42}$ est l'hydrogène, un groupe nitro, $-SO_2T_{32}$ ou $-SO_2N(T_{32})_2$ et $T_{52}$ est l'hydrogène, $-OT_{32}$, $-SO_2T_{32}$ ou $-SO_2NT_{12}T_{32}$, où $T_{32}$ est un radical alkyle en $C_1$-$C_{10}$ ou $-(CH_2)_p-OT_{12}$, et p et $T_{12}$ ainsi que $R_{41}$ ont les significations indiquées dans la revendication 5, la somme des atomes de carbone dans les substituants $R_{32}$, $R_{41}$, $R_{52}$ et $R_{62}$ étant d'au moins 10,

$R_{13}$ est un radical alkyle en $C_1$-$C_4$, un radical alkyle en $C_1$-$C_2$ éventuellement substitué par un groupe hydroxyle, cyano, carboxyle, $-COOCH_3$ ou méthoxy, $-(CH_2CH_2O)_r-L_{12}$ ou $-OL_{12}$, où $L_{12}$ est un radical alkyle en $C_1$-$C_6$ et r a les significations indiquées dans la revendication 2, un radical phényle, tolyle, chlorophényle, bromophényle ou méthoxyphényle,

$R_{23}$ est un radical alkyle en $C_1$-$C_4$, un radical alkyle en $C_1$-$C_2$ éventuellement substitué par un groupe hydroxyle, cyano, carboxyle, $-COOCH_3$ ou méthoxy, $-(CH_2CH_2O)_r-L_{12}$, où $L_{12}$ et r ont les significations indiquées ci-dessus, un radical phényle, tolyle, chlorophényle ou bromophényle, ou bien $R_{23}$ et $R_{13}$ forment un radical répondant aux formules $-(CH_2)_4-$, $-(CH_2)_5-$, $-C_2H_4-O-C_2H_4-$, $C_2H_4-NH-C_2H_4-$, $-C_2H_4-N(CH_3)-C_2H_4-$, $-CH=N-CH=CH-$ ou $-CH=CH-CH=CH-$, et

$X_{14}$ est un radical alkyle en $C_1$-$C_{16}$ ou un radical répondant à la formule $-CHM_{11}-CH_2M_{21}$, où $M_{11}$ est l'hydrogène, un radical méthyle ou éthyle et $M_{21}$ désigne un radical cyano ou $-OM_{31}$, où $M_{31}$ est l'hydrogène, un radical alkyle en $C_1$-$C_6$, ou un radical phényle substitué le cas échéant par un radical alkyle en $C_1$-$C_4$,

$X_{24}$ est l'hydrogène, un radical alkyle en $C_1$-$C_{16}$ ou un radical benzyle,

$X_{34}$ est l'hydrogène, un radical alkyle en $C_1$-$C_4$, un radical phénoxy, chloro, hydroxyle, alcoxy en $C_1$-$C_6$, $-O-CH_2CH_2-OH$, $-O-CH_2CH_2-OZ_{11}$, $-NHCO-Y_4$, $-NHCOH$, $-NHCO-CH_2CH_2-OH$, $-NHCO-CH_2CH_2-OZ_{11}$, $-NHP(O)(OY_4)_2$, $-NHP(O)(OC_6H_4Y_5)_2$ ou $-NHSO_2Y_4$, où $Y_4$ est un radical alkyle en $C_1$-$C_{16}$ ; $Y_5$ est l'hydrogène ou un radical alkyle en $C_1$-$C_6$, et $Z_{11}$ a les significations indiquées dans la revendication 6, et

$X_{44}$ est l'hydrogène, un radical alkyle ou alcoxy chacun en $C_1$-$C_4$, ou $-O-(CH_1CH_2O)_n-Z_{11}$, où $Z_{11}$ et n ont les significations indiquées dans la revendication 6 et la somme des atomes de carbone dans les substituants $X_{14}$, $X_{24}$, $X_{34}$ et $X_{44}$ étant d'au moins 10.

8. Procédé selon la revendication 2, caractérisé par le fait que le substituant oléosoluble répond à la formule (8) et le triazène à la formule

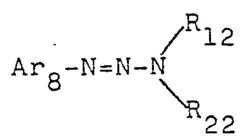
(15)

dans laquelle

$Ar_8$ est un radical de formule :

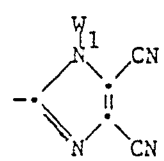 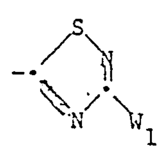 ou 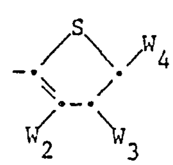

dans laquelle $W_1$ est un radical alkyle en $C_6$-$C_{18}$ éventuellement substitué ou un radical phényle éventuellement substitué, $W_2$ est un radical alcoxycarbonyle en $C_2$-$C_{25}$, nitro ou cyano, et $W_3$ et $W_4$ sont indépendamment l'un de l'autre l'hydrogène ou un radical alkyle en $C_1$-$C_6$, ou bien

$W_3$ forme avec $W_4$ un radical répondant aux formules :

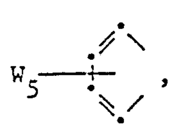 , 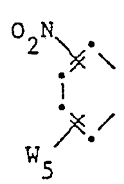 ou 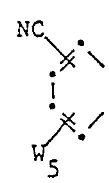

dans lesquelles

$W_5$ est un radical alkyle en $C_6$-$C_{18}$, la somme des atomes de carbone dans les substituants $W_1$, $W_2$, $W_3$ et $W_4$ étant d'au moins 8, et

$R_{12}$ et $R_{22}$ ont les significations indiquées dans la revendication 3.

9. Procédé selon la revendication 1, caractérisé par le fait que la matière contient dans au moins une couche d'émulsion d'halogénure d'argent ou dans chacune des couches voisines un triazène oléosoluble répondant à la formule (1) ou (2) et un constituant de copulation oléosoluble répondant à la formule (3), le

triazène et le constituant de copulation dissous dans un solvant ou dans un mélange de solvants non miscible à l'eau étant incorporés sous une forme finement divisée dans la ou les couches(s) d'émulsion d'halogénure d'argent photosensible(s) ou dans une couche voisine de celle(s)-ci, et par le fait que la matière, après exposition et développement de l'argent de l'image est traitée dans un bain de traitement aqueux qui contient pour la formation de la couleur :

a) un acide fort et

b) un catalyseur de transfert de phase des cations,

le cas échéant pour un blanchiment simultané des couleurs

c) un ligand qui forme des complexes d'argent,

d) un catalyseur de blanchiment des couleurs, et

e) un agent de protection contre l'oxydation,

le cas échéant pour le blanchiment simultané de l'argent

f) un agent oxydant et

le cas échéant pour le fixage simultané

g) un solvant de l'halogénure d'argent.

10. Procédé selon la revendication 9, caractérisé par le fait que le catalyseur de transfert de phase des cations est un acide minéral fort, un acide aliphatique perhalogéné, un acide benzènesulfonique substitué par un ou deux groupes alkyle ou alcoxy chacun en $C_1$-$C_{12}$, un acide benzènesulfonique substitué par 1 à 3 atomes d'halogène, un acide alkylsulfonique en $C_1$-$C_{12}$ halogéné le cas échéant, un acide monoalkylsulfurique en $C_1$-$C_{12}$ ou un sel alcalin ou d'ammonium de ces acides.

11. Procédé selon la revendication 10, caractérisé par le fait que le catalyseur de transfert de phase des cations est utilisé à raison de 10 à 200 g, de préférence de 10 à 100 g par litre de solution de traitement.

12. Matière photographique pour le procédé de préparation d'images photographiques en couleurs selon l'une des revendications 1 à 11, caractérisé par le fait qu'elle contient sur un support opaque ou transparent, au moins une couche d'émulsion d'halogénure d'argent et dans la même couche ou dans une couche voisine une dispersion du triazène oléosoluble répondant aux formules (1) ou (2), et du constituant de copulation oléosoluble répondant à la formule (3), dans un solvant ou dans un mélange de solvants non miscibles à l'eau.

13. Préparation pour le traitement de la matière photographique selon la revendication 12, caractérisée par le fait qu'elle contient :

a) des acides alkyl- ou arylsulfoniques, de l'acide sulfurique, de l'acide sulfamique ou le cas échéant également des mélanges de ces acides,

b) de l'acide trifluoracétique ou trichloracétique ou de l'acide perchlorique,

le cas échéant

c) un ligand qui forme des complexes d'argent,

d) un catalyseur de blanchiment des couleurs,

e) un agent de protection contre l'oxydation,

éventuellement

f) un agent oxydant

et éventuellement

g) un solvant de l'halogénure d'argent.